# EUROPEAN PATENT SPECIFICATION

(11) **EP 3 999 038 B1**
(45) Date of publication and mention of the grant of the patent: **10.01.2024**
(21) Application number: 20753563.4
(22) Date of filing: 16.07.2020
(51) Int. Cl.: A61K 9/16, A61K 9/20, A61K 9/28, A61K 31/4178, A61P 9/04

(54) **CARDIAC SARCOMERE INHIBITOR ORAL FORMULATIONS**
ORALE FORMULIERUNGEN EINES KARDIALEN SARKOMERASE-INHIBITORS
FORMULATIONS PERORALES D'INHIBITEUR DE SARCOMÈRES CARDIAQUES

(30) Priority: 17.07.2019 US 201962875358 P
(43) Date of publication of application: 25.05.2022
(73) Proprietor: Cytokinetics, Inc., South San Francisco CA 94080 (US)
(72) Inventor: QIAO, Chunsheng, Palo Alto, California 94306 (US)
(74) Representative: Carpmaels & Ransford LLP
(86) International application number: PCT/US2020/042389
(87) International publication number: WO 2021/011808

(56) References cited:
- WO-A1-2014/205234
- WO-A1-2019/144041
- PHILIPSON DANIEL J ET AL: "Emerging pharmacologic and structural therapies for hypertrophic cardiomyopathy", HEART FAILURE REVIEWS, KLUWER ACADEMIC PUBLISHERS, DORDRECHT, NL, vol. 22, no. 6, 31 August 2017 (2017-08-31), pages 879-888, XP036335516, ISSN: 1382-4147, DOI: 10.1007/S10741-017-9648-X [retrieved on 2017-08-31]

## Description

### FIELD

The invention, which is defined by the appended claims, relates to formulations comprising cardiac sarcomere inhibitor (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof, processes for making such formulations, and formulations comprising (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof, for use in methods of treating various cardiac diseases and conditions.

### BACKGROUND

The cardiac sarcomere is the basic unit of muscle contraction in the heart. The cardiac sarcomere is a highly ordered cytoskeletal structure composed of cardiac muscle myosin, actin and a set of regulatory proteins. Cardiac muscle myosin is the cytoskeletal motor protein in the cardiac muscle cell. It is directly responsible for converting chemical energy into the mechanical force, resulting in cardiac muscle contraction. The cardiac sarcomere is composed of a network of contractile and structural proteins that regulate cardiac muscle function. The components of the cardiac sarcomere present targets for the treatment of various cardiac diseases and conditions, for example by increasing contractility or facilitating complete relaxation to modulate systolic and diastolic function, respectively. The force and speed of cardiac muscle contraction is a major determinant of organ function and is modulated by the cyclical interactions of actin and myosin. Regulation of actin and myosin binding is determined by a network of myofilament regulatory proteins and the level of intracellular Ca²⁺. The troponin complex and tropomyosin are thin filament proteins which govern the availability of actin binding sites, and the essential and regulatory light chains, and myosin binding protein C modulate the position and mechanical properties of myosin.

Abnormalities in the cardiac sarcomere have been identified as the driving cause for a variety of cardiac diseases and conditions, such as hypertrophic cardiomyopathy (HCM) and heart failure with preserved ejection fraction (HFpEF). Mutations in the proteins of the sarcomere cause disease by rendering the cardiac muscle either 'hyper' or 'hypo' contractile. Modulators of the cardiac sarcomere can be used to rebalance contractility and stop or reverse the course of disease.

Current agents that target the cardiac sarcomere, such as inotropes (drugs that increase the contractile ability of the heart) are poorly selective for cardiac tissue, which leads to recognized adverse effects that limit their use. These adverse effects include cell damage caused by an increased rate of energy expenditure, exacerbation of relaxation abnormalities, and potential arrhythmogenic side effects that may result from increased cytosolic Ca++ and cyclic AMP concentrations in the inotropically stimulated myocardium. Given the limitations of current agents, new approaches are needed to improve cardiac function in HCM and HFpEF.

There remains a great need for agents that exploit new mechanisms of action and may have better outcomes in terms of relief of symptoms, safety, and subject mortality, both short-term and long-term. New agents with an improved therapeutic index over current agents will provide a means to achieve these clinical outcomes. The selectivity of agents directed at the cardiac sarcomere (for example, by targeting cardiac myosin) has been identified as an important means to achieve this improved therapeutic index.

Newly developed cardiac sarcomere inhibitors are described in International Application No. PCT/US2019/014344, published as WO 2019/144041. Specifically, International Application No. PCT/US2019/014344 describes a genus of compounds, including the compound (R)-N-(5-(5-ethyl-1,2,4-oxadiazol-3-yl)-2,3-dihydro-1H-inden-1-yl)-1-methyl-1H-pyrazole-4-carboxamide, having the structure:

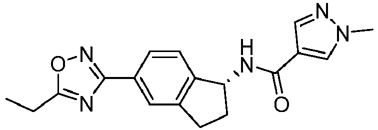

The present disclosure provides formulations comprising the cardiac sarcomere inhibitor (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof. (R)-N-(5-(5-ethyl-1,2,4-oxadiazol-3-yl)-2,3-dihydro-1H-inden-1-yl)-1-methyl-1H-pyrazole-4-carboxamide is a selective allosteric inhibitor of cardiac myosin that has little to no effect on smooth muscle myosin. Benefits of this compound include a wide therapeutic index, low impact on cardiac relaxation, and advantageous pharmacokinetic and safety profiles. The present disclosure also provides processes for making formulations comprising the compound (R)-N-(5-(5-ethyl-1,2,4-oxadiazol-3 -yl)-2,3 -dihydro- 1H-inden-1-yl)-1-methyl-1H-pyrazole-4-carboxamide, or a hydrate, solvate, polymorph, or pharmaceutically acceptable salt thereof, and formulations comprising (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof for use in methods of treating various cardiac diseases and conditions, such as for example methods of treating heart failure including HCM and HFpEF.

### SUMMARY

In one aspect, provided is a formulation comprising: (i) (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof; (ii) a filler; (iii) a binder; (iv) a disintegrant; (v) a surfactant; and (vi) a lubricant.

In another aspect, provided is a formulation comprising: (i) (R)-N-(5-(5-ethyl-1,2,4-oxadiazol-3-yl)-2,3-dihydro-1H-inden-1-yl)-1-methyl-1H-pyrazole-4-carboxamide; (ii) a filler; (iii) a binder; (iv) a disintegrant; (v) a surfactant; and (vi) a lubricant.

In another aspect, provided is a tablet comprising: (i) a core having a total core weight comprising: (a) an intra-granular component comprising: (a-i) (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof; (a-ii) an intra-granular filler; (a-iii) an intra-granular binder; (a-iv) an intra-granular disintegrant; and (a-v) an intra-granular surfactant; and (b) an extra-granular component comprising: (b-i) an extra-granular filler; (b-ii) an extra-granular disintegrant; and (b-iii) an extra-granular lubricant; and optionally (ii) a coating layer comprising a coating agent.

In yet another aspect, provided is a tablet comprising: (i) a core having a total core weight comprising: (a) an intra-granular component comprising: (a-i) (R)-N-(5-(5-ethyl-1,2,4-oxadiazol-3-yl)-2,3-dihydro-1H-inden-1-yl)-1-methyl-1H-pyrazole-4-carboxamide; (a-ii) an intra-granular filler; (a-iii) an intra-granular binder; (a-iv) an intra-granular disintegrant; and (a-v) an intra-granular surfactant; and (b) an extra-granular component comprising: (b-i) an extra-granular filler; (b-ii) an extra-granular disintegrant; and (b-iii) an extra-granular lubricant; and optionally (ii) a coating layer comprising a coating agent.

In yet another aspect, provided is a process for making a tablet comprising: (i) a core having a total core weight comprising: (a) an intra-granular component comprising: (a-i) (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof; (a-ii) an intra-granular filler; (a-iii) an intra-granular binder; (a-iv) an intra-granular disintegrant; and (a-v) an intra-granular surfactant; and (b) an extra-granular component comprising: (b-i) an extra-granular filler; (b-ii) an extra-granular disintegrant; and (b-iii) an extra-granular lubricant; and (ii) a coating layer comprising a coating agent, wherein the process comprises: (1) preparing granules comprising (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof, the intra-granular filler, the intra-granular binder, the intra-granular disintegrant, and the intra-granular surfactant; (2) milling the granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant to form the intra-granular component; (3) blending the intra-granular component with the extra-granular filler, the extra-granular disintegrant, and the extra-granular lubricant to form a final blend mixture; (4) compressing the final blend mixture to form the core; and (5) optionally coating the core with the coating layer; for example wherein preparing granules comprising (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof, the intra-granular filler, the intra-granular binder, the intra-granular disintegrant, and the intra-granular surfactant comprises a wet granulation step; for example wherein the wet granulation step further comprises a wet milling step.

In yet another aspect, provided is a process for making a tablet comprising: (i) a core having a total core weight comprising: (a) an intra-granular component comprising: (a-i) (R)-N-(5-(5-ethyl-1,2,4-oxadiazol-3-yl)-2,3-dihydro-1H-inden-1-yl)-1-methyl-1H-pyrazole-4-carboxamide; (a-ii) an intra-granular filler; (a-iii) an intra-granular binder; (a-iv) an intra-granular disintegrant; and (a-v) an intra-granular surfactant; and (b) an extra-granular component comprising: (b-i) an extra-granular filler; (b-ii) an extra-granular disintegrant; and (b-iii) an extra-granular lubricant; and (ii) a coating layer comprising a coating agent, wherein the process comprises: (1) preparing granules comprising (R)-N-(5-(5-ethyl-1,2,4-oxadiazol-3-yl)-2,3-dihydro-1H-inden-1-yl)-1-methyl-1H-pyrazole-4-carboxamide, the intra-granular filler, the intra-granular binder, the intra-granular disintegrant, and the intra-granular surfactant; (2) milling the granules comprising (R)-N-(5-(5-ethyl-1,2,4-oxadiazol-3-yl)-2,3-dihydro-1H-inden-1-yl)-1-methyl-1H-pyrazole-4-carboxamide, the intra-granular filler, the intra-granular binder, the intra-granular disintegrant, and the intra-granular surfactant to form the intra-granular component; (3) blending the intra-granular component with the extra-granular filler, the extra-granular disintegrant, and the extra-granular lubricant to form a final blend mixture; (4) compressing the final blend mixture to form the core; and (5) optionally coating the core with the coating layer; for example wherein preparing granules comprising (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof, the intra-granular filler, the intra-granular binder, the intra-granular disintegrant, and the intra-granular surfactant comprises a wet granulation step; for example wherein the wet granulation step further comprises a wet milling step.

In yet other aspects, provided is a formulation or a tablet disclosed herein for use in treating heart disease in a subject in need thereof. In some embodiments, the heart disease is hypertrophic cardiomyopathy (HCM). In some embodiments, the HCM is obstructive or nonobstructive or is associated with sarcomeric and/or non-sarcomeric mutations. In some embodiments, the heart disease is heart failure with preserved ejection fraction (HFpEF).

### BRIEF DESCRIPTION OF THE DRAWING

FIG. 1 presents a flow chart illustrating Unit Operations in the preparation of a formulation described herein.
FIG. 2 presents a flow chart illustrating Unit Operations in the preparation of a non-coated tablet described herein.
FIG. 3 presents a flow chart illustrating Unit Operations in the preparation of a coated tablet described herein.

### DETAILED DESCRIPTION

### Definitions

As used in the present specification, the following words and phrases are generally intended to have the meanings as set forth below, except to the extent that the context in which they are used indicates otherwise.

For use herein, unless clearly indicated otherwise, use of the terms "a", "an" and the like refers to one or more.

Reference to "about" a value or parameter herein includes (and describes) embodiments that are directed to that value or parameter per se. For example, description referring to "about X" includes description of "X".

The term "pharmaceutically acceptable salt" refers to a salt of any of the compounds herein which are known to be non-toxic and are commonly used in the pharmaceutical literature. In some embodiments, the pharmaceutically acceptable salt of a compound retains the biological effectiveness of the compounds described herein and are not biologically or otherwise undesirable. Examples of pharmaceutically acceptable salts can be found in Berge et al., Pharmaceutical Salts, J. Pharmaceutical Sciences, January 1977, 66(1), 1-19. Pharmaceutically acceptable acid addition salts can be formed with inorganic acids and organic acids. Inorganic acids from which salts can be derived include, for example, hydrochloric acid, hydrobromic acid, sulfuric acid, nitric acid, and phosphoric acid. Organic acids from which salts can be derived include, for example, acetic acid, propionic acid, glycolic acid, pyruvic acid, lactic acid, oxalic acid, malic acid, maleic acid, malonic acid, succinic acid, fumaric acid, tartaric acid, citric acid, benzoic acid, cinnamic acid, mandelic acid, methanesulfonic acid, ethanesulfonic acid, 2-hydroxyethylsulfonic acid, p-toluenesulfonic acid, stearic acid and salicylic acid. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, and aluminum. Organic bases from which salts can be derived include, for example, primary, secondary, and tertiary amines; substituted amines including naturally occurring substituted amines; cyclic amines; and basic ion exchange resins. Examples of organic bases include isopropylamine, trimethylamine, diethylamine, triethylamine, tripropylamine, and ethanolamine. In some embodiments, the pharmaceutically acceptable base addition salt is selected from ammonium, potassium, sodium, calcium, and magnesium salts.

If the compound described herein is obtained as an acid addition salt, the free base can be obtained by basifying a solution of the acid salt. Conversely, if the compound is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds (see, e.g., Berge et al., Pharmaceutical Salts, J. Pharmaceutical Sciences, January 1977, 66(1), 1-19). Those skilled in the art will recognize various synthetic methodologies that may be used to prepare pharmaceutically acceptable addition salts.

A "solvate" is formed by the interaction of a solvent and a compound. Suitable solvents include, for example, water and alcohols (e.g., ethanol). Solvates include hydrates having any ratio of compound to water, such as monohydrates, dihydrates and hemi-hydrates.

The term "hydrate" refers to the chemical entity formed by the interaction of water and a compound, including, for example, hemi-hydrates, monohydrates, dihydrates, trihydrates, etc.

As used herein, the term "polymorph" or "polymorphic form" refers to a crystalline form of a compound. Different polymorphs may have different physical properties such as, for example, melting temperatures, heats of fusion, solubilities, dissolution rates, and/or vibrational spectra as a result of the arrangement or conformation of the molecules or ions in the crystal lattice. The differences in physical properties exhibited by polymorphs may affect pharmaceutical parameters, such as storage stability, compressibility, density (important in formulation and product manufacturing), and dissolution rate (an important factor in bioavailability). Differences in stability can result from changes in chemical reactivity (*e*.*g*., differential oxidation, such that a dosage form discolors more rapidly when comprised of one polymorph than when comprised of another polymorph), mechanical changes (*e*.*g*., tablets crumble on storage as a kinetically favored polymorph converts to thermodynamically more stable polymorph), or both (*e.g.,* tablets of one polymorph are more susceptible to breakdown at high humidity). As a result of solubility/dissolution differences, in the extreme case, some polymorphic transitions may result in lack of potency or, at the other extreme, toxicity. In addition, the physical properties of a crystalline form may be important in processing; for example, one polymorph might be more likely to form solvates or might be difficult to filter and wash free of impurities (*e*.*g*., particle shape and size distribution might be different between polymorphs).

As used herein, the term "subject" refers to an animal, such as a mammal, bird, or fish. In some embodiments, the subject is a mammal. Mammals include, for example, mice, rats, dogs, cats, pigs, sheep, horses, cows and humans. In some embodiments, the subject is a human, for example a human that has been or will be the object of treatment, observation or experiment. The formulations, tablets, and methods described herein can be useful in both human therapy and veterinary applications.

As used herein, the term "therapeutic" refers to the ability to modulate the cardiac sarcomere. As used herein, "modulation" refers to a change in activity as a direct or indirect response to the presence of a chemical entity as described herein, relative to the activity of in the absence of the chemical entity. The change may be an increase in activity or a decrease in activity, and may be due to the direct interaction of the chemical entity with the a target or due to the interaction of the chemical entity with one or more other factors that in turn affect the target's activity. For example, the presence of the chemical entity may, for example, increase or decrease the target activity by directly binding to the target, by causing (directly or indirectly) another factor to increase or decrease the target activity, or by (directly or indirectly) increasing or decreasing the amount of target present in the cell or organism.

The term "therapeutically effective amount" or "effective amount" refers to that amount of a compound disclosed and/or described herein that is sufficient to affect treatment, as defined herein, when administered to a subject in need of such treatment. A therapeutically effective amount of a compound may be an amount sufficient to treat a disease responsive to modulation of the cardiac sarcomere. The therapeutically effective amount will vary depending upon, for example, the subject and disease condition being treated, the weight and age of the subject, the severity of the disease condition, the particular compound, the dosing regimen to be followed, timing of administration, the manner of administration, all of which can readily be determined by one of ordinary skill in the art. The therapeutically effective amount may be ascertained experimentally, for example by assaying blood concentration of the chemical entity, or theoretically, by calculating bioavailability.

"Treatment" (and related terms, such as "treat", "treated", "treating") includes one or more of: preventing a disease or disorder (i.e., causing the clinical symptoms of the disease or disorder not to develop); inhibiting a disease or disorder; slowing or arresting the development of clinical symptoms of a disease or disorder; and/or relieving a disease or disorder (i.e., causing relief from or regression of clinical symptoms). The term encompasses situations where the disease or disorder is already being experienced by a subject, as well as situations where the disease or disorder is not currently being experienced but is expected to arise. The term covers both complete and partial reduction or prevention of the condition or disorder, and complete or partial reduction of clinical symptoms of a disease or disorder. Thus, compounds described and/or disclosed herein may prevent an existing disease or disorder from worsening, assist in the management of the disease or disorder, or reduce or eliminate the disease or disorder. When used in a prophylactic manner, the compounds disclosed and/or described herein may prevent a disease or disorder from developing or lessen the extent of a disease or disorder that may develop.

"ATPase" refers to an enzyme that hydrolyzes ATP. ATPases include proteins comprising molecular motors such as the myosins.

The compound depicted herein may be present as a salt even if a salt is not depicted, and it is understood that the formulations, tablets, and methods provided herein embrace all salts and solvates of the compound depicted here, as well as the non-salt and non-solvate form of the compound, as is well understood by the skilled artisan. In some embodiments, the salts of the compound provided herein are pharmaceutically acceptable salts.

### Formulations

Provided herein is a formulation comprising: (i) (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof; (ii) a filler; (iii) a binder; (iv) a disintegrant; (v) a surfactant; and (vi) a lubricant.

In some embodiments, provided is a formulation comprising: (i) (R)-N-(5-(5-ethyl-1,2,4-oxadiazol-3-yl)-2,3-dihydro-1H-inden-1-yl)-1-methyl-1H-pyrazole-4-carboxamide; (ii) a filler; (iii) a binder; (iv) a disintegrant; (v) a surfactant; and (vi) a lubricant.

In some embodiments, the filler is selected from the group consisting of powdered cellulose, microcrystalline cellulose, silicified microcrystalline cellulose, kaolin, corn starch, maize starch, starch derivatives, pregelatinized starch, calcium phosphate, calcium hydrogen phosphate, dicalcium phosphate, tricalcium phosphate, compressible sugar, sugar alcohol, mannitol, sorbitol, maltitol, xylitol, lactitol, lactose, dextrose, maltose, sucrose, glucose, fructose, saccharose, raffinose, dextrates, trehalose, maltodextrines, and mixtures of any of the foregoing.

In some embodiments, the binder is selected from the group consisting of arabic gum, acacia gum, alginate, alginic acid, corn starch, copolyvidone, polyvinylpyrrolidone, gelatin, glyceryl behenate, hydroxyethylcellulose, hydroxypropyl cellulose, carboxymethylcellulose, carboxymethylcellulose calcium, carboxymethylcellulose sodium, methylcellulose, hypromellose, lactose, polyvinyl alcohol, povidone, polyethylene oxide, polyacrylates, potato starch, pregelatinized starch, sodium alginate, sodium starch, sodium carboxy methyl cellulose, starch, and mixtures of any of the foregoing.

In some embodiments, the disintegrant is selected from the group consisting of alginic acid, croscarmellose sodium, cellulose, carboxymethylcellulose calcium, carboxymethylcellulose sodium, microcrystalline cellulose, crospovidone, sodium starch glycolate, low-substituted hydroxypropyl cellulose, polacrillin potassium, pregelatinized starch, partially hydrolyzed starch, sodium carboxymethyl starch, starch, sodium alginate, sodium carboxy methyl cellulose, and mixtures of any of the foregoing.

In some embodiments, the surfactant is selected from the group consisting of cetylpyridine chloride, heptadecaethylene oxycetanol, lecithins, polyoxyethylene stearate, nonoxynol 9, nonoxynol 10, octoxynol 9, sorbitan fatty acid esters, Span 20, Span 40, Span 60, Span 80, Span 85, polysorbates, polysorbate 20, polysorbate 21, polysorbate 40, polysorbate 60, polysorbate 61, polysorbate 65, polysorbate 80, sodium salts of fatty alcohol sulfates, sodium lauryl sulfate, sodium salts of sulfosuccinates, sodium dioctylsulfosuccinate, partial esters of fatty acids with alcohols, glycerine monostearate, glyceryl monooleate, ethers of fatty alcohols with polyoxyethylene, esters of fatty acids with polyoxyethylene, copolymers of ethylenoxide and propylenoxide (Pluronic^{®}), benzalkonium chloride, ethoxylated triglycerides, and mixtures of any of the foregoing.

In some embodiments, the lubricant is selected from the group consisting of hydrogenated castor oil, magnesium stearate, glyceryl monostearate, calcium stearate, glyceryl behenate, glycerol distearate, glyceryl dipalmitostearate, behenoyl polyoxyl-8 glycerides, sodium stearyl fumarate, stearic acid, talc, zinc stearate, mineral oil, polyethylene glycol, polaxamer, sodium lauryl sulfate, and mixtures of any of the foregoing.

In some embodiments, the formulation comprises: (i) about 1 % by weight to about 80 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii) about 15 % by weight to about 90 % by weight of the filler; (iii) about 0.1 % by weight to about 10 % by weight of the binder; (iv) about 1 % by weight to about 10 % by weight of the disintegrant; (v) about 0.1 % by weight to about 10 % by weight of the surfactant; and (vi) about 0.1 % by weight to about 10 % by weight of the lubricant.

In some embodiments, the formulation comprises: (i) about 1 % by weight to about 80 % by weight of the (R)-N-(5-(5-ethyl-1,2,4-oxadiazol-3-yl)-2,3-dihydro-1H-inden-1-yl)-1-methyl-1H-pyrazole-4-carboxamide; (ii) about 15 % by weight to about 90 % by weight of the filler; (iii) about 0.1 % by weight to about 10 % by weight of the binder; (iv) about 1 % by weight to about 10 % by weight of the disintegrant; (v) about 0.1 % by weight to about 10 % by weight of the surfactant; and (vi) about 0.1 % by weight to about 10 % by weight of the lubricant.

In some embodiments, the formulation comprises: (i) about 1 % by weight to about 50 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii) about 40 % by weight to about 80 % by weight of the filler; (iii) about 0.5 % by weight to about 5 % by weight of the binder; (iv) about 2 % by weight to about 8 % by weight of the disintegrant; (v) about 0.5 % by weight to about 5 % by weight of the surfactant; and (vi) about 0.5 % by weight to about 5 % by weight of the lubricant.

In some embodiments, the formulation comprises: (i) about 1 % by weight to about 50 % by weight of the (R)-N-(5-(5-ethyl-1,2,4-oxadiazol-3-yl)-2,3-dihydro-1H-inden-1-yl)-1-methyl-1H-pyrazole-4-carboxamide; (ii) about 40 % by weight to about 80 % by weight of the filler; (iii) about 0.5 % by weight to about 5 % by weight of the binder; (iv) about 2 % by weight to about 8 % by weight of the disintegrant; (v) about 0.5 % by weight to about 5 % by weight of the surfactant; and (vi) about 0.5 % by weight to about 5 % by weight of the lubricant.

In some embodiments, the formulation comprises: (i) about 10 % by weight to about 30 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii) about 60 % by weight to about 80 % by weight of the filler; (iii) about 1 % by weight to about 3 % by weight of the binder; (iv) about 4 % by weight to about 6 % by weight of the disintegrant; (v) about 1 % by weight to about 3 % by weight of the surfactant; and (vi) about 0.5 % by weight to about 1.5 % by weight of the lubricant.

In some embodiments, the formulation comprises: (i) about 10 % by weight to about 30 % by weight of the (R)-N-(5-(5-ethyl-1,2,4-oxadiazol-3-yl)-2,3-dihydro-1H-inden-1-yl)-1-methyl-1H-pyrazole-4-carboxamide; (ii) about 60 % by weight to about 80 % by weight of the filler; (iii) about 1 % by weight to about 3 % by weight of the binder; (iv) about 4 % by weight to about 6 % by weight of the disintegrant; (v) about 1 % by weight to about 3 % by weight of the surfactant; and (vi) about 0.5 % by weight to about 1.5 % by weight of the lubricant.

In some embodiments, the formulation comprises: (i) about 20 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii) about 70 % by weight of the filler; (iii) about 2 % by weight of the binder; (iv) about 5 % by weight of the disintegrant; (v) about 2 % by weight of the surfactant; and (vi) about 1 % by weight of the lubricant.

In some embodiments, the formulation comprises: (i) about 20 % by weight of the (R)-N-(5-(5-ethyl-1,2,4-oxadiazol-3-yl)-2,3-dihydro-1H-inden-1-yl)-1-methyl-1H-pyrazole-4-carboxamide; (ii) about 70 % by weight of the filler; (iii) about 2 % by weight of the binder; (iv) about 5 % by weight of the disintegrant; (v) about 2 % by weight of the surfactant; and (vi) about 1 % by weight of the lubricant.

In some embodiments, the formulation comprises: (i) about 1 % by weight to about 10 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii) about 70 % by weight to about 90 % by weight of the filler; (iii) about 1 % by weight to about 3 % by weight of the binder; (iv) about 4 % by weight to about 6 % by weight of the disintegrant; (v) about 1 % by weight to about 3 % by weight of the surfactant; and (vi) about 0.5 % by weight to about 1.5 % by weight of the lubricant.

In some embodiments, the formulation comprises: (i) about 1 % by weight to about 10 % by weight of the (R)-N-(5-(5-ethyl-1,2,4-oxadiazol-3-yl)-2,3-dihydro-1H-inden-1-yl)-1-methyl-1H-pyrazole-4-carboxamide; (ii) about 70 % by weight to about 90 % by weight of the filler; (iii) about 1 % by weight to about 3 % by weight of the binder; (iv) about 4 % by weight to about 6 % by weight of the disintegrant; (v) about 1 % by weight to about 3 % by weight of the surfactant; and (vi) about 0.5 % by weight to about 1.5 % by weight of the lubricant.

In some embodiments, the formulation comprises:(i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii) about 85 % by weight of the filler; (iii) about 2 % by weight of the binder; (iv) about 5 % by weight of the disintegrant; (v) about 2 % by weight of the surfactant; and (vi) about 1 % by weight of the lubricant.

In some embodiments, the formulation comprises: (i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide; (ii) about 85 % by weight of the filler; (iii) about 2 % by weight of the binder; (iv) about 5 % by weight of the disintegrant; (v) about 2 % by weight of the surfactant; and (vi) about 1 % by weight of the lubricant.

In some embodiments, the formulation comprises: (i) about 10 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii) about 80 % by weight of the filler; (iii) about 2 % by weight of the binder; (iv) about 5 % by weight of the disintegrant; (v) about 2 % by weight of the surfactant; and (vi) about 1 % by weight of the lubricant.

In some embodiments, the formulation comprises: (i) about 10 % by weight of the (R)-N-(5-(5-ethyl-1,2,4-oxadiazol-3-yl)-2,3-dihydro-1H-inden-1-yl)-1-methyl-1H-pyrazole-4-carboxamide; (ii) about 80 % by weight of the filler; (iii) about 2 % by weight of the binder; (iv) about 5 % by weight of the disintegrant; (v) about 2 % by weight of the surfactant; and (vi) about 1 % by weight of the lubricant.

In some embodiments, the filler comprises mannitol. In some embodiments, the filler comprises microcrystalline cellulose. In some embodiments, the filler consists of mannitol and microcrystalline cellulose. In some embodiments, the binder is hydroxypropyl cellulose. In some embodiments, the disintegrant is croscarmellose sodium. In some embodiments, the surfactant is sodium lauryl sulfate. In some embodiments, the lubricant is magnesium stearate.

In some embodiments, the formulation comprises: (i) about 1 % by weight to about 50 % by weight of (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii-1) about 10 % by weight to about 60 % by weight of mannitol; (ii-2) about 5 % by weight to about 45 % by weight of microcrystalline cellulose; (iii) about 0.1 % by weight to about 10 % by weight of hydroxypropyl cellulose; (iv) about 1 % by weight to about 10 % by weight of croscarmellose sodium; (v) about 0.1 % by weight to about 10 % by weight of sodium lauryl sulfate; and (vi) about 0.1 % by weight to about 10 % by weight of magnesium stearate.

In some embodiments, the formulation comprises: (i) about 1 % by weight to about 50 % by weight of (R)-N-(5-(5-ethyl-1,2,4-oxadiazol-3-yl)-2,3-dihydro-1H-inden-1-yl)-1-methyl-1H-pyrazole-4-carboxamide; (ii-1) about 10 % by weight to about 60 % by weight of mannitol; (ii-2) about 5 % by weight to about 45 % by weight of microcrystalline cellulose; (iii) about 0.1 % by weight to about 10 % by weight of hydroxypropyl cellulose; (iv) about 1 % by weight to about 10 % by weight of croscarmellose sodium; (v) about 0.1 % by weight to about 10 % by weight of sodium lauryl sulfate; and (vi) about 0.1 % by weight to about 10 % by weight of magnesium stearate.

In some embodiments, the formulation comprises: (i) about 10 % by weight to about 30 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii-1) about 40 % by weight to about 50 % by weight of mannitol; (ii-2) about 20 % by weight to about 30 % by weight of microcrystalline cellulose; (iii) about 1 % by weight to about 3 % by weight of hydroxypropyl cellulose; (iv) about 4 % by weight to about 6 % by weight of croscarmellose sodium; (v) about 1 % by weight to about 3 % by weight of sodium lauryl sulfate; and (vi) about 0.5 % by weight to about 1.5 % by weight of magnesium stearate.

In some embodiments, the formulation comprises: (i) about 10 % by weight to about 30 % by weight of the (R)-N-(5-(5-ethyl-1,2,4-oxadiazol-3-yl)-2,3-dihydro-1H-inden-1-yl)-1-methyl-1H-pyrazole-4-carboxamide; (ii-1) about 40 % by weight to about 50 % by weight of mannitol; (ii-2) about 20 % by weight to about 30 % by weight of microcrystalline cellulose; (iii) about 1 % by weight to about 3 % by weight of hydroxypropyl cellulose; (iv) about 4 % by weight to about 6 % by weight of croscarmellose sodium; (v) about 1 % by weight to about 3 % by weight of sodium lauryl sulfate; and (vi) about 0.5 % by weight to about 1.5 % by weight of magnesium stearate.

In some embodiments, the formulation comprises: (i) about 1 % by weight to about 10 % by weight of (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii-1) about 50 % by weight to about 60 % by weight of mannitol; (ii-2) about 25 % by weight to about 35 % by weight of microcrystalline cellulose; (iii) about 1 % by weight to about 3 % by weight of hydroxypropyl cellulose; (iv) about 4 % by weight to about 6 % by weight of croscarmellose sodium; (v) about 1 % by weight to about 3 % by weight of sodium lauryl sulfate; and (vi) about 0.5 % by weight to about 1.5 % by weight of the magnesium stearate.

In some embodiments, the formulation comprises: (i) about 1 % by weight to about 10 % by weight of (R)-N-(5-(5-ethyl-1,2,4-oxadiazol-3-yl)-2,3-dihydro-1H-inden-1-yl)-1-methyl-1H-pyrazole-4-carboxamide; (ii-1) about 50 % by weight to about 60 % by weight of mannitol; (ii-2) about 25 % by weight to about 35 % by weight of microcrystalline cellulose; (iii) about 1 % by weight to about 3 % by weight of hydroxypropyl cellulose; (iv) about 4 % by weight to about 6 % by weight of croscarmellose sodium; (v) about 1 % by weight to about 3 % by weight of sodium lauryl sulfate; and (vi) about 0.5 % by weight to about 1.5 % by weight of the magnesium stearate.

In some embodiments, the formulation comprises: (i) about 20 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii-1) about 44 % by weight of mannitol; (ii-2) about 26 % by weight of microcrystalline cellulose; (iii) about 2 % by weight of hydroxypropyl cellulose; (iv) about 5 % by weight of croscarmellose sodium; (v) about 2 % by weight of sodium lauryl sulfate; and (vi) about 1 % by weight of magnesium stearate.

In some embodiments, the formulation comprises: (i) about 20 % by weight of the (R)-N-(5-(5-ethyl-1,2,4-oxadiazol-3-yl)-2,3-dihydro-1H-inden-1-yl)-1-methyl-1H-pyrazole-4-carboxamide; (ii-1) about 44 % by weight of mannitol; (ii-2) about 26 % by weight of microcrystalline cellulose; (iii) about 2 % by weight of hydroxypropyl cellulose; (iv) about 5 % by weight of croscarmellose sodium; (v) about 2 % by weight of sodium lauryl sulfate; and (vi) about 1 % by weight of magnesium stearate.

In some embodiments, the formulation comprises: (i) about 10 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii-1) about 50 % by weight of mannitol; (ii-2) about 30 % by weight of microcrystalline cellulose; (iii) about 2 % by weight of hydroxypropyl cellulose; (iv) about 5 % by weight of croscarmellose sodium; (v) about 2 % by weight of sodium lauryl sulfate; and (vi) about 1 % by weight of magnesium stearate.

In some embodiments, the formulation comprises: (i) about 10 % by weight of the (R)-N-(5-(5-ethyl-1,2,4-oxadiazol-3-yl)-2,3-dihydro-1H-inden-1-yl)-1-methyl-1H-pyrazole-4-carboxamide; (ii-1) about 50 % by weight of mannitol; (ii-2) about 30 % by weight of microcrystalline cellulose; (iii) about 2 % by weight of hydroxypropyl cellulose; (iv) about 5 % by weight of croscarmellose sodium; (v) about 2 % by weight of sodium lauryl sulfate; and (vi) about 1 % by weight of magnesium stearate.

In some embodiments, the formulation comprises: (i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (ii-1) about 54 % by weight of mannitol; (ii-2) about 31 % by weight of microcrystalline cellulose; (iii) about 2 % by weight of hydroxypropyl cellulose; (iv) about 5 % by weight of croscarmellose sodium; (v) about 2 % by weight of sodium lauryl sulfate; and (vi) about 1 % by weight of the magnesium stearate.

In some embodiments, the formulation comprises: (i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide; (ii-1) about 54 % by weight of mannitol; (ii-2) about 31 % by weight of microcrystalline cellulose; (iii) about 2 % by weight of hydroxypropyl cellulose; (iv) about 5 % by weight of croscarmellose sodium; (v) about 2 % by weight of sodium lauryl sulfate; and (vi) about 1 % by weight of the magnesium stearate.

Administration of the formulation disclosed herein can be via any accepted mode of administration for solid and semi-solid formulations including, but not limited to, oral, sublingual, subcutaneous, parenteral, intranasal, topical, transdermal, intraperitoneal, intramuscular, intrapulmonary, vaginal, rectal, or intraocular administration. In some embodiments, the formulation is for oral or sublingual administration. In some embodiments, the formulation is for oral administration.

In some embodiments, the formulation is for administration once daily. In some embodiments, the formulation is for administration twice daily. In some embodiments, the formulation is for administration three times daily. In some embodiments, the formulation is for administration four times daily. In some embodiments, the formulation is for administration five times daily.

In some embodiments, the formulation takes the form of a pill, a capsule, or a tablet. In some embodiments, the formulation is a tablet.

### Tablets

Provided herein is a tablet comprising: (i) a core having a total core weight comprising: (a) an intra-granular component comprising: (a-i) (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof; (a-ii) an intra-granular filler; (a-iii) an intra-granular binder; (a-iv) an intra-granular disintegrant; and (a-v) an intra-granular surfactant; and (b) an extra-granular component comprising: (b-i) an extra-granular filler; (b-ii) an extra-granular disintegrant; and (b-iii) an extra-granular lubricant; and optionally (ii) a coating layer comprising a coating agent.

In some embodiments, provided is a tablet comprising: (i) a core having a total core weight comprising: (a) an intra-granular component comprising: (a-i) (R)-N-(5-(5-ethyl-1,2,4-oxadiazol-3-yl)-2,3-dihydro-1H-inden-1-yl)-1-methyl-1H-pyrazole-4-carboxamide; (a-ii) an intra-granular filler; (a-iii) an intra-granular binder; (a-iv) an intra-granular disintegrant; and (a-v) an intra-granular surfactant; and (b) an extra-granular component comprising: (b-i) an extra-granular filler; (b-ii) an extra-granular disintegrant; and (b-iii) an extra-granular lubricant; and optionally (ii) a coating layer comprising a coating agent.

In some embodiments, the intra-granular filler is selected from the group consisting of powdered cellulose, microcrystalline cellulose, silicified microcrystalline cellulose, kaolin, corn starch, maize starch, starch derivatives, pregelatinized starch, calcium phosphate, calcium hydrogen phosphate, dicalcium phosphate, tricalcium phosphate, compressible sugar, sugar alcohol, mannitol, sorbitol, maltitol, xylitol, lactitol, lactose, dextrose, maltose, sucrose, glucose, fructose, saccharose, raffinose, dextrates, trehalose, maltodextrines, and mixtures of any of the foregoing.

In some embodiments, the intra-granular binder is selected from the group consisting of arabic gum, acacia gum, alginate, alginic acid, corn starch, copolyvidone, polyvinylpyrrolidone, gelatin, glyceryl behenate, hydroxyethylcellulose, hydroxypropyl cellulose, carboxymethylcellulose, carboxymethylcellulose calcium, carboxymethylcellulose sodium, methylcellulose, hypromellose, lactose, polyvinyl alcohol, povidone, polyethylene oxide, polyacrylates, potato starch, pregelatinized starch, sodium alginate, sodium starch, sodium carboxy methyl cellulose, starch, and mixtures of any of the foregoing.

In some embodiments, the intra-granular disintegrant is selected from the group consisting of alginic acid, croscarmellose sodium, cellulose, carboxymethylcellulose calcium, carboxymethylcellulose sodium, microcrystalline cellulose, crospovidone, sodium starch glycolate, low-substituted hydroxypropyl cellulose, polacrillin potassium, pregelatinized starch, partially hydrolyzed starch, sodium carboxymethyl starch, starch, sodium alginate, sodium carboxy methyl cellulose, and mixtures of any of the foregoing.

In some embodiments, the intra-granular surfactant is selected from the group consisting of cetylpyridine chloride, heptadecaethylene oxycetanol, lecithins, polyoxyethylene stearate, nonoxynol 9, nonoxynol 10, octoxynol 9, sorbitan fatty acid esters, Span 20, Span 40, Span 60, Span 80, Span 85, polysorbates, polysorbate 20, polysorbate 21, polysorbate 40, polysorbate 60, polysorbate 61, polysorbate 65, polysorbate 80, sodium salts of fatty alcohol sulfates, sodium lauryl sulfate, sodium salts of sulfosuccinates, sodium dioctylsulfosuccinate, partial esters of fatty acids with alcohols, glycerine monostearate, glyceryl monooleate, ethers of fatty alcohols with polyoxyethylene, esters of fatty acids with polyoxyethylene, copolymers of ethylenoxide and propylenoxide (Pluronic^{®}), benzalkonium chloride, ethoxylated triglycerides, and mixtures of any of the foregoing.

In some embodiments, the extra-granular filler is selected from the group consisting of powdered cellulose, microcrystalline cellulose, silicified microcrystalline cellulose, kaolin, corn starch, maize starch, starch derivatives, pregelatinized starch, calcium phosphate, calcium hydrogen phosphate, dicalcium phosphate, tricalcium phosphate, compressible sugar, sugar alcohol, mannitol, sorbitol, maltitol, xylitol, lactitol, lactose, dextrose, maltose, sucrose, glucose, fructose, saccharose, raffinose, dextrates, trehalose, maltodextrines, and mixtures of any of the foregoing.

In some embodiments, the extra-granular disintegrant is selected from the group consisting of alginic acid, croscarmellose sodium, cellulose, carboxymethylcellulose calcium, carboxymethylcellulose sodium, microcrystalline cellulose, crospovidone, sodium starch glycolate, low-substituted hydroxypropyl cellulose, polacrillin potassium, pregelatinized starch, partially hydrolyzed starch, sodium carboxymethyl starch, starch, sodium alginate, sodium carboxy methyl cellulose, and mixtures of any of the foregoing.

In some embodiments, the extra-granular lubricant is selected from the group consisting of hydrogenated castor oil, magnesium stearate, glyceryl monostearate, calcium stearate, glyceryl behenate, glycerol distearate, glyceryl dipalmitostearate, behenoyl polyoxyl-8 glycerides, sodium stearyl fumarate, stearic acid, talc, zinc stearate, mineral oil, polyethylene glycol, polaxamer, sodium lauryl sulfate, and mixtures of any of the foregoing.

In some embodiments, the core comprises: (a) an intra-granular component comprising: (a-i) about 1 % to about 80 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii) about 10 % to about 80 % of the total core weight of the intra-granular filler; (a-iii) about 0.1 % to about 10 % of the total core weight of the intra-granular binder; (a-iv) about 0.1 % to about 5 % of the total core weight of the intra-granular disintegrant; and (a-v) about 0.1 % to about 5 % of the total core weight of the intra-granular surfactant; and (b) an extra-granular component comprising: (b-i) about 5 % to about 15 % of the total core weight of the extra-granular filler; (b-ii) about 0.1 % to about 5 % of the total core weight of the extra-granular disintegrant; and (b-iii) about 0.1 % to about 5 % of the total core weight of the extra-granular lubricant.

In some embodiments, the core comprises: (a) an intra-granular component comprising: (a-i) about 1 % to about 80 % of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii) about 10 % to about 80 % of the total core weight of the intra-granular filler; (a-iii) about 0.1 % to about 10 % of the total core weight of the intra-granular binder; (a-iv) about 0.1 % to about 5 % of the total core weight of the intra-granular disintegrant; and (a-v) about 0.1% to about 5 % of the total core weight of the intra-granular surfactant; and (b) an extra-granular component comprising: (b-i) about 5 % to about 15 % of the total core weight of the extra-granular filler; (b-ii) about 0.1 % to about 5 % of the total core weight of the extra-granular disintegrant; and (b-iii) about 0.1 % to about 5 % of the total core weight of the extra-granular lubricant.

In some embodiments, the core comprises: (a) an intra-granular component comprising: (a-i) about 1 % to about 50 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii) about 40 % to about 80 % of the total core weight of the intra-granular filler; (a-iii) about 1 % to about 5 % of the total core weight of the intra-granular binder; (a-iv) about 1 % to about 5 % of the total core weight of the intra-granular disintegrant; and (a-v) about 1 % to about 5 % of the total core weight of the intra-granular surfactant; and (b) an extra-granular component comprising: (b-i) about 5 % to about 15 % of the total core weight of the extra-granular filler; (b-ii) about 1 % to about 5 % of the total core weight of the extra-granular disintegrant; and (b-iii) about 0.1 % to about 2 % of the total core weight of the extra-granular lubricant.

In some embodiments, the core comprises: (a) an intra-granular component comprising: (a-i) about 1 % to about 50 % of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii) about 40 % to about 80 % of the total core weight of the intra-granular filler; (a-iii) about 1 % to about 5 % of the total core weight of the intra-granular binder; (a-iv) about 1 % to about 5 % of the total core weight of the intra-granular disintegrant; and (a-v) about 1 % to about 5 % of the total core weight of the intra-granular surfactant; and (b) an extra-granular component comprising: (b-i) about 5 % to about 15 % of the total core weight of the extra-granular filler; (b-ii) about 1 % to about 5 % of the total core weight of the extra-granular disintegrant; and (b-iii) about 0.1 % to about 2 % of the total core weight of the extra-granular lubricant.

In some embodiments, the core comprises: (a) an intra-granular component comprising: (a-i) about 10 % to about 30 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii) about 50 % to about 70 % of the total core weight of the intra-granular filler; (a-iii) about 1 % to about 3 % of the total core weight of the intra-granular binder; (a-iv) about 2 % to about 4 % of the total core weight of the intra-granular disintegrant; and (a-v) about 1 % to about 3 % of the total core weight of the intra-granular surfactant; and (b) an extra-granular component comprising: (b-i) about 5 % to about 15 % of the total core weight of the an extra-granular filler; (b-ii) about 1 % to about 3 % of the total core weight of the extra-granular disintegrant; and (b-iii) about 0.1 % to about 1.5 % of the total core weight of the extra-granular lubricant.

In some embodiments, the core comprises: (a) an intra-granular component comprising: (a-i) about 10 % to about 30 % of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii) about 50 % to about 70 % of the total core weight of the intra-granular filler; (a-iii) about 1 % to about 3 % of the total core weight of the intra-granular binder; (a-iv) about 2 % to about 4 % of the total core weight of the intra-granular disintegrant; and (a-v) about 1 % to about 3 % of the total core weight of the intra-granular surfactant; and (b) an extra-granular component comprising: (b-i) about 5 % to about 15 % of the total core weight of the an extra-granular filler; (b-ii) about 1 % to about 3 % of the total core weight of the extra-granular disintegrant; and (b-iii) about 0.1 % to about 1.5 % of the total core weight of the extra-granular lubricant.

In some embodiments, the core comprises: (a) an intra-granular component comprising: (a-i) about 1 % to about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii) about 60 % to about 80 % of the total core weight of the intra-granular filler; (a-iii) about 1 % to about 3 % of the total core weight of the intra-granular binder; (a-iv) about 2 % to about 4 % of the total core weight of the intra-granular disintegrant; and (a-v) about 1 % to about 3 % of the total core weight of the intra-granular surfactant; and (b) an extra-granular component comprising: (b-i) about 5 % to about 15 % of the total core weight of the an extra-granular filler; (b-ii) about 1 % to about 3 % of the total core weight of the extra-granular disintegrant; and (b-iii) about 0.1 % to about 1.5 % of the total core weight of the extra-granular lubricant.

In some embodiments, the core comprises: (a) an intra-granular component comprising: (a-i) about 1 % to about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii) about 60 % to about 80 % of the total core weight of the intra-granular filler; (a-iii) about 1 % to about 3 % of the total core weight of the intra-granular binder; (a-iv) about 2 % to about 4 % of the total core weight of the intra-granular disintegrant; and (a-v) about 1 % to about 3 % of the total core weight of the intra-granular surfactant; and (b) an extra-granular component comprising: (b-i) about 5 % to about 15 % of the total core weight of the an extra-granular filler; (b-ii) about 1 % to about 3 % of the total core weight of the extra-granular disintegrant; and (b-iii) about 0.1 % to about 1.5 % of the total core weight of the extra-granular lubricant.

In some embodiments, the core comprises: (a) an intra-granular component comprising: (a-i) about 5 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii) about 74 % of the total core weight of the intra-granular filler; (a-iii) about 2 % of the total core weight of the intra-granular binder; (a-iv) about 3 % of the total core weight of the intra-granular disintegrant; and (a-v) about 2 % of the total core weight of the intra-granular surfactant; and (b) an extra-granular component comprising: (b-i) about 11% of the total core weight of the an extra-granular filler; (b-ii) about 2 % of the total core weight of the extra-granular disintegrant; and (b-iii) about 1 % of the total core weight of the extra-granular lubricant.

In some embodiments, the core comprises: (a) an intra-granular component comprising: (a-i) about 5 % of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii) about 74 % of the total core weight of the intra-granular filler; (a-iii) about 2 % of the total core weight of the intra-granular binder; (a-iv) about 3 % of the total core weight of the intra-granular disintegrant; and (a-v) about 2 % of the total core weight of the intra-granular surfactant; and (b) an extra-granular component comprising: (b-i) about 11% of the total core weight of the an extra-granular filler; (b-ii) about 2 % of the total core weight of the extra-granular disintegrant; and (b-iii) about 1 % of the total core weight of the extra-granular lubricant.

In some embodiments, the core comprises: (a) an intra-granular component comprising: (a-i) about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii) about 69 % of the total core weight of the intra-granular filler; (a-iii) about 2 % of the total core weight of the intra-granular binder; (a-iv) about 3 % of the total core weight of the intra-granular disintegrant; and (a-v) about 2 % of the total core weight of the intra-granular surfactant; and (b) an extra-granular component comprising: (b-i) about 11 % of the total core weight of the an extra-granular filler; (b-ii) about 2 % of the total core weight of the extra-granular disintegrant; and (b-iii) about 1 % of the total core weight of the extra-granular lubricant.

In some embodiments, the core comprises: (a) an intra-granular component comprising: (a-i) about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii) about 69 % of the total core weight of the intra-granular filler; (a-iii) about 2 % of the total core weight of the intra-granular binder; (a-iv) about 3 % of the total core weight of the intra-granular disintegrant; and (a-v) about 2 % of the total core weight of the intra-granular surfactant; and (b) an extra-granular component comprising: (b-i) about 11 % of the total core weight of the an extra-granular filler; (b-ii) about 2 % of the total core weight of the extra-granular disintegrant; and (b-iii) about 1 % of the total core weight of the extra-granular lubricant.

In some embodiments, the core comprises: (a) an intra-granular component comprising: (a-i) about 20 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii) about 59 % of the total core weight of the intra-granular filler; (a-iii) about 2 % of the total core weight of the intra-granular binder; (a-iv) about 3 % of the total core weight of the intra-granular disintegrant; and (a-v) about 2 % of the total core weight of the intra-granular surfactant; and (b) an extra-granular component comprising: (b-i) about 11 % of the total core weight of the an extra-granular filler; (b-ii) about 2 % of the total core weight of the extra-granular disintegrant; and (b-iii) about 1 % of the total core weight of the extra-granular lubricant.

In some embodiments, the core comprises: (a) an intra-granular component comprising: (a-i) about 20 % of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii) about 59 % of the total core weight of the intra-granular filler; (a-iii) about 2 % of the total core weight of the intra-granular binder; (a-iv) about 3 % of the total core weight of the intra-granular disintegrant; and (a-v) about 2 % of the total core weight of the intra-granular surfactant; and (b) an extra-granular component comprising: (b-i) about 11 % of the total core weight of the an extra-granular filler; (b-ii) about 2 % of the total core weight of the extra-granular disintegrant; and (b-iii) about 1 % of the total core weight of the extra-granular lubricant.

In some embodiments, the intra-granular filler comprises mannitol. In some embodiments, the intra-granular filler comprises microcrystalline cellulose. In some embodiments, the intra-granular filler consists of mannitol and microcrystalline cellulose. In some embodiments, the intra-granular binder is hydroxypropyl cellulose. In some embodiments, the intra-granular disintegrant is croscarmellose sodium. In some embodiments, the intra-granular surfactant is sodium lauryl sulfate. In some embodiments, the extra-granular filler is microcrystalline cellulose. In some embodiments, the extra-granular disintegrant is croscarmellose sodium. In some embodiments, the extra-granular lubricant is magnesium stearate.

In some embodiments, the core comprises: (a) an intra-granular component comprising: (a-i) about 1 % to about 50 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 40 % to about 60 % of the total core weight of mannitol; (a-ii-2) about 10 % to about 30 % of the total core weight of microcrystalline cellulose; (a-iii) about 1 % to about 5 % of the total core weight of hydroxypropyl cellulose; (a-iv) about 1 % to about 5 % of the total core weight of croscarmellose sodium; and (a-v) about 1 % to about 5 % of the total core weight of sodium lauryl sulfate; and (b) an extra-granular component comprising: (b-i) about 5 % to about 15 % of the total core weight of microcrystalline cellulose; (b-ii) about 1 % to about 5 % of the total core weight of croscarmellose sodium; and (b-iii) about 0.1 % to about 2 % of the total core weight of extra-granular lubricant.

In some embodiments, the core comprises: (a) an intra-granular component comprising: (a-i) about 1 % to about 50 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 40 % to about 60 % of the total core weight of mannitol; (a-ii-2) about 10 % to about 30 % of the total core weight of microcrystalline cellulose; (a-iii) about 1 % to about 5 % of the total core weight of hydroxypropyl cellulose; (a-iv) about 1 % to about 5 % of the total core weight of croscarmellose sodium; and (a-v) about 1 % to about 5 % of the total core weight of sodium lauryl sulfate; and (b) an extra-granular component comprising: (b-i) about 5 % to about 15 % of the total core weight of microcrystalline cellulose; (b-ii) about 1 % to about 5 % of the total core weight of croscarmellose sodium; and (b-iii) about 0.1 % to about 2 % of the total core weight of magnesium stearate.

In some embodiments, the core comprises: (a) an intra-granular component comprising: (a-i) about 1 % to about 50 % of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 40 % to about 60 % of the total core weight of mannitol; (a-ii-2) about 10 % to about 30 % of the total core weight of microcrystalline cellulose; (a-iii) about 1 % to about 5 % of the total core weight of hydroxypropyl cellulose; (a-iv) about 1 % to about 5 % of the total core weight of croscarmellose sodium; and (a-v) about 1 % to about 5 % of the total core weight of sodium lauryl sulfate; and (b) an extra-granular component comprising: (b-i) about 5 % to about 15 % of the total core weight of microcrystalline cellulose; (b-ii) about 1 % to about 5 % of the total core weight of croscarmellose sodium; and (b-iii) about 0.1 % to about 2 % of the total core weight of magnesium stearate.

In some embodiments, the core comprises: (a) an intra-granular component comprising: (a-i) about 10 % to about 30 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 40 % to about 50 % of the total core weight of mannitol; (a-ii-2) about 10 % to about 20 % of the total core weight of microcrystalline cellulose; (a-iii) about 1 % to about 3 % of the total core weight of hydroxypropyl cellulose; (a-iv) about 2 % to about 4 % of the total core weight of croscarmellose sodium; and (a-v) about 1 % to about 3 % of the total core weight of sodium lauryl sulfate; and (b) an extra-granular component comprising: (b-i) about 5 % to about 15 % of the total core weight of microcrystalline cellulose; (b-ii) about 1 % to about 3 % of the total core weight of croscarmellose sodium; and (b-iii) about 0.1 % to about 1.5 % of the total core weight of extra-granular lubricant.

In some embodiments, the core comprises: (a) an intra-granular component comprising: (a-i) about 10 % to about 30 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 40 % to about 50 % of the total core weight of mannitol; (a-ii-2) about 10 % to about 20 % of the total core weight of microcrystalline cellulose; (a-iii) about 1 % to about 3 % of the total core weight of hydroxypropyl cellulose; (a-iv) about 2 % to about 4 % of the total core weight of croscarmellose sodium; and (a-v) about 1 % to about 3 % of the total core weight of sodium lauryl sulfate; and (b) an extra-granular component comprising: (b-i) about 5 % to about 15 % of the total core weight of microcrystalline cellulose; (b-ii) about 1 % to about 3 % of the total core weight of croscarmellose sodium; and (b-iii) about 0.1 % to about 1.5 % of the total core weight of magnesium stearate.

In some embodiments, the core comprises: (a) an intra-granular component comprising: (a-i) about 10 % to about 30 % of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 40 % to about 50 % of the total core weight of mannitol; (a-ii-2) about 10 % to about 20 % of the total core weight of microcrystalline cellulose; (a-iii) about 1 % to about 3 % of the total core weight of hydroxypropyl cellulose; (a-iv) about 2 % to about 4 % of the total core weight of croscarmellose sodium; and (a-v) about 1 % to about 3 % of the total core weight of sodium lauryl sulfate; and (b) an extra-granular component comprising: (b-i) about 5 % to about 15 % of the total core weight of microcrystalline cellulose; (b-ii) about 1 % to about 3 % of the total core weight of croscarmellose sodium; and (b-iii) about 0.1 % to about 1.5 % of the total core weight of magnesium stearate.

In some embodiments, the core comprises: (a) an intra-granular component comprising: (a-i) about 1 % by weight to about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 50 % to about 60 % of the total core weight of mannitol; (a-ii-2) about 15 % to about 25 % of the total core weight of microcrystalline cellulose; (a-iii) about 1 % to about 3 % of the total core weight of hydroxypropyl cellulose; (a-iv) about 2 % to about 4 % of the total core weight of croscarmellose sodium; and (a-v) about 1 % to about 3 % of the total core weight of sodium lauryl sulfate; and (b) an extra-granular component comprising: (b-i) about 5 % to about 15 % of the total core weight of microcrystalline cellulose; (b-ii) about 1 % to about 3 % of the total core weight of croscarmellose sodium; and (b-iii) about 0.1 % to about 1.5 % of the total core weight of extra-granular lubricant.

In some embodiments, the core comprises: (a) an intra-granular component comprising: (a-i) about 1 % by weight to about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 50 % to about 60 % of the total core weight of mannitol; (a-ii-2) about 15 % to about 25 % of the total core weight of microcrystalline cellulose; (a-iii) about 1 % to about 3 % of the total core weight of hydroxypropyl cellulose; (a-iv) about 2 % to about 4 % of the total core weight of croscarmellose sodium; and (a-v) about 1 % to about 3 % of the total core weight of sodium lauryl sulfate; and (b) an extra-granular component comprising: (b-i) about 5 % to about 15 % of the total core weight of microcrystalline cellulose; (b-ii) about 1 % to about 3 % of the total core weight of croscarmellose sodium; and (b-iii) about 0.1 % to about 1.5 % of the total core weight of magnesium stearate.

In some embodiments, the core comprises: (a) an intra-granular component comprising: (a-i) about 1 % by weight to about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 50 % to about 60 % of the total core weight of mannitol; (a-ii-2) about 15 % to about 25 % of the total core weight of microcrystalline cellulose; (a-iii) about 1 % to about 3 % of the total core weight of hydroxypropyl cellulose; (a-iv) about 2 % to about 4 % of the total core weight of croscarmellose sodium; and (a-v) about 1 % to about 3 % of the total core weight of sodium lauryl sulfate; and (b) an extra-granular component comprising: (b-i) about 5 % to about 15 % of the total core weight of microcrystalline cellulose; (b-ii) about 1 % to about 3 % of the total core weight of croscarmellose sodium; and (b-iii) about 0.1 % to about 1.5 % of the total core weight of magnesium stearate.

In some embodiments, the core comprises: (a) an intra-granular component comprising: (a-i) about 20 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 44 % of the total core weight of mannitol; (a-ii-2) about 15 % of the total core weight of microcrystalline cellulose; (a-iii) about 2 % of the total core weight of hydroxypropyl cellulose; (a-iv) about 3 % of the total core weight of croscarmellose sodium; and (a-v) about 2 % of the total core weight of sodium lauryl sulfate; and (b) an extra-granular component comprising: (b-i) about 11 % of the total core weight of microcrystalline cellulose; (b-ii) about 2 % of the total core weight of croscarmellose sodium; and (b-iii) about 1 % of the total core weight of extra-granular lubricant.

In some embodiments, the core comprises: (a) an intra-granular component comprising: (a-i) about 20 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 44 % of the total core weight of mannitol; (a-ii-2) about 15 % of the total core weight of microcrystalline cellulose; (a-iii) about 2 % of the total core weight of hydroxypropyl cellulose; (a-iv) about 3 % of the total core weight of croscarmellose sodium; and (a-v) about 2 % of the total core weight of sodium lauryl sulfate; and (b) an extra-granular component comprising: (b-i) about 11 % of the total core weight of microcrystalline cellulose; (b-ii) about 2 % of the total core weight of croscarmellose sodium; and (b-iii) about 1 % of the total core weight of magnesium stearate.

In some embodiments, the core comprises: (a) an intra-granular component comprising: (a-i) about 20 % of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 44 % of the total core weight of mannitol; (a-ii-2) about 15 % of the total core weight of microcrystalline cellulose; (a-iii) about 2 % of the total core weight of hydroxypropyl cellulose; (a-iv) about 3 % of the total core weight of croscarmellose sodium; and (a-v) about 2 % of the total core weight of sodium lauryl sulfate; and (b) an extra-granular component comprising: (b-i) about 11 % of the total core weight of microcrystalline cellulose; (b-ii) about 2 % of the total core weight of croscarmellose sodium; and (b-iii) about 1 % of the total core weight of magnesium stearate.

In some embodiments, the core comprises: (a) an intra-granular component comprising: (a-i) about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 50 % of the total core weight of mannitol; (a-ii-2) about 19 % of the total core weight of microcrystalline cellulose; (a-iii) about 2 % of the total core weight of hydroxypropyl cellulose; (a-iv) about 3 % of the total core weight of croscarmellose sodium; and (a-v) about 2 % of the total core weight of sodium lauryl sulfate; and (b) an extra-granular component comprising: (b-i) about 11 % of the total core weight of microcrystalline cellulose; (b-ii) about 2 % of the total core weight of croscarmellose sodium; and (b-iii) about 1 % of the total core weight of extra-granular lubricant.

In some embodiments, the core comprises: (a) an intra-granular component comprising: (a-i) about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 50 % of the total core weight of mannitol; (a-ii-2) about 19 % of the total core weight of microcrystalline cellulose; (a-iii) about 2 % of the total core weight of hydroxypropyl cellulose; (a-iv) about 3 % of the total core weight of croscarmellose sodium; and (a-v) about 2 % of the total core weight of sodium lauryl sulfate; and (b) an extra-granular component comprising: (b-i) about 11 % of the total core weight of microcrystalline cellulose; (b-ii) about 2 % of the total core weight of croscarmellose sodium; and (b-iii) about 1 % of the total core weight of magnesium stearate.

In some embodiments, the core comprises: (a) an intra-granular component comprising: (a-i) about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 50 % of the total core weight of mannitol; (a-ii-2) about 19 % of the total core weight of microcrystalline cellulose; (a-iii) about 2 % of the total core weight of hydroxypropyl cellulose; (a-iv) about 3 % of the total core weight of croscarmellose sodium; and (a-v) about 2 % of the total core weight of sodium lauryl sulfate; and (b) an extra-granular component comprising: (b-i) about 11 % of the total core weight of microcrystalline cellulose; (b-ii) about 2 % of the total core weight of croscarmellose sodium; and (b-iii) about 1 % of the total core weight of magnesium stearate.

In some embodiments, the core comprises: (a) an intra-granular component comprising: (a-i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 54 % by weight of mannitol; (a-ii-2) about 20 % by weight of microcrystalline cellulose; (a-iii) about 2 % by weight of hydroxypropyl cellulose; (a-iv) about 3 % by weight of croscarmellose sodium; and (a-v) about 2 % by weight of sodium lauryl sulfate; and (b) an extra-granular component comprising: (b-i) about 11 % by weight of microcrystalline cellulose; (b-ii) about 2 % by weight of croscarmellose sodium; and (b-iii) about 1 % by weight of extra-granular lubricant.

In some embodiments, the core comprises: (a) an intra-granular component comprising: (a-i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 54 % by weight of mannitol; (a-ii-2) about 20 % by weight of microcrystalline cellulose; (a-iii) about 2 % by weight of hydroxypropyl cellulose; (a-iv) about 3 % by weight of croscarmellose sodium; and (a-v) about 2 % by weight of sodium lauryl sulfate; and (b) an extra-granular component comprising: (b-i) about 11 % by weight of microcrystalline cellulose; (b-ii) about 2 % by weight of croscarmellose sodium; and (b-iii) about 1 % by weight of magnesium stearate.

In some embodiments, the core comprises: (a) an intra-granular component comprising: (a-i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 54 % by weight of mannitol; (a-ii-2) about 20 % by weight of microcrystalline cellulose; (a-iii) about 2 % by weight of hydroxypropyl cellulose; (a-iv) about 3 % by weight of croscarmellose sodium; and (a-v) about 2 % by weight of sodium lauryl sulfate; and (b) an extra-granular component comprising: (b-i) about 11 % by weight of microcrystalline cellulose; (b-ii) about 2 % by weight of croscarmellose sodium; and (b-iii) about 1 % by weight of magnesium stearate.

In some embodiments, the tablet comprises a coating layer comprising a coating agent. In some embodiments, the coating agent is selected from the group consisting of Opadry QX White 21A180025, Opadry I, and Opadry II. In some embodiments, the coating agent is Opadry QX White 21A180025. In some embodiments, the tablet comprises about 0.5 % to about 10 % of the total core weight of coating agent. In some embodiments, the tablet comprises about 1 % to about 5 % of the total core weight of coating agent. In some embodiments, the tablet comprises about 2 % to about 4 % of the total core weight of coating agent. In some embodiments, the tablet comprises about 3 % of the total core weight of coating agent. In some embodiments, the tablet comprises about 0.5 % to about 10 % of the total core weight of Opadry QX White 21A180025. In some embodiments, the tablet comprises about 1 % to about 5 % of the total core weight of Opadry QX White 21A180025. In some embodiments, the tablet comprises about 2 % to about 4 % of the total core weight of Opadry QX White 21A180025. In some embodiments, the tablet comprises about 3 % of the total core weight of Opadry QX White 21A180025.

In some embodiments, the total core weight is about 50 mg. In some embodiments, the core comprises: (a) an intra-granular component comprising: (a-i) about 2.5 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 27 mg of the total core weight of mannitol; (a-ii-2) about 10 mg of the total core weight of microcrystalline cellulose; (a-iii) about 1 mg of the total core weight of hydroxypropyl cellulose; (a-iv) about 1.5 mg of the total core weight of croscarmellose sodium; and (a-v) about 1 mg of the total core weight of sodium lauryl sulfate; and (b) an extra-granular component comprising: (b-i) about 5.5 mg of the total core weight of microcrystalline cellulose; (b-ii) about 1 mg of the total core weight of croscarmellose sodium; and (b-iii) about 0.5 mg of the total core weight of extra-granular lubricant. In some embodiments, the core comprises: (a) an intra-granular component comprising: (a-i) about 2.5 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 27 mg of the total core weight of mannitol; (a-ii-2) about 10 mg of the total core weight of microcrystalline cellulose; (a-iii) about 1 mg of the total core weight of hydroxypropyl cellulose; (a-iv) about 1.5 mg of the total core weight of croscarmellose sodium; and (a-v) about 1 mg of the total core weight of sodium lauryl sulfate; and (b) an extra-granular component comprising: (b-i) about 5.5 mg of the total core weight of microcrystalline cellulose; (b-ii) about 1 mg of the total core weight of croscarmellose sodium; and (b-iii) about 0.5 mg of the total core weight of magnesium stearate. In some embodiments, the core comprises: (a) an intra-granular component comprising: (a-i) about 2.5 mg of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 27 mg of the total core weight of mannitol; (a-ii-2) about 10 mg of the total core weight of microcrystalline cellulose; (a-iii) about 1 mg of the total core weight of hydroxypropyl cellulose; (a-iv) about 1.5 mg of the total core weight of croscarmellose sodium; and (a-v) about 1 mg of the total core weight of sodium lauryl sulfate; and (b) an extra-granular component comprising: (b-i) about 5.5 mg of the total core weight of microcrystalline cellulose; (b-ii) about 1 mg of the total core weight of croscarmellose sodium; and (b-iii) about 0.5 mg of the total core weight of magnesium stearate. In some embodiments, the tablet comprises about 1.5 mg of coating agent. In some embodiments, the coating agent is Opadry QX White 21A180025.

In some embodiments, the total core weight is about 100 mg. In some embodiments, the core comprises: (a) an intra-granular component comprising: (a-i) about 20 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 44 mg of the total core weight of mannitol; (a-ii-2) about 15 mg of the total core weight of microcrystalline cellulose; (a-iii) about 2 mg of the total core weight of hydroxypropyl cellulose; (a-iv) about 3 mg of the total core weight of croscarmellose sodium; and (a-v) about 2 mg of the total core weight of sodium lauryl sulfate; and (b) an extra-granular component comprising: (b-i) about 11 mg of the total core weight of microcrystalline cellulose; (b-ii) about 2 mg of the total core weight of croscarmellose sodium; and (b-iii) about 1 mg of the total core weight of extra-granular lubricant. In some embodiments, the core comprises: (a) an intra-granular component comprising: (a-i) about 20 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 44 mg of the total core weight of mannitol; (a-ii-2) about 15 mg of the total core weight of microcrystalline cellulose; (a-iii) about 2 mg of the total core weight of hydroxypropyl cellulose; (a-iv) about 3 mg of the total core weight of croscarmellose sodium; and (a-v) about 2 mg of the total core weight of sodium lauryl sulfate; and (b) an extra-granular component comprising: (b-i) about 11 mg of the total core weight of microcrystalline cellulose; (b-ii) about 2 mg of the total core weight of croscarmellose sodium; and (b-iii) about 1 mg of the total core weight of magnesium stearate. In some embodiments, the core comprises: (a) an intra-granular component comprising: (a-i) about 20 mg of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 44 mg of the total core weight of mannitol; (a-ii-2) about 15 mg of the total core weight of microcrystalline cellulose; (a-iii) about 2 mg of the total core weight of hydroxypropyl cellulose; (a-iv) about 3 mg of the total core weight of croscarmellose sodium; and (a-v) about 2 mg of the total core weight of sodium lauryl sulfate; and (b) an extra-granular component comprising: (b-i) about 11 mg of the total core weight of microcrystalline cellulose; (b-ii) about 2 mg of the total core weight of croscarmellose sodium; and (b-iii) about 1 mg of the total core weight of magnesium stearate. In some embodiments, the core comprises: (a) an intra-granular component comprising: (a-i) about 5 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 54 mg of mannitol; (a-ii-2) about 20 mg of microcrystalline cellulose; (a-iii) about 2 mg of hydroxypropyl cellulose; (a-iv) about 3 mg of croscarmellose sodium; and (a-v) about 2 mg of sodium lauryl sulfate; and (b) an extra-granular component comprising: (b-i) about 11 mg of microcrystalline cellulose; (b-ii) about 2 mg of croscarmellose sodium; and (b-iii) about 1 mg of extra-granular lubricant. In some embodiments, the core comprises: (a) an intra-granular component comprising: (a-i) about 5 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 54 mg of mannitol; (a-ii-2) about 20 mg of microcrystalline cellulose; (a-iii) about 2 mg of hydroxypropyl cellulose; (a-iv) about 3 mg of croscarmellose sodium; and (a-v) about 2 mg of sodium lauryl sulfate; and (b) an extra-granular component comprising: (b-i) about 11 mg of microcrystalline cellulose; (b-ii) about 2 mg of croscarmellose sodium; and (b-iii) about 1 mg of magnesium stearate. In some embodiments, the core comprises: (a) an intra-granular component comprising: (a-i) about 5 mg of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 54 mg of mannitol; (a-ii-2) about 20 mg of microcrystalline cellulose; (a-iii) about 2 mg of hydroxypropyl cellulose; (a-iv) about 3 mg of croscarmellose sodium; and (a-v) about 2 mg of sodium lauryl sulfate; and (b) an extra-granular component comprising: (b-i) about 11 mg of microcrystalline cellulose; (b-ii) about 2 mg of croscarmellose sodium; and (b-iii) about 1 mg of magnesium stearate. In some embodiments, the tablet comprises about 3 mg of coating agent. In some embodiments, the coating agent is Opadry QX White 2 1 A 180025.

In some embodiments, the total core weight is about 200 mg. In some embodiments, the core comprises: (a) an intra-granular component comprising: (a-i) about 40 mg of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 88 mg of the total core weight of mannitol; (a-ii-2) about 30 mg of the total core weight of microcrystalline cellulose; (a-iii) about 4 mg of the total core weight of hydroxypropyl cellulose; (a-iv) about 6 mg of the total core weight of croscarmellose sodium; and (a-v) about 4 mg of the total core weight of sodium lauryl sulfate; and (b) an extra-granular component comprising: (b-i) about 22 mg of the total core weight of microcrystalline cellulose; (b-ii) about 4 mg of the total core weight of croscarmellose sodium; and (b-iii) about 2 mg of the total core weight of extra-granular lubricant. In some embodiments, the core comprises: (a) an intra-granular component comprising: (a-i) about 40 mg of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 88 mg of the total core weight of mannitol; (a-ii-2) about 30 mg of the total core weight of microcrystalline cellulose; (a-iii) about 4 mg of the total core weight of hydroxypropyl cellulose; (a-iv) about 6 mg of the total core weight of croscarmellose sodium; and (a-v) about 4 mg of the total core weight of sodium lauryl sulfate; and (b) an extra-granular component comprising: (b-i) about 22 mg of the total core weight of microcrystalline cellulose; (b-ii) about 4 mg of the total core weight of croscarmellose sodium; and (b-iii) about 2 mg of the total core weight of magnesium stearate. In some embodiments, the core comprises: (a) an intra-granular component comprising: (a-i) about 40 mg of the total core weight of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 88 mg of the total core weight of mannitol; (a-ii-2) about 30 mg of the total core weight of microcrystalline cellulose; (a-iii) about 4 mg of the total core weight of hydroxypropyl cellulose; (a-iv) about 6 mg of the total core weight of croscarmellose sodium; and (a-v) about 4 mg of the total core weight of sodium lauryl sulfate; and (b) an extra-granular component comprising: (b-i) about 22 mg of the total core weight of microcrystalline cellulose; (b-ii) about 4 mg of the total core weight of croscarmellose sodium; and (b-iii) about 2 mg of the total core weight of magnesium stearate. In some embodiments, the core comprises: (a) an intra-granular component comprising: (a-i) about 10 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 108 mg of mannitol; (a-ii-2) about 40 mg of microcrystalline cellulose; (a-iii) about 4 mg of hydroxypropyl cellulose; (a-iv) about 6 mg of croscarmellose sodium; and (a-v) about 4 mg of sodium lauryl sulfate; and (b) an extra-granular component comprising: (b-i) about 22 mg of microcrystalline cellulose; (b-ii) about 4 mg of croscarmellose sodium; and (b-iii) about 2 mg of extra-granular lubricant. In some embodiments, the core comprises: (a) an intra-granular component comprising: (a-i) about 10 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 108 mg of mannitol; (a-ii-2) about 40 mg of microcrystalline cellulose; (a-iii) about 4 mg of hydroxypropyl cellulose; (a-iv) about 6 mg of croscarmellose sodium; and (a-v) about 4 mg of sodium lauryl sulfate; and (b) an extra-granular component comprising: (b-i) about 22 mg of microcrystalline cellulose; (b-ii) about 4 mg of croscarmellose sodium; and (b-iii) about 2 mg of magnesium stearate. In some embodiments, the core comprises: (a) an intra-granular component comprising: (a-i) about 10 mg of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 108 mg of mannitol; (a-ii-2) about 40 mg of microcrystalline cellulose; (a-iii) about 4 mg of hydroxypropyl cellulose; (a-iv) about 6 mg of croscarmellose sodium; and (a-v) about 4 mg of sodium lauryl sulfate; and (b) an extra-granular component comprising: (b-i) about 22 mg of microcrystalline cellulose; (b-ii) about 4 mg of croscarmellose sodium; and (b-iii) about 2 mg of magnesium stearate. In some embodiments, the tablet comprises about 6 mg of coating agent. In some embodiments, the coating agent is Opadry QX White 2 1 A 180025.

In some embodiments, the total core weight is about 70 mg. In some embodiments, the core comprises: (a) an intra-granular component comprising: (a-i) about 7 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 35 mg of mannitol; (a-ii-2) about 13.3 mg of microcrystalline cellulose; (a-iii) about 1.4 mg of hydroxypropyl cellulose; (a-iv) about 2.1 mg of croscarmellose sodium; and (a-v) about 1.4 mg of sodium lauryl sulfate; and (b) an extra-granular component comprising: (b-i) about 7.7 mg of microcrystalline cellulose; (b-ii) about 1.4 mg of croscarmellose sodium; and (b-iii) about 0.7 mg of extra-granular lubricant. In some embodiments, the core comprises: (a) an intra-granular component comprising: (a-i) about 7 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 35 mg of mannitol; (a-ii-2) about 13.3 mg of microcrystalline cellulose; (a-iii) about 1.4 mg of hydroxypropyl cellulose; (a-iv) about 2.1 mg of croscarmellose sodium; and (a-v) about 1.4 mg of magnesium stearate; and (b) an extra-granular component comprising: (b-i) about 7.7 mg of microcrystalline cellulose; (b-ii) about 1.4 mg of croscarmellose sodium; and (b-iii) about 0.7 mg of extra-granular lubricant. In some embodiments, the core comprises: (a) an intra-granular component comprising: (a-i) about 7 mg of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 35 mg of mannitol; (a-ii-2) about 13.3 mg of microcrystalline cellulose; (a-iii) about 1.4 mg of hydroxypropyl cellulose; (a-iv) about 2.1 mg of croscarmellose sodium; and (a-v) about 1.4 mg of magnesium stearate; and (b) an extra-granular component comprising: (b-i) about 7.7 mg of microcrystalline cellulose; (b-ii) about 1.4 mg of croscarmellose sodium; and (b-iii) about 0.7 mg of extra-granular lubricant. In some embodiments, the tablet comprises about 2.1 mg of coating agent. In some embodiments, the coating agent is Opadry QX White 21A180025.

In some embodiments, the total core weight is about 400 mg. In some embodiments, the core comprises: (a) an intra-granular component comprising: (a-i) about 40 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 200 mg of mannitol; (a-ii-2) about 76 mg of microcrystalline cellulose; (a-iii) about 8 mg of hydroxypropyl cellulose; (a-iv) about 12 mg of croscarmellose sodium; and (a-v) about 8 mg of sodium lauryl sulfate; and (b) an extra-granular component comprising: (b-i) about 44 mg of microcrystalline cellulose; (b-ii) about 8 mg of croscarmellose sodium; and (b-iii) about 4 mg of extra-granular lubricant. In some embodiments, the core comprises: (a) an intra-granular component comprising: (a-i) about 40 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof; (a-ii-1) about 200 mg of mannitol; (a-ii-2) about 76 mg of microcrystalline cellulose; (a-iii) about 8 mg of hydroxypropyl cellulose; (a-iv) about 12 mg of croscarmellose sodium; and (a-v) about 8 mg of sodium lauryl sulfate; and (b) an extra-granular component comprising: (b-i) about 44 mg of microcrystalline cellulose; (b-ii) about 8 mg of croscarmellose sodium; and (b-iii) about 4 mg of magnesium stearate. In some embodiments, the core comprises: (a) an intra-granular component comprising: (a-i) about 40 mg of the (R)-N-(5-(5-ethyl-1,2,4-oxadiazol-3-yl)-2,3-dihydro-1H-inden-1-yl)-1-methyl-1H-pyrazole-4-carboxamide; (a-ii-1) about 200 mg of mannitol; (a-ii-2) about 76 mg of microcrystalline cellulose; (a-iii) about 8 mg of hydroxypropyl cellulose; (a-iv) about 12 mg of croscarmellose sodium; and (a-v) about 8 mg of sodium lauryl sulfate; and (b) an extra-granular component comprising: (b-i) about 44 mg of microcrystalline cellulose; (b-ii) about 8 mg of croscarmellose sodium; and (b-iii) about 4 mg of magnesium stearate. In some embodiments, the tablet comprises about 12 mg of coating agent. In some embodiments, the coating agent is Opadry QX White 21A180025.

### Fillers

Fillers are substances that can be added to components of a pharmaceutical composition or formulation to increase bulk weight of the material to be formulated, e.g. tabletted, in order to achieve the desired weight. Fillers include but are not limited to powdered cellulose, microcrystalline cellulose, silicified microcrystalline cellulose, kaolin, corn starch, maize starch, starch derivatives, pregelatinized starch, calcium phosphate, calcium hydrogen phosphate, dicalcium phosphate, tricalcium phosphate, compressible sugar, sugar alcohol, mannitol, sorbitol, maltitol, xylitol, lactitol, lactose, dextrose, maltose, sucrose, glucose, fructose, saccharose, raffinose, dextrates, trehalose, maltodextrines, and the like. In some embodiments, the filler is selected from the group consisting of powdered cellulose, microcrystalline cellulose, silicified microcrystalline cellulose, kaolin, corn starch, maize starch, starch derivatives, pregelatinized starch, calcium phosphate, calcium hydrogen phosphate, dicalcium phosphate, tricalcium phosphate, compressible sugar, sugar alcohol, mannitol, sorbitol, maltitol, xylitol, lactitol, lactose, dextrose, maltose, sucrose, glucose, fructose, saccharose, raffinose, dextrates, trehalose, maltodextrines, and mixtures of any of the foregoing. In some embodiments, the filler is selected from the group consisting of microcrystalline cellulose, mannitol, and mixtures of any of the foregoing. In some embodiments, the filler comprises mannitol. In some embodiments, the filler comprises microcrystalline cellulose. In some embodiments, the filler comprises microcrystalline cellulose and mannitol. *Binders*

Binders are substances that can be added to components of a pharmaceutical composition or formulation to act as an adhesive to bind together powders, granules, and other dry ingredients comprised in the pharmaceutical composition or formulation and impart to the pharmaceutical composition or formulation the desired mechanical strength. Binders include, but are not limited to, arabic gum, acacia gum, alginate, alginic acid, corn starch, copolyvidone, polyvinylpyrrolidone, gelatin, glyceryl behenate, hydroxyethylcellulose, hydroxypropyl cellulose, carboxymethylcellulose, carboxymethylcellulose calcium, carboxymethylcellulose sodium, methylcellulose, hypromellose, lactose, polyvinyl alcohol, povidone, polyethylene oxide, polyacrylates, potato starch, pregelatinized starch, sodium alginate, sodium starch, sodium carboxy methyl cellulose, starch, and the likes. In some embodiments, the binder is selected from the group consisting of arabic gum, acacia gum, alginate, alginic acid, corn starch, copolyvidone, polyvinylpyrrolidone, gelatin, glyceryl behenate, hydroxyethylcellulose, hydroxypropyl cellulose, carboxymethylcellulose, carboxymethylcellulose calcium, carboxymethylcellulose sodium, methylcellulose, hypromellose, lactose, polyvinyl alcohol, povidone, polyethylene oxide, polyacrylates, potato starch, pregelatinized starch, sodium alginate, sodium starch, sodium carboxy methyl cellulose, starch, and mixtures of any of the foregoing. In some embodiments, the binder is selected from the group consisting of hydroxyethylcellulose, hydroxypropyl cellulose, carboxymethylcellulose, carboxymethylcellulose calcium, carboxymethylcellulose sodium, methylcellulose, hypromellose, sodium carboxy methyl cellulose, and mixtures of any of the foregoing. In some embodiments, the binder is hydroxypropyl cellulose.

### Disintegrants

Disintegrants are substances that can be added to components of a pharmaceutical composition or formulation to aid their deaggregation. Disintegrants can be formulated to provide rapid breakage of solid pharmaceutical compositions or formulations when they come into contact with moisture. Disintegrants include, but are not limited to, alginic acid, croscarmellose sodium, cellulose, carboxymethylcellulose calcium, carboxymethylcellulose sodium, microcrystalline cellulose, crospovidone, sodium starch glycolate, low-substituted hydroxypropyl cellulose, polacrillin potassium, pregelatinized starch, partially hydrolyzed starch, sodium carboxymethyl starch, starch, sodium alginate, sodium carboxy methyl cellulose, and mixtures of any of the foregoing. In some embodiments, the disintegrant is selected from the group consisting of alginic acid, croscarmellose sodium, cellulose, carboxymethylcellulose calcium, carboxymethylcellulose sodium, microcrystalline cellulose, crospovidone, sodium starch glycolate, low-substituted hydroxypropyl cellulose, polacrillin potassium, pregelatinized starch, partially hydrolyzed starch, sodium carboxymethyl starch, starch, sodium alginate, sodium carboxy methyl cellulose, and mixtures of any of the foregoing. In some embodiments, the disintegrant is selected from the group consisting of croscarmellose sodium, cellulose, carboxymethylcellulose calcium, carboxymethylcellulose sodium, microcrystalline cellulose, low-substituted hydroxypropyl cellulose, and mixtures of any of the foregoing. In some embodiments, the disintegrant is croscarmellose sodium.

### Surfactants

Surfactants are substances that can be added to components of a pharmaceutical composition or formulation to lower the surface tension between two liquids or between a solid and a liquid and increase solubility. Surfactants include, but are not limited to, cetylpyridine chloride, heptadecaethylene oxycetanol, lecithins, polyoxyethylene stearate, nonoxynol 9, nonoxynol 10, octoxynol 9, sorbitan fatty acid esters, Span 20, Span 40, Span 60, Span 80, Span S5, polysorbates, polysorbate 20, polysorbate 21, polysorbate 40, polysorbate 60, polysorbate 61, polysorbate 65, polysorbate 80, sodium salts of fatty alcohol sulfates, sodium lauryl sulfate, sodium salts of sulfosuccinates, sodium dioctylsulfosuccinate, partial esters of fatty acids with alcohols, glycerine monostearate, glyceryl monooleate, ethers of fatty alcohols with polyoxyethylene, esters of fatty acids with polyoxyethylene, copolymers of ethylenoxide and propylenoxide (Pluronic^{®}), benzalkonium chloride, ethoxylated triglycerides, and the likes. In some embodiments, the surfactant is selected from the group consisting of sodium salts of fatty alcohol sulfates, sodium lauryl sulfate, sodium dodecyl sulfates, and mixtures of any of the foregoing. In some embodiments, the surfactant is sodium lauryl sulfate.

### Lubricants

Lubricants are substances that can be added to components of the present compositions to reduce sticking by a solid formulation to the equipment used for production of a unit doss form. Lubricants include, but are not limited to, hydrogenated castor oil, magnesium stearate, glyceryl monostearate, calcium stearate, glyceryl behenate, glycerol distearate, glyceryl dipalmitostearate, behenoyl polyoxyl-8 glycerides, sodium stearyl fumarate, stearic acid, talc, zinc stearate, mineral oil, polyethylene glycol, polaxamer, sodium lauryl sulfate, and the likes. In some embodiments, the lubricant is selected from the group consisting of hydrogenated castor oil, magnesium stearate, calcium stearate, zinc stearate, and mixtures of any of the foregoing. In some embodiments, the lubricant is magnesium stearate.

### Coating Agents

Coating agents are substances that can be added as an outer layer surrounding components of a pharmaceutical composition or formulation. Surfactants include, but are not limited to, Opadry QX White 21A180025, Opadry I, Opadry II, and the like. In some embodiments, the coating agent is selected from the group consisting of Opadry QX White 21A180025, Opadry I, and Opadry II. In some embodiments, the coating agent is Opadry QX White 21A180025.

### Additional Agents

Pharmaceutical compositions or formulations intended for the preparation of oral dosage forms (such as tablets and capsules) may further contain one or more agents selected from the group consisting of sweetening agents, flavoring agents, coloring agents, and preserving agents in order to provide pharmaceutically elegant and palatable preparations.

### Processes

Also provided herein is a process for making a tablet described herein, wherein the process comprises: (1) preparing granules comprising (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof, the intra-granular filler, the intra-granular binder, the intra-granular disintegrant, and the intra-granular surfactant; (2) milling the granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant to form the intra-granular component; (3) blending the intra-granular component with the extra-granular filler, the extra-granular disintegrant, and the extra-granular lubricant to form a final blend mixture; (4) compressing the final blend mixture to form the core; (5) optionally coating the core with the coating layer.

In some embodiments, preparing granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant comprises a wet granulation step. In some embodiments, the wet granulation step is performed with a high shear granulator. In some embodiments, the wet granulation step is performed with a fluid bed granulator. In some embodiments, the wet granulation step is performed with a granulator drum. In some embodiments, the wet granulation step further comprises a wet milling step. In some embodiments, the wet milling step is performed with a conical mill. In some embodiments, the wet milling step is performed with a turbo mill or hammer mill. In some embodiments, the preparing granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant further comprises drying the granules. In some embodiments, drying the granules is performed in a fluid bed dryer. In some embodiments, drying the granules is performed in a tray dryer. In some embodiments, drying the granules is performed in a belt dryer. In some embodiments, drying the granules is performed in a vacuum tray dryer. In some embodiments, drying the granules is performed in a rotary dryer.

In some embodiments, milling the granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant is performed with a conical mill. In some embodiments, milling the granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant is performed with a turbo mill or hammer mill.

In some embodiments, blending the intra-granular component with the extra-granular filler, the extra-granular disintegrant, and the extra-granular lubricant is performed in a V-blender. In some embodiments, blending the intra-granular component with the extra-granular filler, the extra-granular disintegrant, and the extra-granular lubricant is performed in an octagonal blender. In some embodiments, blending the intra-granular component with the extra-granular filler, the extra-granular disintegrant, and the extra-granular lubricant is performed in a mass blender. In some embodiments, blending the intra-granular component with the extra-granular filler, the extra-granular disintegrant, and the extra-granular lubricant is performed in a double cone blender. In some embodiments, blending the intra-granular component with the extra-granular filler, the extra-granular disintegrant, and the extra-granular lubricant is performed in a vertical blender.

In some embodiments, compressing the final blend mixture to form the core is performed with a rotary tablet press. In some embodiments, compressing the final blend mixture to form the core is performed with a single punch tablet press.

In some embodiments, the process comprises coating the core with the coating layer. In some embodiments, coating the core with the coating layer is performed using a conventional coating pan, such as a Pelligrini pan, an immersion sword type pan, or an immersion tube type pan. In some embodiments, coating the core with the coating layer is performed using a perforated coating pan, such as an Accela-Cota pan, a hi-coater, a dria coater, a Glatt pan-coating equipment, a Huttlin butterfly pan, or a Dumoulin Coating equipment.

In some embodiments, provided is a process for making a tablet described herein, wherein the process comprises: (1) preparing granules comprising (R)-N-(5-(5-ethyl-1,2,4-oxadiazol-3-yl)-2,3-dihydro-1H-inden-1-yl)-1-methyl-1H-pyrazole-4-carboxamide, the intra-granular filler, the intra-granular binder, the intra-granular disintegrant, and the intra-granular surfactant; (2) milling the granules comprising (R)-N-(5-(5-ethyl-1,2,4-oxadiazol-3-yl)-2,3-dihydro-1H-inden-1-yl)-1-methyl-1H-pyrazole-4-carboxamide, the intra-granular filler, the intra-granular binder, the intra-granular disintegrant, and the intra-granular surfactant to form the intra-granular component; (3) blending the intra-granular component with the extra-granular filler, the extra-granular disintegrant, and the extra-granular lubricant to form a final blend mixture; (4) compressing the final blend mixture to form the core; (5) optionally coating the core with the coating layer.

In some embodiments, preparing granules comprising (R)-N-(5-(5-ethyl-1,2,4-oxadiazol-3-yl)-2,3-dihydro-1H-inden-1-yl)-1-methyl-1H-pyrazole-4-carboxamide, the intra-granular filler, the intra-granular binder, the intra-granular disintegrant, and the intra-granular surfactant comprises a wet granulation step. In some embodiments, the wet granulation step is performed with a high shear granulator. In some embodiments, the wet granulation step is performed with a fluid bed granulator. In some embodiments, the wet granulation step is performed with a granulator drum. In some embodiments, the wet granulation step further comprises a wet milling step. In some embodiments, the wet milling step is performed with a conical mill. In some embodiments, the wet milling step is performed with a turbo mill or hammer mill. In some embodiments, the preparing granules comprising (R)-N-(5-(5-ethyl-1,2,4-oxadiazol-3-yl)-2,3-dihydro-1H-inden-1-yl)-1-methyl-1H-pyrazole-4-carboxamide, the intra-granular filler, the intra-granular binder, the intra-granular disintegrant, and the intra-granular surfactant further comprises drying the granules. In some embodiments, drying the granules is performed in a fluid bed dryer. In some embodiments, drying the granules is performed in a tray dryer. In some embodiments, drying the granules is performed in a belt dryer. In some embodiments, drying the granules is performed in a vacuum tray dryer. In some embodiments, drying the granules is performed in a rotary dryer.

In some embodiments, milling the granules comprising (R)-N-(5-(5-ethyl-1,2,4-oxadiazol-3-yl)-2,3-dihydro-1H-inden-1-yl)-1-methyl-1H-pyrazole-4-carboxamide, the intra-granular filler, the intra-granular binder, the intra-granular disintegrant, and the intra-granular surfactant is performed with a conical mill. In some embodiments, milling the granules comprising (R)-N-(5-(5-ethyl-1,2,4-oxadiazol-3-yl)-2,3-dihydro-1H-inden-1-yl)-1-methyl-1H-pyrazole-4-carboxamide, the intra-granular filler, the intra-granular binder, the intra-granular disintegrant, and the intra-granular surfactant is performed with a turbo mill or hammer mill.

In some embodiments, blending the intra-granular component with the extra-granular filler, the extra-granular disintegrant, and the extra-granular lubricant is performed in a V-blender. In some embodiments, blending the intra-granular component with the extra-granular filler, the extra-granular disintegrant, and the extra-granular lubricant is performed in an octagonal blender. In some embodiments, blending the intra-granular component with the extra-granular filler, the extra-granular disintegrant, and the extra-granular lubricant is performed in a mass blender. In some embodiments, blending the intra-granular component with the extra-granular filler, the extra-granular disintegrant, and the extra-granular lubricant is performed in a double cone blender. In some embodiments, blending the intra-granular component with the extra-granular filler, the extra-granular disintegrant, and the extra-granular lubricant is performed in a vertical blender.

In some embodiments, compressing the final blend mixture to form the core is performed with a rotary tablet press. In some embodiments, compressing the final blend mixture to form the core is performed with a single punch tablet press.

In some embodiments, the process comprises coating the core with the coating layer. In some embodiments, coating the core with the coating layer is performed using a conventional coating pan, such as a Pelligrini pan, an immersion sword type pan, or an immersion tube type pan. In some embodiments, coating the core with the coating layer is performed using a perforated coating pan, such as an Accela-Cota pan, a hi-coater, a dria coater, a Glatt pan-coating equipment, a Huttlin butterfly pan, or a Dumoulin Coating equipment.

The formulation or tablet described and/or disclosed herein may be used to treat or prevent a disease or condition in a subject.

Without being bound by theory, (R)-N-(5-(5-ethyl-1,2,4-oxadiazol-3-yl)-2,3-dihydro-1H-inden-1-yl)-1-methyl-1H-pyrazole-4-carboxamide is believed to act by inhibiting myosin. This inhibition potentially decreases the number of independent myosin heads interacting with actin filaments reducing the amount of contraction. Reducing contraction of cardiac muscle can be important for the treatment of heart diseases in which over-contraction is an issue. A formulation or tablet as described herein may be used in methods of treating or preventing heart disease in a subject, comprising administering to the subject in need thereof said formulation or tablet. A formulation or tablet as described herein may be used in methods of treating or preventing heart disease in a subject in need thereof comprising administering to the subject a therapeutically effective amount of said formulation or tablet. In some embodiments, provided is a formulation or tablet as described herein for use in methods of treating heart disease in a subject in need thereof comprising administering to the subject a therapeutically effective amount of said formulation or tablet. In some embodiments, provided is a formulation or tablet as described herein for use in methods of treating an established or diagnosed heart disease in a subject in need thereof comprising administering to the subject a therapeutically effective amount of said formulation or tablet. A formulation or tablet as described herein may be used in methods of preventing heart disease in a subject in need thereof comprising administering to the subject a therapeutically effective amount of said formulation or tablet.

Also provided herein is the use of a formulation or tablet as described herein in the manufacture of a medicament for treatment of a heart disease in a subject. A formulation or tablet as described herein may be used in a method of treatment of the human or animal body by therapy. A formulation or tablet as described herein, may be used in a method of treatment of the human or animal body by therapy. A formulation or tablet as described herein may be used in treating or preventing heart disease. In some embodiments, provided herein is a formulation or tablet as described herein, for use in treating heart disease. In some embodiments, provided herein is a formulation or tablet as described herein, for use in treating an established or diagnosed heart disease. A formulation or tablet as described herein may be used in preventing heart disease. A formulation or tablet as described herein may be used in treating a disease or condition associated with HCM. A formulation or tablet as described herein may be used in treating a disease or condition associated with secondary left ventricular wall thickening. A formulation or tablet as described herein may be used in ameliorating a symptom associated with heart disease. A formulation or tablet as described herein may be used in reducing the risk of a symptom associated with heart disease. A formulation or tablet as described herein may be used in treating a disease or condition associated with small left ventricular cavity, cavity obliteration, hyperdynamic left ventricular contraction, obstruction of blood flow out of the left ventricle, cardiac hypertrophy, small cardiac stroke volume, impaired relaxation of the left ventricle, high left ventricle filling pressure, myocardial ischemia, or cardiac fibrosis. A formulation or tablet as described herein may be used in treating a disease or condition associated with small left ventricular cavity and cavity obliteration, hyperdynamic left ventricular contraction, myocardial ischemia, or cardiac fibrosis. A formulation or tablet as described herein may be used in treating muscular dystrophies. A formulation or tablet as described herein may be used in treating a glycogen storage disease. A formulation or tablet as described herein may be used in modulating the cardiac sarcomere, such as inhibiting the cardiac sarcomere. A formulation or tablet as described herein may be used in potentiating cardiac myosin.

In some embodiments, the subject is a mammal. In some embodiments, the subject is a mouse, rat, dog, cat, pig, sheep, horse, cow, or human. In some embodiments, the subject is a human. In some embodiments, the subject has an established or diagnosed heart disease. In some embodiments, the subject has established or diagnosed hypertrophic cardiomyopathy (HCM). In some embodiments, the subject is at risk for developing heart disease. In some embodiments, the subject has a mutation that increases risk for heart disease. In some embodiments, the subject has a mutation that increases risk for hypertrophic cardiomyopathy (HCM). In some embodiments, the mutation is a sarcomeric mutation. In some embodiments, the mutation is a mutation in myosin heavy chain β (MHC-β), cardiac muscle troponin T (cTnT), tropomyosin alpha-1 chain (TPM1), myosin-binding protein C cardiac-type (MYBPC3), cardiac troponin I (cTnI), myosin essential light chain (ELC), titin (TTN), myosin regulatory light chain 2 ventricular/cardiac muscle isoform (MLC-2), cardiac muscle alpha actin, muscle LIM protein (MLP), or protein kinase AMP-activated non-catalytic subunit gamma 2 (PRKAG2). In some embodiments, the mutation is a mutation in MHC-β. In some embodiments, the subject has established or diagnosed hypertrophic cardiomyopathy without a confirmed genetic etiology.

In some embodiments, the subject has a high risk of progressive symptoms. In some embodiments, the subject has a high risk of atrial fibrillation, ventricular tachyarrhythmias, stroke, and/or sudden death. In some embodiments, the subject has a reduced exercise capacity. In some embodiments, the reduced exercise capacity is as compared to an age-matched control population. In some embodiments, the subject is eligible for surgical intervention or percutaneous ablation to treat the heart disease.

In some embodiments, the heart disease is hypertrophic cardiomyopathy (HCM). In some embodiments, the heart disease is obstructive HCM. In some embodiments, the heart disease is nonobstructive HCM. In some embodiments, the HCM is associated with a sarcomeric mutation. In some embodiments, the HCM is associated with a non-sarcomeric mutation. In some embodiments, the heart disease is obstructive or nonobstructive HCM caused by sarcomeric and/or non-sarcomeric mutations. In some embodiments, the sarcomeric mutation is a mutation in a myosin heavy chain β (MHC-β), cardiac muscle troponin T (cTnT), tropomyosin alpha-1 chain (TPM1), myosin-binding protein C cardiac-type (MYBPC3), cardiac troponin I (cTnI), myosin essential light chain (ELC), titin (TTN), myosin regulatory light chain 2 ventricular/cardiac muscle isoform (MLC-2), cardiac muscle alpha actin, or muscle LIM protein (MLP). In some embodiments, the sarcomeric mutation is a mutation in MHC-β. In some embodiments, the non-sarcomeric mutation is a mutation in protein kinase AMP-activated non-catalytic subunit gamma 2 (PRKAG2).

A formulation or a tablet as described herein may be used in methods of treating a disease or condition associated with HCM, comprising administering to the subject in need thereof said formulation or tablet. The disease or condition may be Fabry's Disease, Danon Disease, mitochondrial cardiomyopathies, or Noonan Syndrome.

Also provided herein is the use of a formulation or a tablet as described herein in the manufacture of a medicament for treatment of a disease or condition associated with HCM.

In some embodiments, the heart disease is heart failure with preserved ejection fraction (HFpEF). In some embodiments, the heart disease is diastolic dysfunction. In some embodiments, the heart disease is cardiomyopathy. In some embodiments, the heart disease is primary or secondary restrictive cardiomyopathy. In some embodiments, the heart disease is condition or symptoms caused by coronary artery disease. In some embodiments, the heart disease is myocardial infarction or angina pectoris. In some embodiments, the heart disease is left ventricular outflow tract obstruction. In some embodiments, the heart disease is hypertensive heart disease. In some embodiments, the heart disease is congenital heart disease. In some embodiments, the heart disease is cardiac ischemia and/or coronary heart disease. In some embodiments, the heart disease is diabetic heart disease. In other embodiments, the heart disease is congestive heart failure. In some embodiments, the heart disease is right heart failure. In other embodiments, the heart disease is cardiorenal syndrome. In some embodiments, the heart disease is infiltrative cardiomyopathy. In some embodiments, the heart disease is a condition that is or is related to cardiac senescence or diastolic dysfunction due to aging. In some embodiments, the heart disease is a condition that is or is related to left ventricular hypertrophy and/or concentric left ventricular remodeling.

A formulation or a tablet as described herein may be used in methods of treating a disease or condition associated with secondary left ventricular wall thickening in a subject, comprising administering to the subject in need thereof said formulation or tablet. The disease may be hypertension, valvular heart diseases (aortic stenosis, Mitral valve regurgitation), metabolic syndromes (diabetes, obesity), end stage renal disease, scleroderma, sleep apnea, amyloidosis, Fabry's disease, Friedreich Ataxia, Danon disease, Noonan syndrome, or Pompe disease.

Also provided herein is the use of a formulation or a tablet as described herein in the manufacture of a medicament for treatment of a disease or condition associated with secondary left ventricular wall thickening.

A formulation or a tablet as described herein may be used in methods of ameliorating a symptom associated with heart disease in a subject, comprising administering to the subject in need thereof said formulation or tablet, wherein the symptom is one or more selected from poor or reduced cardiac elasticity, poor or reduced diastolic left ventricular relaxation, abnormal left atrial pressure (e.g., abnormally high left atrial pressure), paroxysmal or permanent atrial fibrillation, increased left atrial and pulmonary capillary wedge pressures, increased left ventricular diastolic pressures, syncope, ventricular relaxation during diastole, ventricular fibrosis, left ventricular hypertrophy, left ventricular mass, increased left ventricular wall thickness, left ventricular mid-cavity obstruction, increased systolic anterior motion of mitral valve, left ventricular outflow tract obstruction, chest pain, exertional dyspnea, pre-syncope, abnormal exercise capacity, and fatigue.

A formulation or tablet as described herein may be used in methods of reducing the risk of a symptom associated with heart disease in a subject, comprising administering to the subject in need thereof said formulation or tablet, wherein the symptom is one or more selected from sudden cardiac death, poor or reduced cardiac elasticity, poor or reduced diastolic left ventricular relaxation, abnormal left atrial pressure (e.g., abnormally high left atrial pressure), paroxysmal or permanent atrial fibrillation, increased left atrial and pulmonary capillary wedge pressures, increased left ventricular diastolic pressures, syncope, ventricular relaxation during diastole, ventricular fibrosis, left ventricular hypertrophy, left ventricular mass, increased left ventricular wall thickness, left ventricular mid-cavity obstruction, increased systolic anterior motion of mitral valve, left ventricular outflow tract obstruction, chest pain, exertional dyspnea, pre-syncope, abnormal exercise capacity, and fatigue.

A formulation or a tablet as described herein may be used in methods of treating a disease or condition associated with small left ventricular cavity, cavity obliteration, hyperdynamic left ventricular contraction, obstruction of blood flow out of the left ventricle, cardiac hypertrophy, small cardiac stroke volume, impaired relaxation of the left ventricle, high left ventricle filling pressure, myocardial ischemia, or cardiac fibrosis in a subject, comprising administering to the subject in need thereof said formulation or tablet.

A formulation or a tablet as described herein may be used in methods of treating a disease or condition associated with small left ventricular cavity and cavity obliteration, hyperdynamic left ventricular contraction, myocardial ischemia, or cardiac fibrosis in a subject, comprising administering to the subject in need thereof said formulation or tablet.

Also provided herein is the use of a formulation or a tablet as described herein in the manufacture of a medicament for treatment of a disease or condition associated with small left ventricular cavity and cavity obliteration, hyperdynamic left ventricular contraction, myocardial ischemia, or cardiac fibrosis.

A formulation or a tablet as described herein may be used in methods of treating muscular dystrophies in a subject (*e.g*., Duchenne muscular dystrophy), comprising administering to the subject in need thereof said formulation or tablet. A formulation or a tablet as described herein may be used in the manufacture of a medicament for treatment of muscular dystrophies (*e.g*., Duchenne muscular dystrophy).

A formulation or a tablet as described herein, may be used in methods of treating a glycogen storage disease in a subject, comprising administering to the subject in need thereof said formulation or tablet. A formulation or a tablet as described herein may be used in the manufacture of a medicament for treatment of a glycogen storage disease.

A formulation or a tablet as described herein may be used inmethods for modulating the cardiac sarcomere in a subject which method comprises administering to a subject in need thereof a therapeutically effective amount of said formulation or tablet as described herein. A formulation or a tablet as described herein may be for used in methods of inhibiting the cardiac sarcomere, comprising contacting the cardiac sarcomere with said formulation or tablet. A formulation or a tablet as described herein may be used in the manufacture of a medicament for inhibiting the cardiac sarcomere of a subject.

A formulation or a tablet as described herein provided may be used inmethods for potentiating cardiac myosin in a subject which method comprises administering to a subject in need thereof a therapeutically effective amount of said formulation or a tablet as described herein. A formulation or a tablet as described herein may be used in the manufacture of a medicament for potentiating cardiac myosin in a subject.

In some embodiments, the methods provided and disclosed herein further comprise monitoring the effectiveness of the treatment. Examples of indicators include, but are not limited to improvement in one or more of the following: New York Heart Association (NYHA) Functional Classification, exercise capacity, cardiac elasticity, diastolic left ventricular relaxation, left atrial pressure, paroxysmal or permanent atrial fibrillation, left atrial and pulmonary capillary wedge pressures, left ventricular diastolic pressures, syncope, ventricular relaxation during diastole, ventricular fibrosis, left ventricular hypertrophy, left ventricular mass, left ventricular wall thickness, left ventricular mid-cavity obstruction systolic anterior motion of mitral valve, left ventricular outflow tract obstruction, chest pain, exertional dyspnea, pre-syncope, abnormal exercise capacity, and fatigue. These indicators can be monitored by techniques known in the art including self-reporting; ECG, including ambulatory ECG; echocardiography; cardiac MRI; CT; biopsy; cardiopulmonary exercise testing (CPET); and actigraphy.

In some embodiments, the formulation or tablet described and/or disclosed herein reduces the contractility of a cardiomyocyte. In some embodiments, the formulation or tablet described and/or disclosed herein reduces the contractility of a cardiomyocyte by greater than 40%, such as greater than 45%, 50%, 60%, 70%, 80%, or 90%. In some embodiments, the formulation or tablet described and/or disclosed herein reduced the contractility of a cardiomyocyte 40%-90%, such as 40%-80%, 40-70%, 50%-90%, 50%-80% or 50%-70%. In some embodiments, the formulation or tablet described and/or disclosed herein does not significantly alter calcium transients in the cardiomyocyte. In some embodiments, the formulation or tablet described and/or disclosed herein decreases the ATPase activity in a cardiomyocyte. Methods of measuring contractility, ATPase activity, and calcium transients are known in the art, for example, by calcium labeling, electrophysiological recordings, and microscopic imaging. In some embodiments, the formulation or tablet described and/or disclosed herein does not significantly inhibit or induce a cytochrome P450 (CYP) protein.

In some embodiments, the subject has a left ventricular wall that is thicker than normal prior to treatment. In some embodiments, the subject has a left ventricular wall thickness that is greater than 15 mm, such as greater than 18 mm, 20 mm, 22 mm, 25 mm, or 30 mm prior to treatment. In some embodiments, the left ventricular wall thickness is reduced by greater than 5%, such as greater than 8%, 10%, 12%, 15%, 20%, or 30% following treatment. Left ventricular wall thickness can be measured by methods known in the art, such as by echocardiography, CT scan, or a cardiac MRI.

In some embodiments, the subject has abnormal cardiac fibrosis prior to treatment. In some embodiments, the abnormal cardiac fibrosis is reduced by greater than 5%, such as greater than 8%, 10%, 12%, 15%, 20%, or 30% following treatment. Cardiac fibrosis can be measured by methods known in the art, such as by biopsy or a cardiac MRI.

In some embodiments, the subject has reduced exercise capacity prior to treatment. In some embodiments, the exercise capacity of the subject is increased by greater than 5%, such as greater than 8%, 10%, 12%, 15%, 20% or 30% following treatment. In some embodiments, the exercise capacity is measured by cardiopulmonary exercise testing (CPET). CPET measures changes in oxygen consumption (VO₂ max). Methods of measuring CPET and VO₂ max are well known in the art (Malhotra et al., JACC: Heart Failure, 2016, 4(8): 607-616; Guazzi et al., J Amer College Cardiol, 2017, 70 (13): 1618-1636; Rowin et al., JACC: Cariovasc Imaging, 2017, 10(11):1374-1386). In some embodiments, VO₂ max is improved by more than 1 mL/kg/m², such as more than 1.2 mL/kg/m², 1.4 mL/kg/m², 1.5 mL/kg/m², 1.7 mL/kg/m², 2 mL/kg/m², 2.2 mL/kg/m², 2.5 mL/kg/m², 3 mL/kg/m², 3.2 mL/kg/m², or 3.5 mL/kg/m² following treatment.

In some embodiments, the subject has a New York Heart Association (NYHA) Functional Classification of II, III, or IV prior to treatment. In some embodiments, the subject has a New York Heart Association (NYHA) Functional Classification of III or IV prior to treatment. In some embodiments, the subject has a New York Heart Association (NYHA) Functional Classification of IV prior to treatment. In some embodiments, the subject remains in the same NYHA functional class or has a reduced NYHA functional class following treatment.

In some embodiments, VO₂ max is improved by more than 1 mL/kg/m², such as more than 1.2 mL/kg/m², 1.4 mL/kg/m², 1.5 mL/kg/m², 1.7 mL/kg/m², or 2 mL/kg/m² and the subject has a reduced NYHA functional class following treatment. In some embodiments, VO₂ max is improved by more than 2.5 mL/kg/m², 3 mL/kg/m², 3.2 mL/kg/m², or 3.5 mL/kg/m² and the subject remains in the same NYHA functional class or has a reduced NYHA functional class following treatment.

In some embodiments, daily function and/or activity level of the subject is improved following treatment. Improved daily function and/or activity level may be measured, for example, by journaling or actigraphy, such as a FITBIT^{®} or FITBIT^{®}-like monitors.

In some embodiments, the subject has one or more of decreased shortness of breath, decreased chest pain, decreased arrhythmia burden, such as atrial fibrillation and ventricular arrhythmias, decreased incidence of heart failure, and decreased ventricular outflow obstruction following treatment.

### Kits

Any of the formulations or tablets provided herein may be used in articles of manufacture and kits containing any of said formulations or tablets provided herein. The article of manufacture may comprise a container with a label. Suitable containers include, for example, bottles, vials, and test tubes. The containers may be formed from a variety of materials such as glass or plastic. The container may hold a pharmaceutical composition provided herein. The label on the container may indicate that the pharmaceutical composition is used for preventing, treating or suppressing a condition described herein, and may also indicate directions for either *in vivo* or *in vitro* use.

A formulation or a tablet as described herein may be used in kits containing said formulation or tablet as described herein and instructions for use. The kits may contain instructions for use in the treatment of a heart disease in a subject in need thereof. A kit may also contain sterile packaging.

### Combinations

The formulation or tablet described and/or disclosed herein may be administered alone or in combination with other therapies and/or therapeutic agents useful in the treatment of the aforementioned disorders, diseases, or conditions.

The formulation or tablet described and/or disclosed herein may be combined with one or more other therapies to treat a heart disease, such as HCM or HFpEF. In some embodiments, the one or more therapies include therapies that retard the progression of heart failure by down-regulating neurohormonal stimulation of the heart and attempt to prevent cardiac remodeling (e.g., ACE inhibitors, angiotensin receptor blockers (ARBs), β-blockers, aldosterone receptor antagonists, or neural endopeptidase inhibitors). In some embodiments, the one or more therapies include therapies that improve cardiac function by stimulating cardiac contractility (e.g., positive inotropic agents, such as the β-adrenergic agonist dobutamine or the phosphodiesterase inhibitor milrinone). In other embodiments, the one or more therapies include therapies that reduce cardiac preload (e.g., diuretics, such as furosemide) or afterload (vasodilators of any class, including but not limited to calcium channel blockers, phosphodiesterase inhibitors, endothelin receptor antagonists, renin inhibitors, or smooth muscle myosin modulators).

The formulation or tablet described and/or disclosed herein may be combined with one or more other therapies to treat HCM or HFpEF. In some embodiments, the formulation or tablet described and/or disclosed herein may be combined with a β-blocker, verapamil, and/or disopyramide.
carboxymethylcellulose calcium, carboxymethylcellulose sodium, methylcellulose, hypromellose, lactose, polyvinyl alcohol, povidone, polyethylene oxide, polyacrylates, potato starch, pregelatinized starch, sodium alginate, sodium starch, sodium carboxy methyl cellulose, starch, and mixtures of any of the foregoing.

Embodiment 4. The formulation of any one of embodiments 1-3, wherein the disintegrant is selected from the group consisting of alginic acid, croscarmellose sodium, cellulose, carboxymethylcellulose calcium, carboxymethylcellulose sodium, microcrystalline cellulose, crospovidone, sodium starch glycolate, low-substituted hydroxypropyl cellulose, polacrillin potassium, pregelatinized starch, partially hydrolyzed starch, sodium carboxymethyl starch, starch, sodium alginate, sodium carboxy methyl cellulose, and mixtures of any of the foregoing.

Embodiment 5. The formulation of any one of embodiments 1-4, wherein the surfactant is selected from the group consisting of cetylpyridine chloride, heptadecaethylene oxycetanol, lecithins, polyoxyethylene stearate, nonoxynol 9, nonoxynol 10, octoxynol 9, sorbitan fatty acid esters, Span 20, Span 40, Span 60, Span 80, Span 85, polysorbates, polysorbate 20, polysorbate 21, polysorbate 40, polysorbate 60, polysorbate 61, polysorbate 65, polysorbate 80, sodium salts of fatty alcohol sulfates, sodium lauryl sulfate, sodium salts of sulfosuccinates, sodium dioctylsulfosuccinate, partial esters of fatty acids with alcohols, glycerine monostearate, glyceryl monooleate, ethers of fatty alcohols with polyoxyethylene, esters of fatty acids with polyoxyethylene, copolymers of ethylenoxide and propylenoxide (Pluronic^{®}), benzalkonium chloride, ethoxylated triglycerides, and mixtures of any of the foregoing.

Embodiment 6. The formulation of any one of embodiments 1-5, wherein the lubricant is selected from the group consisting of hydrogenated castor oil, magnesium stearate, glyceryl monostearate, calcium stearate, glyceryl behenate, glycerol distearate, glyceryl dipalmitostearate, behenoyl polyoxyl-8 glycerides, sodium stearyl fumarate, stearic acid, talc, zinc stearate, mineral oil, polyethylene glycol, polaxamer, sodium lauryl sulfate, and mixtures of any of the foregoing.

Embodiment 7. The formulation of any one of embodiments 1-6, wherein the formulation comprises:
(i) about 1 % by weight to about 80 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii) about 15 % by weight to about 90 % by weight of the filler;
(iii) about 0.1 % by weight to about 10 % by weight of the binder;
(iv) about 1 % by weight to about 10 % by weight of the disintegrant;
(v) about 0.1 % by weight to about 10 % by weight of the surfactant; and
(vi) about 0.1 % by weight to about 10 % by weight of the lubricant.

Embodiment 8. The formulation of any one of embodiments 1-7, wherein the formulation comprises:
(i) about 1 % by weight to about 50 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii) about 40 % by weight to about 80 % by weight of the filler;
(iii) about 0.5 % by weight to about 5 % by weight of the binder;
(iv) about 2 % by weight to about 8 % by weight of the disintegrant;
(v) about 0.5 % by weight to about 5 % by weight of the surfactant; and
(vi) about 0.5 % by weight to about 5 % by weight of the lubricant.

Embodiment 9. The formulation of any one of embodiments 1-8, wherein the formulation comprises:
(i) about 10 % by weight to about 30 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii) about 60 % by weight to about 80 % by weight of the filler;
(iii) about 1 % by weight to about 3 % by weight of the binder;
(iv) about 4 % by weight to about 6 % by weight of the disintegrant;
(v) about 1 % by weight to about 3 % by weight of the surfactant; and
(vi) about 0.5 % by weight to about 1.5 % by weight of the lubricant.

Embodiment 10. The formulation of any one of embodiments 1-9, wherein the formulation comprises:
(i) about 20 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii) about 70 % by weight of the filler;
(iii) about 2 % by weight of the binder;
(iv) about 5 % by weight of the disintegrant;
(v) about 2 % by weight of the surfactant; and
(vi) about 1 % by weight of the lubricant.

Embodiment 11. The formulation of any one of embodiments 1-8, wherein the formulation comprises:
(i) about 1 % by weight to about 10 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii) about 70 % by weight to about 90 % by weight of the filler;
(iii) about 1 % by weight to about 3 % by weight of the binder;
(iv) about 4 % by weight to about 6 % by weight of the disintegrant;
(v) about 1 % by weight to about 3 % by weight of the surfactant; and
(vi) about 0.5 % by weight to about 1.5 % by weight of the lubricant.

Embodiment 12. The formulation of any one of embodiments 1-8 and 11, wherein the formulation comprises:
(i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii) about 85 % by weight of the filler;
(iii) about 2 % by weight of the binder;
(iv) about 5 % by weight of the disintegrant;
(v) about 2 % by weight of the surfactant; and
(vi) about 1 % by weight of the lubricant.

Embodiment 13. The formulation of any one of embodiments 1-9 and 11, wherein the formulation comprises:
(i) about 10 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii) about 80 % by weight of the filler;
(iii) about 2 % by weight of the binder;
(iv) about 5 % by weight of the disintegrant;
(v) about 2 % by weight of the surfactant; and
(vi) about 1 % by weight of the lubricant.

Embodiment 14. The formulation of any one of embodiments 1-13, wherein the filler comprises mannitol.

Embodiment 15. The formulation of any one of embodiments 1-14, wherein the filler comprises microcrystalline cellulose.

Embodiment 16. The formulation of any one of embodiments 1-15, wherein the filler consists of mannitol and microcrystalline cellulose.

Embodiment 17. The formulation of any one of embodiments 1-16, wherein the binder is hydroxypropyl cellulose.

Embodiment 18. The formulation of any one of embodiments 1-17, wherein the disintegrant is croscarmellose sodium.

Embodiment 19. The formulation of any one of embodiments 1-18, wherein the surfactant is sodium lauryl sulfate.

Embodiment 20. The formulation of any one of embodiments 1-19, wherein the lubricant is magnesium stearate.

Embodiment 21. The formulation of any one of embodiments 1-8, wherein the formulation comprises:
(i) about 1 % by weight to about 50 % by weight of (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii-1) about 10 % by weight to about 60 % by weight of mannitol;
(ii-2) about 5 % by weight to about 45 % by weight of microcrystalline cellulose;
(iii) about 0.1 % by weight to about 10 % by weight of hydroxypropyl cellulose;
(iv) about 1 % by weight to about 10 % by weight of croscarmellose sodium;
(v) about 0.1 % by weight to about 10 % by weight of sodium lauryl sulfate; and
(vi) about 0.1 % by weight to about 10 % by weight of magnesium stearate.

Embodiment 22. The formulation of any one of embodiments 1-8 and 21, wherein the formulation comprises:
(i) about 10 % by weight to about 30 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii-1) about 40 % by weight to about 50 % by weight of mannitol;
(ii-2) about 20 % by weight to about 30 % by weight of microcrystalline cellulose;
(iii) about 1 % by weight to about 3 % by weight of hydroxypropyl cellulose;
(iv) about 4 % by weight to about 6 % by weight of croscarmellose sodium;
(v) about 1 % by weight to about 3 % by weight of sodium lauryl sulfate; and
(vi) about 0.5 % by weight to about 1.5 % by weight of magnesium stearate.

Embodiment 23. The formulation of any one of embodiments 1-8 and 21, wherein the formulation comprises:
(i) about 1 % by weight to about 10 % by weight of (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii-1) about 50 % by weight to about 60 % by weight of mannitol;
(ii-2) about 25 % by weight to about 35 % by weight of microcrystalline cellulose;
(iii) about 1 % by weight to about 3 % by weight of hydroxypropyl cellulose;
(iv) about 4 % by weight to about 6 % by weight of croscarmellose sodium;
(v) about 1 % by weight to about 3 % by weight of sodium lauryl sulfate; and
(vi) about 0.5 % by weight to about 1.5 % by weight of the magnesium stearate.

Embodiment 24. The formulation of any one of embodiments 1-8, 21, and 22, wherein the formulation comprises:
(i) about 20 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii-1) about 44 % by weight of mannitol;
(ii-2) about 26 % by weight of microcrystalline cellulose;
(iii) about 2 % by weight of hydroxypropyl cellulose;
(iv) about 5 % by weight of croscarmellose sodium;
(v) about 2 % by weight of sodium lauryl sulfate; and
(vi) about 1 % by weight of magnesium stearate.

Embodiment 25. The formulation of any one of embodiments 1-8, 21-23, wherein the formulation comprises:
(i) about 10 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii-1) about 50 % by weight of mannitol;
(ii-2) about 30 % by weight of microcrystalline cellulose;
(iii) about 2 % by weight of hydroxypropyl cellulose;
(iv) about 5 % by weight of croscarmellose sodium;
(v) about 2 % by weight of sodium lauryl sulfate; and
(vi) about 1 % by weight of the magnesium stearate.

Embodiment 26. The formulation of any one of embodiments 1-8, 21 and 23, wherein the formulation comprises:
(i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
(ii-1) about 54 % by weight of mannitol;
(ii-2) about 31 % by weight of microcrystalline cellulose;
(iii) about 2 % by weight of hydroxypropyl cellulose;
(iv) about 5 % by weight of croscarmellose sodium;
(v) about 2 % by weight of sodium lauryl sulfate; and
(vi) about 1 % by weight of the magnesium stearate.

Embodiment 27. The formulation of any of the preceding embodiments, wherein the formulation is for oral administration.

Embodiment 28. The formulation of any of the preceding embodiments, wherein the formulation is for administration once daily.

Embodiment 29. The formulation of any of the preceding embodiments, wherein the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, is (R)-N-(5-(5-ethyl-1,2,4-oxadiazol-3-yl)-2,3-dihydro-1H-inden-1-yl)-1-methyl-1H-pyrazole-4-carboxamide.

Embodiment 30. A tablet comprising:
(i) a core having a total core weight comprising:
   (a) an intra-granular component comprising:
      (a-i) (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
      (a-ii) an intra-granular filler;
      (a-iii) an intra-granular binder;
      (a-iv) an intra-granular disintegrant; and
      (a-v) an intra-granular surfactant;
         and
   (b) an extra-granular component comprising:
      (b-i) an extra-granular filler;
      (b-ii) an extra-granular disintegrant; and
      (b-iii) an extra-granular lubricant;
         and optionally
(ii) a coating layer comprising a coating agent.

Embodiment 31. The tablet of embodiment 30, wherein the intra-granular filler is selected from the group consisting of powdered cellulose, microcrystalline cellulose, silicified microcrystalline cellulose, kaolin, corn starch, maize starch, starch derivatives, pregelatinized starch, calcium phosphate, calcium hydrogen phosphate, dicalcium phosphate, tricalcium phosphate, compressible sugar, sugar alcohol, mannitol, sorbitol, maltitol, xylitol, lactitol, lactose, dextrose, maltose, sucrose, glucose, fructose, saccharose, raffinose, dextrates, trehalose, maltodextrines, and mixtures of any of the foregoing.

Embodiment 32. The tablet of embodiment 30 or 31, wherein the intra-granular binder is selected from the group consisting of arabic gum, acacia gum, alginate, alginic acid, corn starch, copolyvidone, polyvinylpyrrolidone, gelatin, glyceryl behenate, hydroxyethylcellulose, hydroxypropyl cellulose, carboxymethylcellulose, carboxymethylcellulose calcium, carboxymethylcellulose sodium, methylcellulose, hypromellose, lactose, polyvinyl alcohol, povidone, polyethylene oxide, polyacrylates, potato starch, pregelatinized starch, sodium alginate, sodium starch, sodium carboxy methyl cellulose, starch, and mixtures of any of the foregoing.

Embodiment 33. The tablet of any one of embodiments 30-32, wherein the intra-granular disintegrant is selected from the group consisting of alginic acid, croscarmellose sodium, cellulose, carboxymethylcellulose calcium, carboxymethylcellulose sodium, microcrystalline cellulose, crospovidone, sodium starch glycolate, low-substituted hydroxypropyl cellulose, polacrillin potassium, pregelatinized starch, partially hydrolyzed starch, sodium carboxymethyl starch, starch, sodium alginate, sodium carboxy methyl cellulose, and mixtures of any of the foregoing.

Embodiment 34. The tablet of any one of embodiments 30-33, wherein the intra-granular surfactant is selected from the group consisting of cetylpyridine chloride, heptadecaethylene oxycetanol, lecithins, polyoxyethylene stearate, nonoxynol 9, nonoxynol 10, octoxynol 9, sorbitan fatty acid esters, Span 20, Span 40, Span 60, Span 80, Span 85, polysorbates, polysorbate 20, polysorbate 21, polysorbate 40, polysorbate 60, polysorbate 61, polysorbate 65, polysorbate 80, sodium salts of fatty alcohol sulfates, sodium lauryl sulfate, sodium salts of sulfosuccinates, sodium dioctylsulfosuccinate, partial esters of fatty acids with alcohols, glycerine monostearate, glyceryl monooleate, ethers of fatty alcohols with polyoxyethylene, esters of fatty acids with polyoxyethylene, copolymers of ethylenoxide and propylenoxide (Plutonic^{®}), benzalkonium chloride, ethoxylated triglycerides, and mixtures of any of the foregoing.

Embodiment 35. The tablet of any one of embodiments 30-34, wherein the extra-granular filler is selected from the group consisting of powdered cellulose, microcrystalline cellulose, silicified microcrystalline cellulose, kaolin, corn starch, maize starch, starch derivatives, pregelatinized starch, calcium phosphate, calcium hydrogen phosphate, dicalcium phosphate, tricalcium phosphate, compressible sugar, sugar alcohol, mannitol, sorbitol, maltitol, xylitol, lactitol, lactose, dextrose, maltose, sucrose, glucose, fructose, saccharose, raffinose, dextrates, trehalose, maltodextrines, and mixtures of any of the foregoing.

Embodiment 36. The tablet of any one of embodiments 30-35, wherein the extra-granular disintegrant is selected from the group consisting of alginic acid, croscarmellose sodium, cellulose, carboxymethylcellulose calcium, carboxymethylcellulose sodium, microcrystalline cellulose, crospovidone, sodium starch glycolate, low-substituted hydroxypropyl cellulose, polacrillin potassium, pregelatinized starch, partially hydrolyzed starch, sodium carboxymethyl starch, starch, sodium alginate, sodium carboxy methyl cellulose, and mixtures of any of the foregoing.

Embodiment 37. The tablet of any one of embodiments 30-36, wherein the extra-granular lubricant is selected from the group consisting of hydrogenated castor oil, magnesium stearate, glyceryl monostearate, calcium stearate, glyceryl behenate, glycerol distearate, glyceryl dipalmitostearate, behenoyl polyoxyl-8 glycerides, sodium stearyl fumarate, stearic acid, talc, zinc stearate, mineral oil, polyethylene glycol, polaxamer, sodium lauryl sulfate, and mixtures of any of the foregoing.

Embodiment 38. The tablet of any one of embodiments 30-37, wherein the core comprises:
(a) an intra-granular component comprising:
   (a-i) about 1 % to about 80 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii) about 10 % to about 80 % of the total core weight of the intra-granular filler;
   (a-iii) about 0.1 % to about 10 % of the total core weight of the intra-granular binder;
   (a-iv) about 0.1 % to about 5 % of the total core weight of the intra-granular disintegrant; and
   (a-v) about 0.1 % to about 5 % of the total core weight of the intra-granular surfactant;
      and
(b) an extra-granular component comprising:
   (b-i) about 5 % to about 15 % of the total core weight of the extra-granular filler;
   (b-ii) about 0.1 % to about 5 % of the total core weight of the extra-granular disintegrant; and
   (b-iii) about 0.1 % to about 5 % of the total core weight of the extra-granular lubricant.

Embodiment 39. The tablet of any one of embodiments 30-38, wherein the core comprises:
(a) an intra-granular component comprising:
   (a-i) about 1 % to about 50 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii) about 40 % to about 80 % of the total core weight of the intra-granular filler;
   (a-iii) about 1 % to about 5 % of the total core weight of the intra-granular binder;
   (a-iv) about 1 % to about 5 % of the total core weight of the intra-granular disintegrant; and
   (a-v) about 1 % to about 5 % of the total core weight of the intra-granular surfactant;
      and
(b) an extra-granular component comprising:
   (b-i) about 5 % to about 15 % of the total core weight of the extra-granular filler;
   (b-ii) about 1 % to about 5 % of the total core weight of the extra-granular disintegrant; and
   (b-iii) about 0.1 % to about 2 % of the total core weight of the extra-granular lubricant.

Embodiment 40. The tablet of any one of embodiments 30-39, wherein the core comprises:
(a) an intra-granular component comprising:
   (a-i) about 10 % to about 30 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii) about 50 % to about 70 % of the total core weight of the intra-granular filler;
   (a-iii) about 1 % to about 3 % of the total core weight of the intra-granular binder;
   (a-iv) about 2 % to about 4 % of the total core weight of the intra-granular disintegrant; and
   (a-v) about 1 % to about 3 % of the total core weight of the intra-granular surfactant;
      and
(b) an extra-granular component comprising:
   (b-i) about 5 % to about 15 % of the total core weight of the an extra-granular filler;
   (b-ii) about 1 % to about 3 % of the total core weight of the extra-granular disintegrant; and
   (b-iii) about 0.1 % to about 1.5 % of the total core weight of the extra-granular lubricant.

Embodiment 41. The tablet of any one of embodiments 30-39, wherein the core comprises:
(a) an intra-granular component comprising:
   (a-i) about 1 % to about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii) about 60 % to about 80 % of the total core weight of the intra-granular filler;
   (a-iii) about 1 % to about 3 % of the total core weight of the intra-granular binder;
   (a-iv) about 2 % to about 4 % of the total core weight of the intra-granular disintegrant; and
   (a-v) about 1 % to about 3 % of the total core weight of the intra-granular surfactant;
      and
(b) an extra-granular component comprising:
   (b-i) about 5 % to about 15 % of the total core weight of the an extra-granular filler;
   (b-ii) about 1 % to about 3 % of the total core weight of the extra-granular disintegrant; and
   (b-iii) about 0.1 % to about 1.5 % of the total core weight of the extra-granular lubricant.

Embodiment 42. The tablet of any one of embodiments 30-39 and 41, wherein the core comprises:
(a) an intra-granular component comprising:
   (a-i) about 5 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii) about 74 % of the total core weight of the intra-granular filler;
   (a-iii) about 2 % of the total core weight of the intra-granular binder;
   (a-iv) about 3 % of the total core weight of the intra-granular disintegrant; and
   (a-v) about 2 % of the total core weight of the intra-granular surfactant;
      and
(b) an extra-granular component comprising:
   (b-i) about 11 % of the total core weight of the an extra-granular filler;
   (b-ii) about 2 % of the total core weight of the extra-granular disintegrant; and
   (b-iii) about 1 % of the total core weight of the extra-granular lubricant.

Embodiment 43. The tablet of any one of embodiments 30-41, wherein the core comprises:
(a) an intra-granular component comprising:
   (a-i) about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii) about 69 % of the total core weight of the intra-granular filler;
   (a-iii) about 2 % of the total core weight of the intra-granular binder;
   (a-iv) about 3 % of the total core weight of the intra-granular disintegrant; and
   (a-v) about 2 % of the total core weight of the intra-granular surfactant;
      and
(b) an extra-granular component comprising:
   (b-i) about 11 % of the total core weight of the an extra-granular filler;
   (b-ii) about 2 % of the total core weight of the extra-granular disintegrant; and
   (b-iii) about 1 % of the total core weight of the extra-granular lubricant.

Embodiment 44. The tablet of any one of embodiments 30-40, wherein the core comprises:
(a) an intra-granular component comprising:
   (a-i) about 20 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii) about 59 % of the total core weight of the intra-granular filler;
   (a-iii) about 2 % of the total core weight of the intra-granular binder;
   (a-iv) about 3 % of the total core weight of the intra-granular disintegrant; and
   (a-v) about 2 % of the total core weight of the intra-granular surfactant;
      and
(b) an extra-granular component comprising:
   (b-i) about 11 % of the total core weight of the an extra-granular filler;
   (b-ii) about 2 % of the total core weight of the extra-granular disintegrant; and
   (b-iii) about 1 % of the total core weight of the extra-granular lubricant.

Embodiment 45. The tablet of any one of embodiments 30-44, wherein the intra-granular filler comprises mannitol.

Embodiment 46. The tablet of any one of embodiments 30-45, wherein the intra-granular filler comprises microcrystalline cellulose.

Embodiment 47. The tablet of any one of embodiments 30-46, wherein the intra-granular filler consists of mannitol and microcrystalline cellulose.

Embodiment 48. The tablet of any one of embodiments 30-47, wherein the intra-granular binder is hydroxypropyl cellulose.

Embodiment 49. The tablet of any one of embodiments 30-48, wherein the intra-granular disintegrant is croscarmellose sodium.

Embodiment 50. The tablet of any one of embodiments 30-49, wherein the intra-granular surfactant is sodium lauryl sulfate.

Embodiment 51. The tablet of any one of embodiments 30-50, wherein the extra-granular filler is microcrystalline cellulose.

Embodiment 52. The tablet of any one of embodiments 30-51, wherein the extra-granular disintegrant is croscarmellose sodium.

Embodiment 53. The tablet of any one of embodiments 30-52, wherein the extra-granular lubricant is magnesium stearate.

Embodiment 54. The tablet of any one of embodiments 30-39 and 45-52, wherein the core comprises:
(a) an intra-granular component comprising:
   (a-i) about 1 % to about 50 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 40 % to about 60 % of the total core weight of mannitol;
   (a-ii-2) about 10 % to about 30 % of the total core weight of microcrystalline cellulose;
   (a-iii) about 1 % to about 5 % of the total core weight of hydroxypropyl cellulose;
   (a-iv) about 1 % to about 5 % of the total core weight of croscarmellose sodium; and
   (a-v) about 1 % to about 5 % of the total core weight of sodium lauryl sulfate;
      and
(b) an extra-granular component comprising:
   (b-i) about 5 % to about 15 % of the total core weight of microcrystalline cellulose;
   (b-ii) about 1 % to about 5 % of the total core weight of croscarmellose sodium; and
   (b-iii) about 0.1 % to about 2 % of the total core weight of extra-granular lubricant.

Embodiment 55. The tablet of any one of embodiments 30-39 and 45-53, wherein the core comprises:
(a) an intra-granular component comprising:
   (a-i) about 1 % to about 50 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 40 % to about 60 % of the total core weight of mannitol;
   (a-ii-2) about 10 % to about 30 % of the total core weight of microcrystalline cellulose;
   (a-iii) about 1 % to about 5 % of the total core weight of hydroxypropyl cellulose;
   (a-iv) about 1 % to about 5 % of the total core weight of croscarmellose sodium; and
   (a-v) about 1 % to about 5 % of the total core weight of sodium lauryl sulfate;
      and
(b) an extra-granular component comprising:
   (b-i) about 5 % to about 15 % of the total core weight of microcrystalline cellulose;
   (b-ii) about 1 % to about 5 % of the total core weight of croscarmellose sodium; and
   (b-iii) about 0.1 % to about 2 % of the total core weight of magnesium stearate.

Embodiment 56. The tablet of any one of embodiments 30-40, 45-52, and 54, wherein the core comprises:
(a) an intra-granular component comprising:
   (a-i) about 10 % to about 30 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 40 % to about 50 % of the total core weight of mannitol;
   (a-ii-2) about 10 % to about 20 % of the total core weight of microcrystalline cellulose;
   (a-iii) about 1 % to about 3 % of the total core weight of hydroxypropyl cellulose;
   (a-iv) about 2 % to about 4 % of the total core weight of croscarmellose sodium; and
   (a-v) about 1 % to about 3 % of the total core weight of sodium lauryl sulfate;
      and
(b) an extra-granular component comprising:
   (b-i) about 5 % to about 15 % of the total core weight of microcrystalline cellulose;
   (b-ii) about 1 % to about 3 % of the total core weight of croscarmellose sodium; and
   (b-iii) about 0.1 % to about 1.5 % of the total core weight of extra-granular lubricant.

Embodiment 57. The tablet of any one of embodiments 30-40, 45-56, wherein the core comprises:
(a) an intra-granular component comprising:
   (a-i) about 10 % to about 30 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 40 % to about 50 % of the total core weight of mannitol;
   (a-ii-2) about 10 % to about 20 % of the total core weight of microcrystalline cellulose;
   (a-iii) about 1 % to about 3 % of the total core weight of hydroxypropyl cellulose;
   (a-iv) about 2 % to about 4 % of the total core weight of croscarmellose sodium; and
   (a-v) about 1 % to about 3 % of the total core weight of sodium lauryl sulfate;
      and
(b) an extra-granular component comprising:
   (b-i) about 5 % to about 15 % of the total core weight of microcrystalline cellulose;
   (b-ii) about 1 % to about 3 % of the total core weight of croscarmellose sodium; and
   (b-iii) about 0.1 % to about 1.5 % of the total core weight of magnesium stearate.

Embodiment 58. The tablet of any one of embodiments 30-39, 41, 45-52, and 54, wherein the core comprises:
(a) an intra-granular component comprising:
   (a-i) about 1 % by weight to about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 50 % to about 60 % of the total core weight of mannitol;
   (a-ii-2) about 15 % to about 25 % of the total core weight of microcrystalline cellulose;
   (a-iii) about 1 % to about 3 % of the total core weight of hydroxypropyl cellulose;
   (a-iv) about 2 % to about 4 % of the total core weight of croscarmellose sodium; and
   (a-v) about 1 % to about 3 % of the total core weight of sodium lauryl sulfate;
      and
(b) an extra-granular component comprising:
   (b-i) about 5 % to about 15 % of the total core weight of microcrystalline cellulose;
   (b-ii) about 1 % to about 3 % of the total core weight of croscarmellose sodium; and
   (b-iii) about 0.1 % to about 1.5 % of the total core weight of extra-granular lubricant.

Embodiment 59. The tablet of any one of embodiments 30-39, 41, 45-55, wherein the core comprises:
(a) an intra-granular component comprising:
   (a-i) about 1 % by weight to about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 50 % to about 60 % of the total core weight of mannitol;
   (a-ii-2) about 15 % to about 25 % of the total core weight of microcrystalline cellulose;
   (a-iii) about 1 % to about 3 % of the total core weight of hydroxypropyl cellulose;
   (a-iv) about 2 % to about 4 % of the total core weight of croscarmellose sodium; and
   (a-v) about 1 % to about 3 % of the total core weight of sodium lauryl sulfate;
      and
(b) an extra-granular component comprising:
   (b-i) about 5 % to about 15 % of the total core weight of microcrystalline cellulose;
   (b-ii) about 1 % to about 3 % of the total core weight of croscarmellose sodium; and
   (b-iii) about 0.1 % to about 1.5 % of the total core weight of magnesium stearate.

Embodiment 60. The tablet of any one of embodiments 30-39, 41, 42, 45-52, 54, and 58, wherein the core comprises:
(a) an intra-granular component comprising:
   (a-i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 54 % by weight of mannitol;
   (a-ii-2) about 20 % by weight of microcrystalline cellulose;
   (a-iii) about 2 % by weight of hydroxypropyl cellulose;
   (a-iv) about 3 % by weight of croscarmellose sodium; and
   (a-v) about 2 % by weight of sodium lauryl sulfate;
      and
(b) an extra-granular component comprising:
   (b-i) about 11 % by weight of microcrystalline cellulose;
   (b-ii) about 2 % by weight of croscarmellose sodium; and
   (b-iii) about 1 % by weight of extra-granular lubricant.

Embodiment 61. The tablet of any one of embodiments 30-39, 41, 42, 45-55, 58, and 59, wherein the core comprises:
(a) an intra-granular component comprising:
   (a-i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 54 % by weight of mannitol;
   (a-ii-2) about 20 % by weight of microcrystalline cellulose;
   (a-iii) about 2 % by weight of hydroxypropyl cellulose;
   (a-iv) about 3 % by weight of croscarmellose sodium; and
   (a-v) about 2 % by weight of sodium lauryl sulfate;
      and
(b) an extra-granular component comprising:
   (b-i) about 11 % by weight of microcrystalline cellulose;
   (b-ii) about 2 % by weight of croscarmellose sodium; and
   (b-iii) about 1 % by weight of magnesium stearate.

Embodiment 62. The tablet of any one of embodiments 30-41, 45-52, 54, 56, and 58, wherein the core comprises:
(a) an intra-granular component comprising:
   (a-i) about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 50 % of the total core weight of mannitol;
   (a-ii-2) about 19 % of the total core weight of microcrystalline cellulose;
   (a-iii) about 2 % of the total core weight of hydroxypropyl cellulose;
   (a-iv) about 3 % of the total core weight of croscarmellose sodium; and
   (a-v) about 2 % of the total core weight of sodium lauryl sulfate;
      and
(b) an extra-granular component comprising:
   (b-i) about 11 % of the total core weight of microcrystalline cellulose;
   (b-ii) about 2 % of the total core weight of croscarmellose sodium; and
   (b-iii) about 1 % of the total core weight of extra-granular lubricant.

Embodiment 63. The tablet of any one of embodiments 30-41 and 45-59, wherein the core comprises:
(a) an intra-granular component comprising:
   (a-i) about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 50 % of the total core weight of mannitol;
   (a-ii-2) about 19 % of the total core weight of microcrystalline cellulose;
   (a-iii) about 2 % of the total core weight of hydroxypropyl cellulose;
   (a-iv) about 3 % of the total core weight of croscarmellose sodium; and
   (a-v) about 2 % of the total core weight of sodium lauryl sulfate;
      and
(b) an extra-granular component comprising:
   (b-i) about 11 % of the total core weight of microcrystalline cellulose;
   (b-ii) about 2 % of the total core weight of croscarmellose sodium; and
   (b-iii) about 1 % of the total core weight of magnesium stearate.

Embodiment 64. The tablet of any one of embodiments 30-40, 44-52, 54, and 56, wherein the core comprises:
(a) an intra-granular component comprising:
   (a-i) about 20 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 44 % of the total core weight of mannitol;
   (a-ii-2) about 15 % of the total core weight of microcrystalline cellulose;
   (a-iii) about 2 % of the total core weight of hydroxypropyl cellulose;
   (a-iv) about 3 % of the total core weight of croscarmellose sodium; and
   (a-v) about 2 % of the total core weight of sodium lauryl sulfate;
      and
(b) an extra-granular component comprising:
   (b-i) about 11 % of the total core weight of microcrystalline cellulose;
   (b-ii) about 2 % of the total core weight of croscarmellose sodium; and
   (b-iii) about 1 % of the total core weight of extra-granular lubricant.

Embodiment 65. The tablet of any one of embodiments 30-40, 44-57, wherein the core comprises:
(a) an intra-granular component comprising:
   (a-i) about 20 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 44 % of the total core weight of mannitol;
   (a-ii-2) about 15 % of the total core weight of microcrystalline cellulose;
   (a-iii) about 2 % of the total core weight of hydroxypropyl cellulose;
   (a-iv) about 3 % of the total core weight of croscarmellose sodium; and
   (a-v) about 2 % of the total core weight of sodium lauryl sulfate;
      and
(b) an extra-granular component comprising:
   (b-i) about 11 % of the total core weight of microcrystalline cellulose;
   (b-ii) about 2 % of the total core weight of croscarmellose sodium; and
   (b-iii) about 1 % of the total core weight of magnesium stearate.

Embodiment 66. The tablet of any one of embodiments 30-65, comprising a coating layer.

Embodiment 67. The tablet of any one of embodiments 30-66, wherein the coating agent is selected from the group consisting of Opadry QX White 21A180025, Opadry I, and Opadry II.

Embodiment 68. The tablet of any one of embodiments 30-67, wherein the coating agent is Opadry QX White 21A180025.

Embodiment 69. The tablet of any one of embodiments 30-68, wherein the tablet comprises about 0.5 % to about 10 % of the total core weight of coating agent.

Embodiment 70. The tablet of any one of embodiments 30-69, wherein the tablet comprises about 1 % to about 5 % of the total core weight of coating agent.

Embodiment 71. The tablet of any one of embodiments 30-71, wherein the tablet comprises about 2 % to about 4 % of the total core weight of coating agent.

Embodiment 72. The tablet of any one of embodiments 30-71, wherein the tablet comprises about 3 % of the total core weight of coating agent.

Embodiment 73. The tablet of any one of embodiments 30-72, wherein the total core weight is about 50 mg.

Embodiment 74. The tablet of embodiment 73, wherein the core comprises:
(a) an intra-granular component comprising:
   (a-i) about 2.5 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 27 mg of mannitol;
   (a-ii-2) about 10 mg of microcrystalline cellulose;
   (a-iii) about 1 mg of hydroxypropyl cellulose;
      (a-iv) about 1.5 mg of croscarmellose sodium; and
   (a-v) about 1 mg of sodium lauryl sulfate;
      and
(b) an extra-granular component comprising:
   (b-i) about 5.5 mg of microcrystalline cellulose;
   (b-ii) about 1 mg of croscarmellose sodium; and
   (b-iii) about 0.5 mg of extra-granular lubricant.

Embodiment 75. The tablet of embodiment 73 or 74, wherein the core comprises:
(a) an intra-granular component comprising:
   (a-i) about 2.5 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 27 mg of mannitol;
   (a-ii-2) about 10 mg of microcrystalline cellulose;
   (a-iii) about 1 mg of hydroxypropyl cellulose;
   (a-iv) about 1.5 mg of croscarmellose sodium; and
   (a-v) about 1 mg of sodium lauryl sulfate;
      and
(b) an extra-granular component comprising:
   (b-i) about 5.5 mg of microcrystalline cellulose;
   (b-ii) about 1 mg of croscarmellose sodium; and
   (b-iii) about 0.5 mg of magnesium stearate.

Embodiment 76. The tablet of any one of embodiments 73-75, wherein the tablet comprises about 1.5 mg of coating agent.

Embodiment 77. The tablet of embodiment 76, wherein the coating agent is Opadry QX White 21A180025

Embodiment 78. The tablet of any one of embodiments 30-72, wherein the total core weight is about 100 mg.

Embodiment 79. The tablet of embodiment 78, wherein the core comprises:
(a) an intra-granular component comprising:
   (a-i) about 5 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 54 mg of mannitol;
   (a-ii-2) about 20 mg of microcrystalline cellulose;
   (a-iii) about 2 mg of hydroxypropyl cellulose;
   (a-iv) about 3 mg of croscarmellose sodium; and
   (a-v) about 2 mg of sodium lauryl sulfate;
      and
(b) an extra-granular component comprising:
   (b-i) about 11 mg of microcrystalline cellulose;
   (b-ii) about 2 mg of croscarmellose sodium; and
   (b-iii) about 1 mg of extra-granular lubricant.

Embodiment 80. The tablet of embodiment 78 or 79, wherein the core comprises:
(a) an intra-granular component comprising:
   (a-i) about 5 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 54 mg of mannitol;
   (a-ii-2) about 20 mg of microcrystalline cellulose;
   (a-iii) about 2 mg of hydroxypropyl cellulose;
   (a-iv) about 3 mg of croscarmellose sodium; and
   (a-v) about 2 mg of sodium lauryl sulfate;
      and
(b) an extra-granular component comprising:
   (b-i) about 11 mg of microcrystalline cellulose;
   (b-ii) about 2 mg of croscarmellose sodium; and
   (b-iii) about 1 mg of magnesium stearate.

Embodiment 81. The tablet of embodiment 78, wherein the core comprises:
(a) an intra-granular component comprising:
   (a-i) about 20 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 44 mg of the total core weight of mannitol;
   (a-ii-2) about 15 mg of the total core weight of microcrystalline cellulose;
   (a-iii) about 2 mg of the total core weight of hydroxypropyl cellulose;
   (a-iv) about 3 mg of the total core weight of croscarmellose sodium; and
   (a-v) about 2 mg of the total core weight of sodium lauryl sulfate;
      and
(b) an extra-granular component comprising:
   (b-i) about 11 mg of the total core weight of microcrystalline cellulose;
   (b-ii) about 2 mg of the total core weight of croscarmellose sodium; and
   (b-iii) about 1 mg of the total core weight of extra-granular lubricant.

Embodiment 82. The tablet of embodiment 78 or 81, wherein the core comprises:
(a) an intra-granular component comprising:
   (a-i) about 20 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 44 mg of the total core weight of mannitol;
   (a-ii-2) about 15 mg of the total core weight of microcrystalline cellulose;
   (a-iii) about 2 mg of the total core weight of hydroxypropyl cellulose;
   (a-iv) about 3 mg of the total core weight of croscarmellose sodium; and
   (a-v) about 2 mg of the total core weight of sodium lauryl sulfate;
      and
(b) an extra-granular component comprising:
   (b-i) about 11 mg of the total core weight of microcrystalline cellulose;
   (b-ii) about 2 mg of the total core weight of croscarmellose sodium; and
   (b-iii) about 1 mg of the total core weight of magnesium stearate.

Embodiment 83. The tablet of any one of embodiments 78-82, wherein the tablet comprises about 3 mg of coating agent.

Embodiment 84. The tablet of embodiment 83, wherein the coating agent is Opadry QX White 21A180025

Embodiment 85. The tablet of any one of embodiments 30-72, wherein the total core weight is about 200 mg.

Embodiment 86. The tablet of embodiment 85, wherein the core comprises:
(a) an intra-granular component comprising:
   (a-i) about 10 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 108 mg of mannitol;
   (a-ii-2) about 40 mg of microcrystalline cellulose;
   (a-iii) about 4 mg of hydroxypropyl cellulose;
   (a-iv) about 6 mg of croscarmellose sodium; and
   (a-v) about 4 mg of sodium lauryl sulfate;
      and
(b) an extra-granular component comprising:
   (b-i) about 22 mg of microcrystalline cellulose;
   (b-ii) about 4 mg of croscarmellose sodium; and
   (b-iii) about 2 mg of extra-granular lubricant.

Embodiment 87. The tablet of embodiment 85 or 86, wherein the core comprises:
(a) an intra-granular component comprising:
   (a-i) about 10 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 108 mg of mannitol;
   (a-ii-2) about 40 mg of microcrystalline cellulose;
   (a-iii) about 4 mg of hydroxypropyl cellulose;
   (a-iv) about 6 mg of croscarmellose sodium; and
   (a-v) about 4 mg of sodium lauryl sulfate; and
(b) an extra-granular component comprising:
   (b-i) about 22 mg of microcrystalline cellulose;
   (b-ii) about 4 mg of croscarmellose sodium; and
   (b-iii) about 2 mg of magnesium stearate.

Embodiment 88. The tablet of embodiment 85, wherein the core comprises:
(a) an intra-granular component comprising:
   (a-i) about 40 mg of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 88 mg of the total core weight of mannitol;
   (a-ii-2) about 30 mg of the total core weight of microcrystalline cellulose;
   (a-iii) about 4 mg of the total core weight of hydroxypropyl cellulose;
   (a-iv) about 6 mg of the total core weight of croscarmellose sodium; and
   (a-v) about 4 mg of the total core weight of sodium lauryl sulfate;
      and
(b) an extra-granular component comprising:
   (b-i) about 22 mg of the total core weight of microcrystalline cellulose;
   (b-ii) about 4 mg of the total core weight of croscarmellose sodium; and
   (b-iii) about 2 mg of the total core weight of extra-granular lubricant.

Embodiment 89. The tablet of embodiment 85 or 88, wherein the core comprises:
(a) an intra-granular component comprising:
   (a-i) about 40 mg of the total core weight of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 88 mg of the total core weight of mannitol;
   (a-ii-2) about 30 mg of the total core weight of microcrystalline cellulose;
   (a-iii) about 4 mg of the total core weight of hydroxypropyl cellulose;
   (a-iv) about 6 mg of the total core weight of croscarmellose sodium; and
   (a-v) about 4 mg of the total core weight of sodium lauryl sulfate;
      and
(b) an extra-granular component comprising:
   (b-i) about 22 mg of the total core weight of microcrystalline cellulose;
   (b-ii) about 4 mg of the total core weight of croscarmellose sodium; and
   (b-iii) about 2 mg of the total core weight of magnesium stearate.

Embodiment 90. The tablet of any one of embodiments 85-89, wherein the tablet comprises about 6 mg of coating agent.

Embodiment 91. The tablet of embodiment 90, wherein the coating agent is Opadry QX White 21A180025

Embodiment 92. The tablet of any one of embodiments 30-72, wherein the total core weight is about 70 mg.

Embodiment 93. The tablet of embodiment 92, wherein the core comprises:
(a) an intra-granular component comprising:
   (a-i) about 7 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 35 mg of mannitol;
   (a-ii-2) about 13.3 mg of microcrystalline cellulose;
   (a-iii) about 1.4 mg of hydroxypropyl cellulose;
   (a-iv) about 2.1 mg of croscarmellose sodium; and
   (a-v) about 1.4 mg of sodium lauryl sulfate;
      and
(b) an extra-granular component comprising:
   (b-i) about 7.7 mg of microcrystalline cellulose;
   (b-ii) about 1.4 mg of croscarmellose sodium; and
   (b-iii) about 0.7 mg of extra-granular lubricant.

Embodiment 94. The tablet of embodiment 92 or 93, wherein the core comprises:
(a) an intra-granular component comprising:
   (a-i) about 7 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 35 mg of mannitol;
   (a-ii-2) about 13.3 mg of microcrystalline cellulose;
   (a-iii) about 1.4 mg of hydroxypropyl cellulose;
   (a-iv) about 2.1 mg of croscarmellose sodium; and
   (a-v) about 1.4 mg of sodium lauryl sulfate;
      and
(b) an extra-granular component comprising:
   (b-i) about 7.7 mg of microcrystalline cellulose;
   (b-ii) about 1.4 mg of croscarmellose sodium; and
   (b-iii) about 0.7 mg of magnesium stearate.

Embodiment 95. The tablet of any one of embodiments 92-94, wherein the tablet comprises about 2.1 mg of coating agent.

Embodiment 96. The tablet of embodiment 95, wherein the coating agent is Opadry QX White 21A180025

Embodiment 97. The tablet of any one of embodiments 30-72, wherein the total core weight is about 400 mg.

Embodiment 98. The tablet of embodiment 97, wherein the core comprises:
(a) an intra-granular component comprising:
   (a-i) about 40 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 200 mg of mannitol;
   (a-ii-2) about 76 mg of microcrystalline cellulose;
   (a-iii) about 8 mg of hydroxypropyl cellulose;
   (a-iv) about 12 mg of croscarmellose sodium; and
   (a-v) about 8 mg of sodium lauryl sulfate;
      and
(b) an extra-granular component comprising:
   (b-i) about 44 mg of microcrystalline cellulose;
   (b-ii) about 8 mg of croscarmellose sodium; and
   (b-iii) about 4 mg of extra-granular lubricant.

Embodiment 99. The tablet of embodiment 97 or 98, wherein the core comprises:
(a) an intra-granular component comprising:
   (a-i) about 40 mg of the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof;
   (a-ii-1) about 200 mg of mannitol;
   (a-ii-2) about 76 mg of microcrystalline cellulose;
   (a-iii) about 8 mg of hydroxypropyl cellulose;
   (a-iv) about 12 mg of croscarmellose sodium; and
   (a-v) about 8 mg of sodium lauryl sulfate;
      and
(b) an extra-granular component comprising:
   (b-i) about 44 mg of microcrystalline cellulose;
   (b-ii) about 8 mg of croscarmellose sodium; and
   (b-iii) about 4 mg of magnesium stearate.

Embodiment 100. The tablet of any one of embodiments 97-98, wherein the tablet comprises about 12 mg of coating agent.

Embodiment 101. The tablet of embodiment 100, wherein the coating agent is Opadry QX White 21A180025

Embodiment 102. The tablet of any one of embodiments 30-101, wherein the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, is (R)-N-(5-(5-ethyl-1,2,4-oxadiazol-3-yl)-2,3-dihydro-1H-inden-1-yl)-1-methyl-1H-pyrazole-4-carboxamide.

Embodiment 103. A process for making the tablet of any one of embodiments 30-102, wherein the process comprises:
(1) preparing granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant;
(2) milling the granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant to form the intra-granular component;
(3) blending the intra-granular component with the extra-granular filler, the extra-granular disintegrant, and the extra-granular lubricant to form a final blend mixture;
(4) compressing the final blend mixture to form the core;
(5) optionally coating the core with the coating layer.

Embodiment 104. The process of embodiment 102, wherein preparing granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant comprises a wet granulation step.

Embodiment 105. The process of embodiment 104, wherein the wet granulation step is performed with a high shear granulator.

Embodiment 106. The process of embodiment 104 or 105, wherein the wet granulation step further comprises a wet milling step.

Embodiment 107. The process of embodiment 106, wherein the wet milling step is performed with a conical mill.

Embodiment 108. The process of any one of embodiments 103-107, wherein preparing granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant further comprises drying the granules.

Embodiment 109. The process of embodiment 108, wherein drying the granules is performed in a fluid bed dryer.

Embodiment 110. The process of any one of embodiments 103-109, wherein milling the granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant is performed with a conical mill.

Embodiment 111. The process of any one of embodiments 103-110, wherein blending the intra-granular component with the extra-granular filler, the extra-granular disintegrant, and the extra-granular lubricant is performed in a V-blender.

Embodiment 112. The process of any one of embodiments 103-111, wherein compressing the final blend mixture to form the core is performed with a rotary tablet press.

Embodiment 113. The process of any one of embodiments 103-112, comprising coating the core with the coating layer.

Embodiment 114. The process of any one of embodiments 103-113, comprising coating the core with the coating layer using a conventional coating pan.

Embodiment 115. The process of any one of embodiments 103-114, wherein the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, is (R)-N-(5-(5-ethyl-1,2,4-oxadiazol-3-yl)-2,3-dihydro-1H-inden-1-yl)-1-methyl-1H-pyrazole-4-carboxamide.

Embodiment 116. A process for making a tablet comprising:
(i) a core having a total core weight comprising:
   (a) an intra-granular component comprising:
      (a-i) (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
      (a-ii) an intra-granular filler;
      (a-iii) an intra-granular binder;
      (a-iv) an intra-granular disintegrant; and
      (a-v) an intra-granular surfactant;
         and
   (b) an extra-granular component comprising:
      (b-i) an extra-granular filler;
      (b-ii) an extra-granular disintegrant; and
      (b-iii) an extra-granular lubricant;
         and
(ii) a coating layer comprising a coating agent.
   wherein the process comprises:
   (1) preparing granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant;
   (2) milling the granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant to form the intra-granular component;
   (3) blending the intra-granular component with the extra-granular filler, the extra-granular disintegrant, and the extra-granular lubricant to form a final blend mixture;
   (4) compressing the final blend mixture to form the core;
   (5) coating the core with the coating layer.

Embodiment 117. The process of embodiment 116, wherein preparing granules comprising (R)-N-(5-(5-ethyl-1,2,4-oxadiazol-3 -yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant comprises a wet granulation step.

Embodiment 118. The process of embodiment 117, wherein the wet granulation step is performed with a high shear granulator.

Embodiment 119. The process of embodiment 117 or 118, wherein the wet granulation step further comprises a wet milling step.

Embodiment 120. The process of embodiment 119, wherein the wet milling step is performed with a conical mill.

Embodiment 121. The process of any one of embodiments 116-120, wherein preparing granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant further comprises drying the granules.

Embodiment 122. The process of embodiment 121, wherein drying the granules is performed in a fluid bed dryer.

Embodiment 123. The process of any one of embodiments 116-122, wherein milling the granules comprising (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, the intra-granular filler, the intra-granular binder, the intra-granular disintegrant, and the intra-granular surfactant is performed with a conical mill.

Embodiment 124. The process of any one of embodiments 116-123, wherein blending the intra-granular component with the extra-granular filler, the extra-granular disintegrant, and the extra-granular lubricant is performed in a V-blender.

Embodiment 125. The process of any one of embodiments 116-124, wherein compressing the final blend mixture to form the core is performed with a rotary tablet press.

Embodiment 126. The process of any one of embodiments 116-125, comprising coating the core with the coating layer.

Embodiment 127. The process of any one of embodiments 116-126, comprising coating the core with the coating layer using a coating pan.

Embodiment 128. The process of any one of embodiments 116-127, wherein the (R)-N-(5-(5-ethyl-1,2,4-oxadiazol-3-yl)-2,3-dihydro-1H-inden-1-yl)-1-methyl-1H-pyrazole-4-carboxamide, or the hydrate, solvate, polymorph, or pharmaceutically acceptable salt thereof, is (R)-N-(5-(5-ethyl-1,2,4-oxadiazol-3-yl)-2,3-dihydro-1H-inden-1-yl)-1-methyl-1H-pyrazole-4-carboxamide.

Embodiment 129. A method of treating heart disease in a subject in need thereof, comprising administering to the subject the formulation of any one of embodiments 1-29, or the tablet of any one of embodiments 30-102.

Embodiment 130. The method of embodiment 129, wherein the heart disease is hypertrophic cardiomyopathy (HCM).

Embodiment 131. The method of embodiment 130, wherein the HCM is obstructive or nonobstructive or is associated with a sarcomeric and/or non-sarcomeric mutation.

Embodiment 132. The method of embodiment 129, wherein the heart disease is heart failure with preserved ejection fraction (HFpEF).

Embodiment 133. The method of embodiment 129, wherein the heart disease is selected from the group consisting of diastolic dysfunction, primary or secondary restrictive cardiomyopathy, myocardial infarction and angina pectoris, left ventricular outflow tract obstruction, hypertensive heart disease, congenital heart disease, cardiac ischemia, coronary heart disease, diabetic heart disease, congestive heart failure, right heart failure, cardiorenal syndrome, and infiltrative cardiomyopathy.

Embodiment 134. The method of embodiment 129, wherein the heart disease is or is related to one or more conditions selected from the group consisting of cardiac senescence, diastolic dysfunction due to aging, left ventricular hypertrophy and concentric left ventricular remodeling.

Embodiment 135. A method of treating a disease or condition associated with HCM in a subject in need thereof, comprising administering to the subject the formulation of any one of embodiments 1-29, or the tablet of any one of embodiments 30-102.

Embodiment 136. The method of embodiment 135, wherein the disease or condition is selected from the group consisting of Fabry's Disease, Danon Disease, mitochondrial cardiomyopathies, and Noonan Syndrome.

Embodiment 137. A method of treating a disease or condition that is associated with secondary left ventricular wall thickening in a subject in need thereof, comprising administering to the subject the formulation of any one of embodiments 1-29, or the tablet of any one of embodiments 30-102.

Embodiment 138. The method of embodiment 137, wherein the disease or condition is selected from the group consisting of hypertension, valvular heart diseases, metabolic syndromes, end stage renal disease, scleroderma, sleep apnea, amyloidosis, Fabry's disease, Friedreich Ataxia, Danon disease, Noonan syndrome, and Pompe disease.

Embodiment 139. A method of treating a disease or condition that is associated with small left ventricular cavity and cavity obliteration, hyperdynamic left ventricular contraction, myocardial ischemia, or cardiac fibrosis in a subject in need thereof, comprising administering to the subject the formulation of any one of embodiments 1-29, or the tablet of any one of embodiments 30-102.

Embodiment 140. A method of treating a disease or condition selected from muscular dystrophies and glycogen storage diseases in a subject in need thereof, comprising administering to the subject the formulation of any one of embodiments 1-29, or the tablet of any one of embodiments 30-102.

### EXAMPLES

The following examples are offered to illustrate but not to limit the compositions, uses, and methods provided herein.

The following abbreviations are used throughout the Examples: TEA (trimethylamine), DCM (dichloromethane), (Boc)₂O (di-tert-butyl dicarbonate), EA (Ethyl acetate), PE (Petroleum ether, DMF (N,N-dimethylformamide), DIEA (N-ethyl-N-isopropylpropan-2-amine), HATU (1-[Bis(dimethylamino)methylene]-1H-1,2,3-triazolo[4,5-b]pyridinium 3-oxid hexafluorophosphate), HOAt (1-Hydroxy-7-azabenzotriazole), HOBt (Hydroxybenzotriazole), EDCI (1-Ethyl-3-(3-dimethylaminopropyl)carbodiimide), MeOH (methanol), EtOH (ethanol), iPrOH (propan-2-ol), ACN (acetonitrile), TFA (trifluoroacetic acid), DPPA (Diphenylphosphoryl azide), DBU (1,8-Diazabicyclo(5.4.0)undec-7-ene), THF (tetrahydrofuran), PPh₃ (triphenylphosphane), SM (starting material), Hex (hexane), NCS (N-chlorosuccinimide), r.t. (room temperature), DCE (dichloroethane), FA (formic acid), CHCl₃ (Chloroform), BnBr (benzyl bromide), HCl (hydrogen chloride), equiv (equivalent), DSC (bis(2,5-dioxopyrrolidin-1-yl) carbonate), RH (relative humidity), QL (quantitation limit), RRT (relative retention time), RS (related substances).

### Example 1 - Synthesis of (R)-N-(5-(5-ethyl-1,2,4-oxadiazol-3-yl)-2,3-dihydro-1H-inden-1-yl)-1-methyl-1H-pyrazole-4-carboxamide

### (i) Synthesis of Intermediate A:

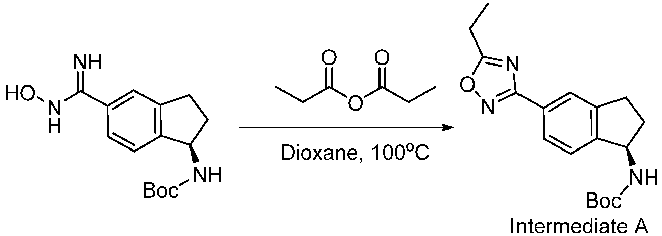

To a solution of *tert*-butyl *N*-[(*1R*)-5-(*N*-hydroxycarbamimidoyl)-2,3-dihydro-1*H-*inden-1-yl] carbamate (16 g, 54.9 mmol, 1.0 equiv) in dioxane (300 mL) was added propanoyl propanoate (8.4 g, 64.5 mmol, 1.2 equiv). The mixture was stirred at 105 °C for 8 h, cooled to r.t., concentrated under reduced pressure, and purified by silica gel chromatography (EA/PE, 1/9) to give 17.5 g (97%) of *tert*-butyl *N*-[(1*R*)-5-(5-ethyl-1,2,4-oxadiazol-3-yl)-2,3-dihydro-1*H*-inden-1-yl]carbamate as a white solid.

### (ii) Synthesis of Intermediate B:

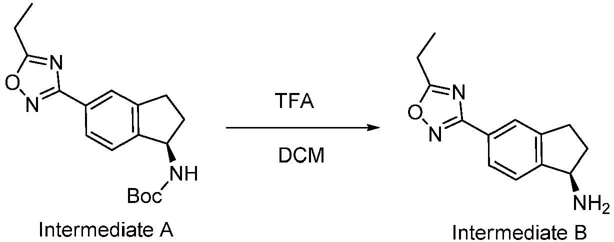

To a solution of *tert*-butyl *N*-[(1*R*)-5-(5-ethyl-1,2,4-oxadiazol-3-yl)-2,3-dihydro-1*H*-inden-1-yl]carbamate (17.6 g, 53.4 mmol, 1.0 equiv) in DCM (120 mL) was added TFA (24 mL). The mixture was stirred at room temperature overnight and concentrated under reduced pressure. The mixture was then poured into ethanol (50 mL) and water (5 mL) and the pH was adjusted to 12 with sodium hydroxide solution (2 N). The mixture was then extracted with dichloromethane (200 mL) three times. The combined organic layers were dried over anhydrous sodium sulfate and concentrated under reduced pressure to give 11.2 g of (1*R*)-5-(5-ethyl-1,2,4-oxadiazol-3-yl)-2,3-dihydro-1*H*-inden-1-amine as a brown oil.

### (iii) Synthesis of (R)-N-(5-(5-ethyl-1,2,4-oxadiazol-3-yl)-2,3-dihydro-1H-inden-1-yl)-1-methyl-1H-pyrazole-4-carboxamide:

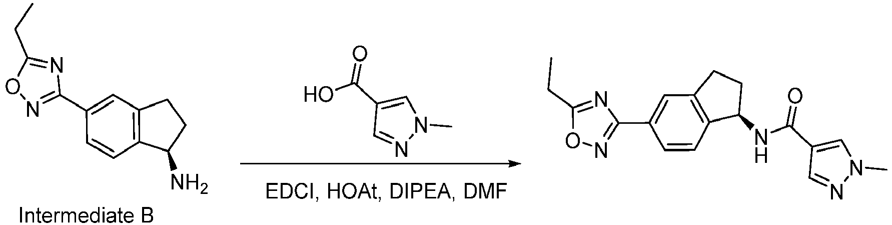

To a solution of 1-methyl-1*H*-pyrazole-4-carboxylic acid (6.1 g, 48.4 mmol, 1.0 equiv) in DMF (300 mL) were added DIEA (12.6 g, 97.5 mmol, 2.0 equiv), HOAt (19.8 g, 145.8 mmol, 3.0 equiv), and EDCI (28 g, 146.1 mmol, 3.0 equiv). The mixture was stirred for 15 min, and (1*R*)-5-(5-ethyl-1,2,4-oxadiazol-3-yl)-2,3-dihydro-1*H*-inden-1-amine (11.2 g, 48.9 mmol, 1.0 equiv) was then added. The mixture was then stirred for 3 h, diluted with DCM, washed with NH₄Cl solution three times, dried over sodium sulfate, concentrated under reduced pressure, and purified by silica gel chromatography (EA/PE, 74/26) to give an intermediate product. The intermediate product was triturated with a mixture of EA and PE (1/10) to afford 14.5 g (88%) of (*R*)-*N*-(5-(5-ethyl-1,2,4-oxadiazol-3-yl)-2,3-dihydro-1*H-*inden-1-yl)-1-methyl-1*H*-pyrazole-4-carboxamide as a white solid. LRMS (ES) m/z 338 (M+H). ¹H-NMR: (DMSO, 300MHz, *ppm*): δ 8.41 (1H, d, *J* = 8.4 Hz), 8.16 (1H, s), 7.91-7.79 (3H, m), 7.34 (1H, d, *J* = 7.9 Hz), 5.53 (1H, q, *J* = 8.3 Hz), 3.84 (3H, s), 3.13-2.81 (4H, m), 2.44 (1H, dd, *J* = 7.9, 4.7 Hz), 1.95 (1H, m), 1.33 (3H, t, *J* = 7.5 Hz).

### EXAMPLE 2 - Preparation of an Exemplary Formulation

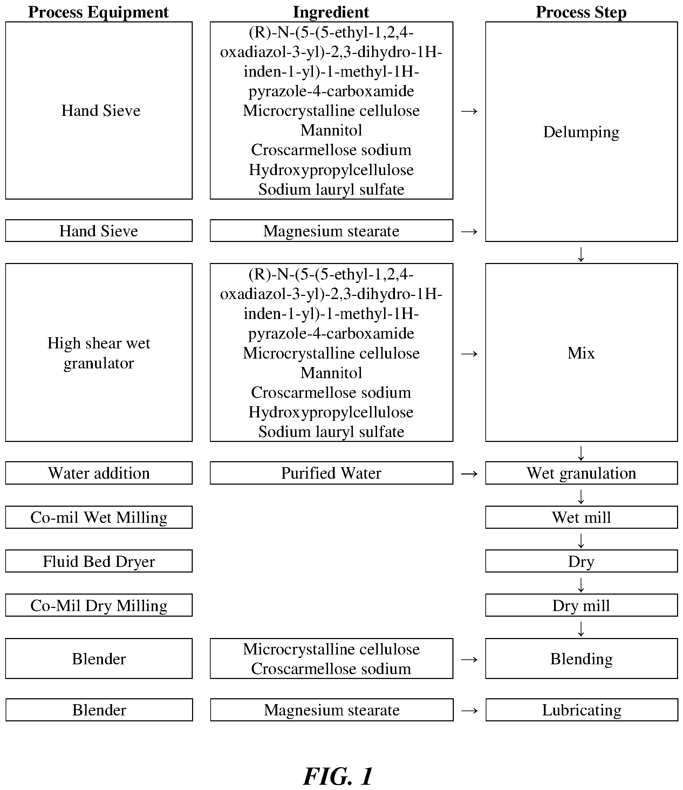

FIG. 1 presents a flow chart illustrating Unit Operations in the preparation of a formulation described herein.

(*R*)-*N*-(5-(5-ethyl-1,2,4-oxadiazol-3-yl)-2,3-dihydro-1*H*-inden-1-yl)-1-methyl-1*H*-pyrazole-4-carboxamide, microcrystalline cellulose, mannitol, croscarmellose sodium, hydroxypropylcellulose, and sodium lauryl sulfate were delumped with a hand sieve. Magnesium stearate was delumped with a hand sieve. (*R*)-*N*-(5-(5-ethyl-1,2,4-oxadiazol-3-yl)-2,3-dihydro-1*H*-inden-1-yl)-1-methyl-1*H*-pyrazole-4-carboxamide, microcrystalline cellulose, mannitol, croscarmellose sodium, hydroxypropylcellulose, and sodium lauryl sulfate were mixed in a high shear granulator, and a wet granulation step was performed, followed by wet milling, drying and dry milling. The resulting solid mixture was blended with microcrystalline cellulose and croscarmellose sodium. Finally, the resulting mixture was blended with magnesium stearate to afford a formulation comprising (*R*)-*N*-(5-(5-ethyl-1,2,4-oxadiazol-3-yl)-2,3-dihydro-1*H*-inden-1-yl)-1-methyl-1*H*-pyrazole-4-carboxamide.

### EXAMPLE 3 - Preparation of Non-Coated Tablet

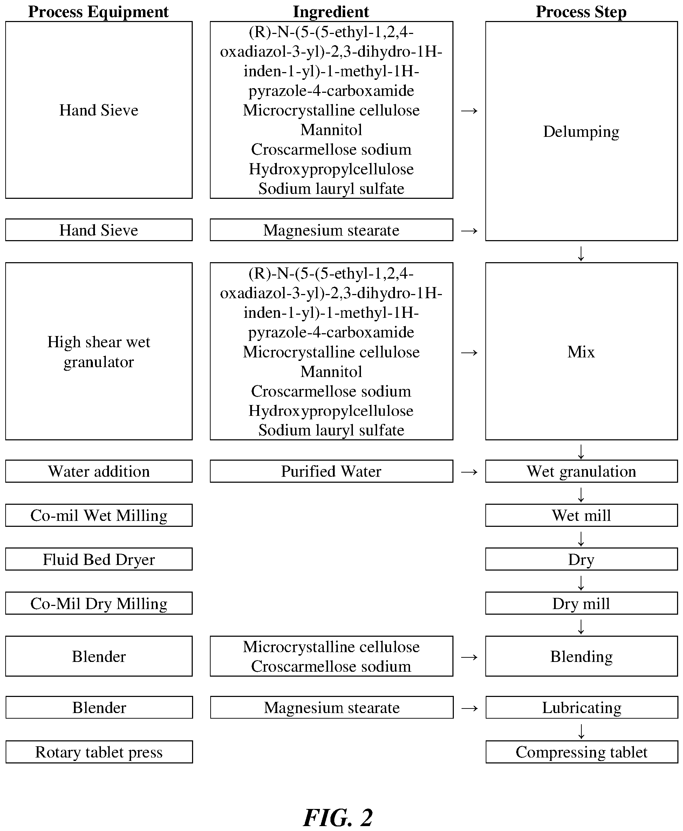

FIG. 2 presents a flow chart illustrating Unit Operations in the preparation of a non-coated tablet described herein.

(*R*)-*N*-(5-(5-ethyl-1,2,4-oxadiazol-3-yl)-2,3-dihydro-1*H*-inden-1-yl)-1-methyl-1*H-*pyrazole-4-carboxamide, microcrystalline cellulose, mannitol, croscarmellose sodium, hydroxypropylcellulose, and sodium lauryl sulfate were delumped with a hand sieve. Magnesium stearate was delumped with a hand sieve. (*R*)-*N*-(5-(5-ethyl-1,2,4-oxadiazol-3-yl)-2,3-dihydro-1*H*-inden-1-yl)-1-methyl-1*H*-pyrazole-4-carboxamide, microcrystalline cellulose, mannitol, croscarmellose sodium, hydroxypropylcellulose, and sodium lauryl sulfate were mixed in a high shear granulator, and a wet granulation step was performed, followed by wet milling, drying and dry milling. The resulting solid mixture was blended with microcrystalline cellulose and croscarmellose sodium. The resulting mixture is blended with magnesium stearate. This final mixture is then compressed into tablets to afford non-coated tablets.

### EXAMPLE 4 - Preparation of Coated Tablet

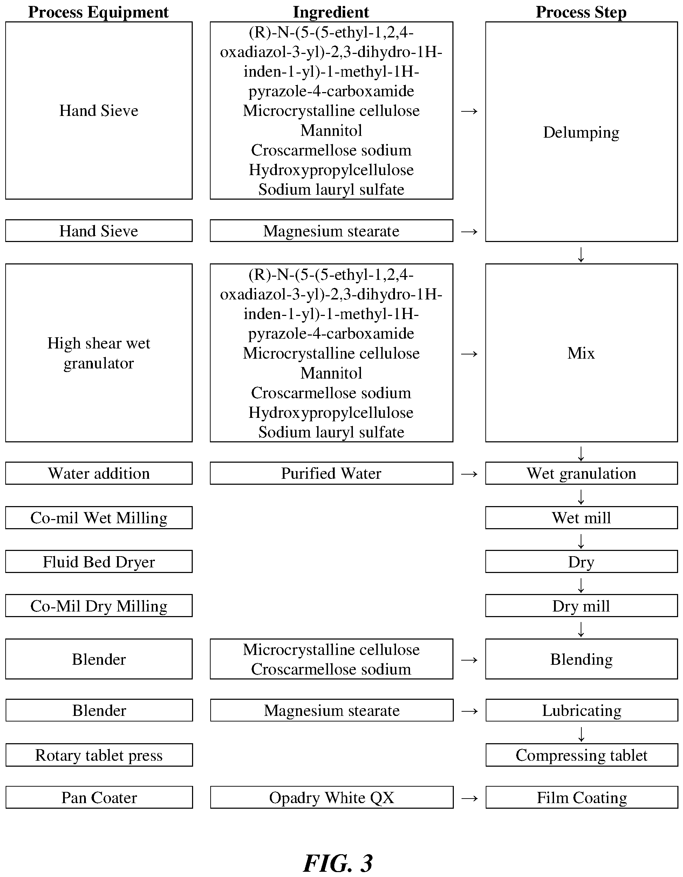

FIG. 3 presents a flow chart illustrating Unit Operations in the preparation of a coated tablet described herein.

(*R*)-*N*-(5-(5-ethyl-1,2,4-oxadiazol-3-yl)-2,3-dihydro-1*H*-inden-1-yl)-1-methyl-1*H-*pyrazole-4-carboxamide, microcrystalline cellulose, mannitol, croscarmellose sodium, hydroxypropylcellulose, and sodium lauryl sulfate were delumped with a hand sieve. Magnesium stearate was delumped with a hand sieve. (*R*)-*N*-(5-(5-ethyl-1,2,4-oxadiazol-3-yl)-2,3-dihydro-1*H*-inden-1-yl)-1-methyl-1*H*-pyrazole-4-carboxamide, microcrystalline cellulose, mannitol, croscarmellose sodium, hydroxypropylcellulose, and sodium lauryl sulfate were mixed in a high shear granulator, and a wet granulation step was performed, followed by wet milling, drying and dry milling. The resulting solid mixture was blended with microcrystalline cellulose and croscarmellose sodium. The resulting mixture is blended with magnesium stearate. This final mixture is then compressed into tablets to afford non-coated tablets. These non-coated tablets are then coated with Opadry White QX to afford coated tablets.

### EXAMPLE 5 - 2.5 mg Formulation

Tablets comprising 5 mg of (R)-N-(5-(5-ethyl-1,2,4-oxadiazol-3-yl)-2,3-dihydro-1H-inden-1-yl)-1-methyl-1H-pyrazole-4-carboxamide were prepared according to the process of Example 4, a flowchart of which is shown in FIG. 3. The following ingredients and quantities were used in this process.

**Table 1**

| **Item Description** | **Function** | **% (w/w)** | **2.5mg/tablet** |
|---|---|---|---|
| **Intra-Granular** | | | |
| (R)-N-(5-(5-ethyl-1,2,4-oxadiazol-3-yl)-2,3-dihydro-1H-inden-1-yl)-1-methyl-1H-pyrazole-4-carboxamide API | API | 5.0 | 2.5 |
| Mannitol USP (Mannogen EZ SD) | Filler | 54.0 | 27.0 |
| Microcrystalline Cellulose, NF (Avicel PH 101) | Filler | 20.0 | 10.0 |
| Croscarmellose Sodium NF (AC-Di-Sol) | Disintegrant | 3.0 | 1.5 |
| Hydroxypropyl Cellulose, NF (Klucel EXF) | Binder | 2.0 | 1.0 |
| Sodium Lauryl Sulfate NF (Kolliphor) Fine Grade | Surfactant | 2.0 | 1.0 |

| **Extra-Granular** | | | |
|---|---|---|---|
| Microcrystalline Cellulose, NF (Avicel PH 102) | Filler | 11.0 | 5.5 |
| Croscarmellose Sodium NF (AC-Di-Sol) | Disintegrant | 2.0 | 1.0 |
| Magnesium Stearate, Non Bov NF | Lubricant | 1.0 | 0.5 |
| **TOTAL Tablet Core** | | **100.00** | **50.00** |

| **Film Coating** | | | |
|---|---|---|---|
| Opadry QX White 21A180025 | | **3.0** | **1.5** |
| Total Film Coated Tablet | | **103.0** | **51.5** |

### EXAMPLE 6-5 mg Formulation

Tablets comprising 5 mg of (R)-N-(5-(5-ethyl-1,2,4-oxadiazol-3-yl)-2,3-dihydro-1H-inden-1-yl)-1-methyl-1H-pyrazole-4-carboxamide were prepared according to the process of Example 4, a flowchart of which is shown in FIG. 3. The following ingredients and quantities were used in this process.

**Table 2**

| **Item Description** | **Function** | **% (w/w)** | **5mg/tablet** |
|---|---|---|---|
| **Intra-Granular** | | | |
| (R)-N-(5-(5-ethyl-1,2,4-oxadiazol-3-yl)-2,3-dihydro-1H-inden-1-yl)-1-methyl-1H-pyrazole-4-carboxamide API | API | 5.0 | 5.0 |
| Mannitol USP (Mannogen EZ SD) | Filler | 54.0 | 54.0 |
| Microcrystalline Cellulose, NF (Avicel PH 101) | Filler | 20.0 | 20.0 |
| Croscarmellose Sodium NF (AC-Di-Sol) | Disintegrant | 3.0 | 3.0 |
| Hydroxypropyl Cellulose, NF (Klucel EXF) | Binder | 2.0 | 2.0 |
| Sodium Lauryl Sulfate NF (Kolliphor) Fine Grade | Surfactant | 2.0 | 2.0 |

| **Extra-Granular** | | | |
|---|---|---|---|
| Microcrystalline Cellulose, NF (Avicel PH 102) | Filler | 11.0 | 11.0 |
| Croscarmellose Sodium NF (AC-Di-Sol) | Disintegrant | 2.0 | 2.0 |
| Magnesium Stearate, Non Bov NF | Lubricant | 1.0 | 1.0 |
| **TOTAL Tablet Core** | | **100.00** | **100.00** |

| **Film Coating** | | | |
|---|---|---|---|
| Opadry QX White 21A180025 | | **3.0** | **3.0** |
| Total Film Coated Tablet | | **103.0** | **103.0** |

### EXAMPLE 7 - 10 mg Formulation

Tablets comprising 10 mg of (R)-N-(5-(5-ethyl-1,2,4-oxadiazol-3-yl)-2,3-dihydro-1H-inden-1-yl)-1-methyl-1H-pyrazole-4-carboxamide were prepared according to the process of Example 4, a flowchart of which is shown in FIG. 3. The following ingredients and quantities were used in this process.

**Table 3**

| **Item Description** | **Function** | **% (w/w)** | **10mg/tablet** |
|---|---|---|---|
| **Intra-Granular** | | | |
| (R)-N-(5-(5-ethyl-1,2,4-oxadiazol-3-yl)-2,3-dihydro-1H-inden-1-yl)-1-methyl-1H-pyrazole-4-carboxamide API | API | 5.0 | 10.0 |
| Mannitol USP (Mannogen EZ SD) | Filler | 54.0 | 108.0 |
| Microcrystalline Cellulose, NF (Avicel PH 101) | Filler | 20.0 | 40.0 |
| Croscarmellose Sodium NF (AC-Di-Sol) | Disintegrant | 3.0 | 6.0 |
| Hydroxypropyl Cellulose, NF (Klucel EXF) | Binder | 2.0 | 4.0 |
| Sodium Lauryl Sulfate NF (Kolliphor) Fine Grade | Surfactant | 2.0 | 4.0 |

| **Extra-Granular** | | | |
|---|---|---|---|
| Microcrystalline Cellulose, NF (Avicel PH 102) | Filler | 11.0 | 22.0 |
| Croscarmellose Sodium NF (AC-Di-Sol) | Disintegrant | 2.0 | 4.0 |
| Magnesium Stearate, Non Bov NF | Lubricant | 1.0 | 2.0 |
| **TOTAL Tablet Core** | | **100.00** | **200.00** |

| **Film Coating** | | | |
|---|---|---|---|
| Opadry QX White 21A180025 | | **3.0** | **6.0** |
| Total Film Coated Tablet | | **103.0** | **206.0** |

### EXAMPLE 8 - 20 mg Formulation

Tablets comprising 20 mg of (R)-N-(5-(5-ethyl-1,2,4-oxadiazol-3-yl)-2,3-dihydro-1H-inden-1-yl)-1-methyl-1H-pyrazole-4-carboxamide were prepared according to the process of Example 4, a flowchart of which is shown in FIG. 3. The following ingredients and quantities were used in this process.

**Table 4**

| **Item Description** | **Function** | **% (w/w)** | **20mg/tablet** |
|---|---|---|---|
| **Intra-Granular** | | | |
| (R)-N-(5-(5-ethyl-1,2,4-oxadiazol-3-yl)-2,3-dihydro-1H-inden-1-yl)-1-methyl-1H-pyrazole-4-carboxamide API | API | 20.0 | 20.0 |
| Mannitol USP (Mannogen EZ SD) | Filler | 44.0 | 44.0 |
| Microcrystalline Cellulose, NF (Avicel PH 101) | Filler | 15.0 | 15.0 |
| Croscarmellose Sodium NF (AC-Di-Sol) | Disintegrant | 3.0 | 3.0 |
| Hydroxypropyl Cellulose, NF (Klucel EXF) | Binder | 2.0 | 2.0 |
| Sodium Lauryl Sulfate NF (Kolliphor) Fine Grade | Surfactant | 2.0 | 2.0 |

| **Extra-Granular** | | | |
|---|---|---|---|
| Microcrystalline Cellulose, NF (Avicel PH 102) | Filler | 11.0 | 11.0 |
| Croscarmellose Sodium NF (AC-Di-Sol) | Disintegrant | 2.0 | 2.0 |
| Magnesium Stearate, Non Bov NF | Lubricant | 1.0 | 1.0 |
| **TOTAL Tablet Core** | | **100.00** | **100.00** |

| **Film Coating** | | | |
|---|---|---|---|
| Opadry QX White 21A180025 | | **3.0** | **3.0** |
| Total Film Coated Tablet | | **103.0** | **103.0** |

### EXAMPLE 9-40 mg Formulation

Tablets comprising 40 mg of (R)-N-(5-(5-ethyl-1,2,4-oxadiazol-3-yl)-2,3-dihydro-1H-inden-1-yl)-1-methyl-1H-pyrazole-4-carboxamide were prepared according to the process of Example 4, a flowchart of which is shown in FIG. 3. The following ingredients and quantities were used in this process.

**Table 5**

| **Item Description** | **Function** | **% (w/w)** | **40mg/tablet** |
|---|---|---|---|
| **Intra-Granular** | | | |
| (R)-N-(5-(5-ethyl-1,2,4-oxadiazol-3-yl)-2,3-dihydro-1H-inden-1-yl)-1-methyl-1H-pyrazole-4-carboxamide API | API | 20.0 | 40.0 |
| Mannitol USP (Mannogen EZ SD) | Filler | 44.0 | 88.0 |
| Microcrystalline Cellulose, NF (Avicel PH 101) | Filler | 15.0 | 30.0 |
| Croscarmellose Sodium NF (AC-Di-Sol) | Disintegrant | 3.0 | 6.0 |
| Hydroxypropyl Cellulose, NF (Klucel EXF) | Binder | 2.0 | 4.0 |
| Sodium Lauryl Sulfate NF (Kolliphor) Fine Grade | Surfactant | 2.0 | 4.0 |

| **Extra-Granular** | | | |
|---|---|---|---|
| Microcrystalline Cellulose, NF (Avicel PH 102) | Filler | 11.0 | 22.0 |
| Croscarmellose Sodium NF (AC-Di-Sol) | Disintegrant | 2.0 | 4.0 |
| Magnesium Stearate, Non Bov NF | Lubricant | 1.0 | 2.0 |
| **TOTAL Tablet Core** | | **100.00** | **200.00** |

| **Film Coating** | | | |
|---|---|---|---|
| Opadry QX White 21A180025 | | **3.0** | **6.0** |
| Total Film Coated Tablet | | **103.0** | **206.0** |

### EXAMPLE 10 - 7 mg Formulation

Tablets comprising 7 mg of (R)-N-(5-(5-ethyl-1,2,4-oxadiazol-3-yl)-2,3-dihydro-1H-inden-1-yl)-1-methyl-1H-pyrazole-4-carboxamide were prepared according to the process of Example 4, a flowchart of which is shown in FIG. 3. The following ingredients and quantities were used in this process.

**Table 6**

| **Item Description** | **Function** | **% (w/w)** | **7mg/tablet** |
|---|---|---|---|
| **Intra-Granular** | | | |
| (R)-N-(5-(5-ethyl-1,2,4-oxadiazol-3-yl)-2,3-dihydro-1H-inden-1-yl)-1-methyl-1H-pyrazole-4-carboxamide API | API | 10 | 7.0 |
| Mannitol USP (Mannogen EZ SD) | Filler | 50.0 | 35.0 |
| Microcrystalline Cellulose, NF (Avicel PH 101) | Filler | 19.0 | 13.3 |
| Croscarmellose Sodium NF (AC-Di-Sol) | Disintegrant | 3.0 | 2.1 |
| Hydroxypropyl Cellulose, NF (Klucel EXF) | Binder | 2.0 | 1.4 |
| Sodium Lauryl Sulfate NF (Kolliphor) Fine Grade | Surfactant | 2.0 | 1.4 |

| **Extra-Granular** | | | |
|---|---|---|---|
| Microcrystalline Cellulose, NF (Avicel PH 102) | Filler | 11.0 | 7.7 |
| Croscarmellose Sodium NF (AC-Di-Sol) | Disintegrant | 2.0 | 1.4 |
| Magnesium Stearate, Non Bov NF | Lubricant | 1.0 | 0.7 |
| **TOTAL Tablet Core** | | **100.00** | **70.00** |

| **Film Coating** | | | |
|---|---|---|---|
| Opadry QX White 21A180025 | | **3.0** | **2.1** |
| Total Film Coated Tablet | | **103.0** | **72.1** |

### EXAMPLE 11 - 40 mg Formulation

Tablets comprising 40 mg of (R)-N-(5-(5-ethyl-1,2,4-oxadiazol-3-yl)-2,3-dihydro-1H-inden-1-yl)-1-methyl-1H-pyrazole-4-carboxamide were prepared according to the process of Example 4, a flowchart of which is shown in FIG. 3. The following ingredients and quantities were used in this process

**Table 7**

| **Item Description** | **Function** | **% (w/w)** | **40mg/tablet** |
|---|---|---|---|
| **Intra-Granular** | | | |
| (R)-N-(5-(5-ethyl-1,2,4-oxadiazol-3-yl)-2,3-dihydro-1H-inden-1-yl)-1-methyl-1H-pyrazole-4-carboxamide API | API | 10 | 40.0 |
| Mannitol USP (Mannogen EZ SD) | Filler | 50.0 | 200.0 |
| Microcrystalline Cellulose, NF (Avicel PH 101) | Filler | 19.0 | 76.0 |
| Croscarmellose Sodium NF (AC-Di-Sol) | Disintegrant | 3.0 | 12.0 |
| Hydroxypropyl Cellulose, NF (Klucel EXF) | Binder | 2.0 | 8.0 |
| Sodium Lauryl Sulfate NF (Kolliphor) Fine Grade | Surfactant | 2.0 | 8.0 |

| **Extra-Granular** | | | |
|---|---|---|---|
| Microcrystalline Cellulose, NF (Avicel PH 102) | Filler | 11.0 | 44.0 |
| Croscarmellose Sodium NF (AC-Di-Sol) | Disintegrant | 2.0 | 8.0 |
| Magnesium Stearate, Non Bov NF | Lubricant | 1.0 | 4.0 |
| **TOTAL Tablet Core** | | **100.00** | **400.00** |

| **Film Coating** | | | |
|---|---|---|---|
| Opadry QX White 21A180025 | | **3.0** | **12.0** |
| Total Film Coated Tablet | | **103.0** | **412.0** |

### EXAMPLE 11 - Long-term Stability of 5 mg Formulation

Tablets comprising 5 mg of (R)-N-(5-(5-ethyl-1,2,4-oxadiazol-3-yl)-2,3-dihydro-1H-inden-1-yl)-1-methyl-1H-pyrazole-4-carboxamide as described in Example 6. The tablets were packaged in 30 cc white round-wide mouth HDPE bottles with induction seal and 28 mm Securx cap, SG-75M liner. Each bottle contained 40 tables. The tablets were stored for 12 months at 30 °C +/- 2 °C and 65% RH +/- 5% RH. After the 12 months period the tablets were analyzed for appearance, potency, presence of unknown compounds, ability to release the active ingredient upon dissolution, and water content. Results are shown in Table 8.

**Table 8**

| **Test** | **Acceptance Criteria** | **Results** |
|---|---|---|
| Appearance | white to off-white, round, film-coated tablet | white, round, film-coated tablet |
| Potency (mean) | 90 % - 110 % of label claim (%LC) based on 5 mg (R)-N-(5-(5-ethyl-1,2,4-oxadiazol-3-yl)-2,3-dihydro-1H-inden-1-yl)-1-methyl-1H-pyrazole-4-carboxamide | 101.7 %LC |
| Unspecified Unknown (RRT 0.89) Compounds %RS | Report Results | <QL |
| Related Substance Total %RS | Report Results | <QL |
| Dissolution, 10 min (mean) | N/A | 98 % released |
| Dissolution, 20 min (mean) | N/A | 101 % released |
| Dissolution, 30 min (mean) | N/A | 101 % released |
| Dissolution, 45 min (mean) | N/A | 101 % released |
| Dissolution, 60 min (mean) | N/A | 101 % released |
| Dissolution, infinity (mean) | N/A | 102 % released |
| Water Content | N/A | 1.5 % |

| | | |
|---|---|---|
| RRT: relative retention time as determined by HPLC %RS: percentage of related substance as determinded by HPLC surface area | | |

### EXAMPLE 12 - Long-term Stability of 40 mg Formulation

Tablets comprising 40 mg of (R)-N-(5-(5-ethyl-1,2,4-oxadiazol-3-yl)-2,3-dihydro-1H-inden-1-yl)-1-methyl-1H-pyrazole-4-carboxamide as described in Example 9. The tablets were packaged in 30 cc white round-wide mouth HDPE bottles with induction seal and 28 mm Securx cap, SG-75M liner. Each bottle contained 40 tables. The tablets were stored for 12 months at 30 °C +/- 2 °C and 65% RH +/- 5% RH. After the 12 months period the tablets were analyzed for appearance, potency, presence of unknown compounds, ability to release the active ingredient upon dissolution, and water content. Results are shown in Table 9.

**Table 9**

| **Test** | **Acceptance Criteria** | **Results** |
|---|---|---|
| Appearance | white to off-white, oval, film-coated tablet | white, oval, film-coated tablet |
| Potency (mean) | 90 % - 110 % of label claim (%LC) based on 40 mg (R)-N-(5-(5-ethyl-1,2,4-oxadiazol-3-yl)-2,3-dihydro-1H-inden-1-yl)-1-methyl-1H-pyrazole-4-carboxamide | 103.6 %LC |
| Unspecified Unknown (RRT 0.89) Compounds %RS | Report Results | <QL |
| Related Substance Total %RS | Report Results | <QL |
| Dissolution, 10 min (mean) | N/A | 90 % released |
| Dissolution, 20 min (mean) | N/A | 97 % released |
| Dissolution, 30 min (mean) | N/A | 99 % released |
| Dissolution, 45 min (mean) | N/A | 100 % released |
| Dissolution, 60 min (mean) | N/A | 100 % released |
| Dissolution, infinity (mean) | N/A | 101 % released |
| Water Content | N/A | 1.2 % |

| | | |
|---|---|---|
| RRT: relative retention time as determined by HPLC %RS: percentage of related substance as determinded by HPLC surface area | | |

### BIOLOGICAL EXAMPLE B-1

### MYOFIBRIL ASSAYS

To evaluate the effect of (R)-N-(5-(5-ethyl-1,2,4-oxadiazol-3-yl)-2,3-dihydro-1H-inden-1-yl)-1-methyl-1H-pyrazole-4-carboxamide on the ATPase activity of full-length cardiac myosin in the context of the native sarcomere, skinned myofibril assays were performed. Bovine cardiac myofibrils were obtained by homogenizing bovine cardiac left ventricular tissue in the presence of a detergent such as triton X-100. Such treatment removes membranes and a majority of the soluble cytoplasmic proteins but leaves intact the cardiac sarcomeric acto-myosin apparatus. Myofibril preparations retain the ability to hydrolyze ATP in a Ca²⁺ regulated manner. ATPase activities of such myofibril preparations in the presence and absence of (R)-N-(5-(5-ethyl-1,2,4-oxadiazol-3-yl)-2,3-dihydro-1H-inden-1-yl)-1-methyl-1H-pyrazole-4-carboxamide were assayed at Ca²⁺ concentrations activating to a defined fraction of the maximal rate (i.e., 25%, 75%). (R)-N-(5-(5-ethyl-1,2,4-oxadiazol-3-yl)-2,3-dihydro-1H-inden-1-yl)-1-methyl-1H-pyrazole-4-carboxamide was assessed for their ability to inhibit the steady-state ATPase activity of bovine cardiac myofibrils using pyruvate kinase and lactate dehydrogenase (PK/LDH)-coupled enzyme system. This assay regenerates myosin-produced ADP into ATP by oxidizing NADH, producing an absorbance change at 340 nm. Prior to testing (R)-N-(5-(5-ethyl-1,2,4-oxadiazol-3-yl)-2,3-dihydro-1H-inden-1-yl)-1-methyl-1H-pyrazole-4-carboxamide, the bovine cardiac myofibrils were assessed for their calcium responsiveness and the calcium concentration that achieves either a 50% (pCa₅₀) or 75% (pCavs) activation of the myofibril system was chosen as the final condition for assessing the inhibitory activity of (R)-N-(5-(5-ethyl-1,2,4-oxadiazol-3-yl)-2,3-dihydro-1H-inden-1-yl)-1-methyl-1H-pyrazole-4-carboxamide. All enzymatic activity was measured in a buffered solution containing 12 mM PIPES (piperazine-N,N'-bis(2-ethanesulfonic acid), 2 mM magnesium chloride at pH 6.8 (PM 12 buffer). Final assay conditions were 1 mg/mL of bovine cardiac myofibrils, 4 U/mL pyruvate kinase, 6 U/mL lactate dehydrogenase, 50 µM ATP, 0.1 mg/mL BSA (bovine serum albumin), 10 ppm antifoam, 1 mM DTT, 0.5 mM NADH, 1.5 mM PEP, 0.6 mM EGTA, and an amount of CaCl₂ sufficient to achieve either 50% or 75% activation of the myofibril ATPase activity. Results for (R)-N-(5-(5-ethyl-1,2,4-oxadiazol-3-yl)-2,3-dihydro-1H-inden-1-yl)-1-methyl-1H-pyrazole-4-carboxamide are provided in Table A. (R)-N-(5-(5-ethyl-1,2,4-oxadiazol-3-yl)-2,3-dihydro-1H-inden-1-yl)-1-methyl-1H-pyrazole-4-carboxamide was prepared in accordance with the synthetic procedures described herein.

**Table A**

| CDMF75 IC₁₅ (µM) | CDMF75 IC₅₀ (µM) |
|---|---|
| 0.4 | 1.4 |

### BIOLOGICAL EXAMPLE B-2

### MYOCYTE ASSAYS

### (i) Preparation Of Adult Cardiac Ventricular Rat Myocytes:

Adult male Sprague-Dawley rats were anesthetized and the hearts were quickly excised, rinsed and the ascending aorta was cannulated. Continuous retrograde perfusion was initiated on the hearts at a perfusion pressure of 60 cm H₂O. Hearts were first perfused with a nominally Ca²⁺-free modified Krebs solution of the following composition: 113 mM NaCl, 4.7 mM KCl, 0.6 mM KH₂PO₄, 0.6 mM Na₂HPO₄, 1.2 mM MgSO₄, 12 mM NaHCO₃, 10 mM KHCO₃, 30 mM taurine, 5.5 mM glucose and 10 mM Hepes (all Sigma). This medium is not recirculated and is continually aerated with a 95% O₂/5% CO₂ mixture. After approximately 3 minutes the heart was perfused with a modified Krebs buffer supplemented with collagenase (Worthington) and 12.5 µM final calcium concentration. The heart was removed from the cannulae after the heart appeared blanched and soft in appearance. The atria and vessels were removed and the ventricles were gently dissected into smaller pieces with forceps. The tissue was homogenized by repeated pipette trituration and the collagenase reaction was stopped by 10% bovine calf serum (BCS), sedimentation and resuspension in perfusion buffer containing 5% BCS and 12.5uM CaCl₂. Myocytes were made calcium tolerant by stepwise addition of a CaCl₂ solution to a final concentration of 1.2mM. Cells were then washed and resuspended in Tyrode's buffer (137 mM NaCl, 3.7 mM KCl, 0.5 mM MgCl, 11 mM glucose, 4 mM Hepes, and 1.2 mM CaCl₂, pH 7.4). Cells were kept for 60 min at 37°C prior to initiating experiments and used within 5 hrs of isolation. Preparations of cells were used only if cells first passed QC criteria by demonstrating a contractile response to standard (>150% of basal) and isoproterenol (ISO; > 250% of basal) treatment. Additionally, only cells whose basal contractility was between 3 and 8 % were used in subsequent experiments with compounds.

### (ii) Adult Ventricular Myocyte Contractility Experiments:

Aliquots of myocytes in Tyrode's buffer were placed in perfusion chambers (series 20 RC-27NE; Warner Instruments) complete with heating platforms. Myocytes were allowed to attach, the chambers were heated to 37°C, and the cells were perfused with 37°C Tyrode's buffer. Myocytes were field stimulated at 1 Hz in with platinum electrodes (20% above threshold). Only cells that had clear striations and were quiescent prior to pacing were used for contractility experiments. To determine basal contractility, myocytes were imaged through a 40x objective. Using a variable frame rate (60-240 Hz) charge-coupled device camera, the images were digitized and displayed on a computer screen at a sampling speed of 240 Hz (IonOptix Milton, MA). Once cell contraction was stable over time, (R)-N-(5-(5-ethyl-1,2,4-oxadiazol-3-yl)-2,3-dihydro-1H-inden-1-yl)-1-methyl-1H-pyrazole-4-carboxamide (0.01 - 15 µM) was perfused into the chambers on the myocytes for 5 minutes. Contractility of the myocytes and contraction and relaxation velocities were then recorded using edge detection.

### (iii) Contractility Analysis:

Five or more individual myocytes were tested from two or more different myocyte preparations. For each cell, twenty or more contractility transients at basal (defined as 1 min prior to compound infusion) and after compound addition (defined as 5 min after starting compound perfusion), were averaged and compared. These average transients were analyzed using the Ion Wizard software (IonOptix) to determine changes in diastolic length and fractional shortening. Fractional shortening was calculated as: ((resting length - length at peak contraction) divided by the resting length). The percent change in fractional shortening from baseline was calculated as: ((post-dose fractional shortening / basal fractional shortening)* 100). The percent reduction in fractional shortening from baseline was calculated as: (100 - percent change in fractional shortening from baseline). Maximum contraction and relaxation velocities (um/sec) was also determined. Results from individual cells are averaged and the SEM calculated.

The effect of (R)-N-(5-(5-ethyl-1,2,4-oxadiazol-3-yl)-2,3-dihydro-1H-inden-1-yl)-1-methyl-1H-pyrazole-4-carboxamide on the fractional shortening (FS) of the myocytes is shown in Table B.

**Table B**

| **Concentration (µM)** | **%FS (% reduction from baseline) ± SEM** | **# of cells tested** |
|---|---|---|
| 5 | 67.4 ±-5.8 | 5 |

| | | |
|---|---|---|
| %FS = Average of each cell's (post baseline percent peak height / pre-baseline percent peak height) x 100 | | |

### BIOLOGICAL EXAMPLE B-3

### ECHOCARDIOGRAPHY ASSESSMENT OF ACUTE PHARMACODYNAMIC EFFECT IN RAT CARDIAC CONTRACTILITY

Assessment of in vivo cardiac function by echocardiography was performed in male Sprague Dawley rats under isoflurane (1-3%) anesthesia. 2-D M-mode images of the left ventricle were acquired in the parasternal long-axis view before, during, and after administration of (R)-N-(5-(5-ethyl-1,2,4-oxadiazol-3-yl)-2,3-dihydro-1H-inden-1-yl)-1-methyl-1H-pyrazole-4-carboxamide by continuous IV infusion or oral gavage. *In vivo* fractional shortening was determined by M-mode image analysis with the following calculation: ((End diastolic diameter - end systolic diameter)/ end diastolic diameter x 100). For continuous IV infusion experiments, three pre-dose baseline M-mode images were taken at 1 minute intervals prior to infusion of (R)-N-(5-(5-ethyl-1,2,4-oxadiazol-3-yl)-2,3-dihydro-1H-inden-1-yl)-1-methyl-1H-pyrazole-4-carboxamide. (R)-N-(5-(5-ethyl-1,2,4-oxadiazol-3-yl)-2,3-dihydro-1H-inden-1-yl)-1-methyl-1H-pyrazole-4-carboxamide was formulated in 50% Propylene Glycol (PG): 16% Captisol: 10% dimethylacetamide (DMA) and delivered via a jugular vein catheter at the rate of 1 mL/kg/h. During infusion, M-mode images were taken at 5 minute intervals. The infusion stopped when fractional shortening reached up to a 60% reduction from baseline. Blood samples were taken to determine the plasma concentration of (R)-N-(5-(5-ethyl-1,2,4-oxadiazol-3-yl)-2,3-dihydro-1H-inden-1-yl)-1-methyl-1H-pyrazole-4-carboxamide. Data was reported as an estimated IC₅₀ value, which is the concentration at which fractional shortening is 50% of the pre-dose baseline contractility. The IC₅₀ results are summarized in Table C.

**Table C**

| **IC₅₀ (Mean ± S.D., µM)** |
|---|
| 7.2 ± 0.20 |

For oral dosing studies, three pre-dose baseline M-Mode images were taken at 1 minute intervals prior to compound administration. (R)-N-(5-(5-ethyl-1,2,4-oxadiazol-3-yl)-2,3-dihydro-1H-inden-1-yl)-1-methyl-1H-pyrazole-4-carboxamide was formulated in a 0.5% hydroxypropyl methylcellulose 2910 (HPMC 2910): 0.1% Tween 80 suspension and delivered as a single dose (5 mL/kg) by oral gavage. Rats were lightly anesthetized for M-Mode echocardiography measurements at select time points over a 24 hour period. Different dose levels were evaluated for (R)-N-(5-(5-ethyl-1,2,4-oxadiazol-3-yl)-2,3-dihydro-1H-inden-1-yl)-1-methyl-1H-pyrazole-4-carboxamide. The compound effect on cardiac fractional shortening at the highest dose evaluated is presented in Table D as a percent reduction of baseline fractional shortening (=100%).

**Table D**

| **Dose (mg/kg)** | **FS (% reduction from baseline) at 1-2h post dose (Mean ± S.D.)** | **FS (% reduction from baseline) at 4h post dose (Mean ± S.D.)** |
|---|---|---|
| 2 | 43 ± 9 | 31 ± 9 |

Concurrent with echocardiography measurements, blood samples were taken to determine the corresponding (R)-N-(5-(5-ethyl-1,2,4-oxadiazol-3-yl)-2,3-dihydro-1H-inden-1-yl)-1-methyl-1H-pyrazole-4-carboxamide plasma concentration. The data in Table E summarizes the estimated IC₅₀ and IC₁₀ values, which is the concentration at which fractional shortening is 50% and 10% of the pre-dose baseline contractility, respectively.

**Table E**

| **IC₅₀ (µM)** | **IC₁₀ (µM)** |
|---|---|
| 7.9 | 0.8 |

While the foregoing written description of the compounds, uses, and methods described herein enables one of ordinary skill in the art to make and use the compounds, uses, and methods described herein, those of ordinary skill in the art will understand and appreciate the existence of variations, combinations, and equivalents of the specific embodiment, method, and examples herein. The compounds, uses, and methods provided herein should therefore not be limited by the above-described embodiments, methods, or examples, but rather encompasses all embodiments and methods within the scope of the claims.

## Claims

1. A formulation comprising:
(i) (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(ii) a filler;
(iii) a binder;
(iv) a disintegrant;
(v) a surfactant; and
(vi) a lubricant.

2. The formulation of claim 1, wherein (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof, is (R)-N-(5-(5-ethyl-1,2,4-oxadiazol-3-yl)-2,3-dihydro-1H-inden-1-yl)-1-methyl-1H-pyrazole-4-carboxamide.

3. The formulation of claim 1 or 2, wherein:
(a) the filler is selected from the group consisting of powdered cellulose, microcrystalline cellulose, silicified microcrystalline cellulose, kaolin, corn starch, maize starch, starch derivatives, pregelatinized starch, calcium phosphate, calcium hydrogen phosphate, dicalcium phosphate, tricalcium phosphate, compressible sugar, sugar alcohol, mannitol, sorbitol, maltitol, xylitol, lactitol, lactose, dextrose, maltose, sucrose, glucose, fructose, saccharose, raffinose, dextrates, trehalose, maltodextrines, and mixtures of any of the foregoing; and/or
(b) the binder is selected from the group consisting of arabic gum, acacia gum, alginate, alginic acid, corn starch, copolyvidone, polyvinylpyrrolidone, gelatin, glyceryl behenate, hydroxyethylcellulose, hydroxypropyl cellulose, carboxymethylcellulose, carboxymethylcellulose calcium,
carboxymethylcellulose sodium, methylcellulose, hypromellose, lactose, polyvinyl alcohol, povidone, polyethylene oxide, polyacrylates, potato starch, pregelatinized starch, sodium alginate, sodium starch, sodium carboxy methyl cellulose, starch, and mixtures of any of the foregoing; and/or
(c) the disintegrant is selected from the group consisting of alginic acid, croscarmellose sodium, cellulose, carboxymethylcellulose calcium, carboxymethylcellulose sodium, microcrystalline cellulose, crospovidone, sodium starch glycolate, low-substituted hydroxypropyl cellulose, polacrillin potassium, pregelatinized starch, partially hydrolyzed starch, sodium carboxymethyl starch, starch, sodium alginate, sodium carboxy methyl cellulose, and mixtures of any of the foregoing; and/or
(d) the surfactant is selected from the group consisting of cetylpyridine chloride, heptadecaethylene oxycetanol, lecithins, polyoxyethylene stearate, nonoxynol 9, nonoxynol 10, octoxynol 9, sorbitan fatty acid esters, Span 20, Span 40, Span 60, Span 80, Span 85, polysorbates, polysorbate 20, polysorbate 21, polysorbate 40, polysorbate 60, polysorbate 61, polysorbate 65, polysorbate 80, sodium salts of fatty alcohol sulfates, sodium lauryl sulfate, sodium salts of sulfosuccinates, sodium dioctylsulfosuccinate, partial esters of fatty acids with alcohols, glycerine monostearate, glyceryl monooleate, ethers of fatty alcohols with polyoxyethylene, esters of fatty acids with polyoxyethylene, copolymers of ethylenoxide and propylenoxide (Pluronic^{®}), benzalkonium chloride, ethoxylated triglycerides, and mixtures of any of the foregoing; and/or
(e) the lubricant is selected from the group consisting of hydrogenated castor oil, magnesium stearate, glyceryl monostearate, calcium stearate, glyceryl behenate, glycerol distearate, glyceryl dipalmitostearate, behenoyl polyoxyl-8 glycerides, sodium stearyl fumarate, stearic acid, talc, zinc stearate, mineral oil, polyethylene glycol, polaxamer, sodium lauryl sulfate, and mixtures of any of the foregoing; and/or
(f) the formulation comprises:
(i) about 1 % by weight to about 80 % by weight of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(ii) about 15 % by weight to about 90 % by weight of the filler;
(iii) about 0.1 % by weight to about 10 % by weight of the binder;
(iv) about 1 % by weight to about 10 % by weight of the disintegrant;
(v) about 0.1 % by weight to about 10 % by weight of the surfactant; and
(vi) about 0.1 % by weight to about 10 % by weight of the lubricant.

4. The formulation of any one of claims 1-3, wherein the formulation comprises:
(a)
(i) about 20 % by weight of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(ii) about 70 % by weight of the filler;
(iii) about 2 % by weight of the binder;
(iv) about 5 % by weight of the disintegrant;
(v) about 2 % by weight of the surfactant; and
(vi) about 1 % by weight of the lubricant;
(b)
(i) about 10 % by weight of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(ii) about 80 % by weight of the filler;
(iii) about 2 % by weight of the binder;
(iv) about 5 % by weight of the disintegrant;
(v) about 2 % by weight of the surfactant; and
(vi) about 1 % by weight of the lubricant;
(c)
(i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(ii) about 85 % by weight of the filler;
(iii) about 2 % by weight of the binder;
(iv) about 5 % by weight of the disintegrant;
(v) about 2 % by weight of the surfactant; and
(vi) about 1 % by weight of the lubricant;
(d)
(i) about 20 % by weight of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(ii-1) about 44 % by weight of mannitol;
(ii-2) about 26 % by weight of microcrystalline cellulose;
(iii) about 2 % by weight of hydroxypropyl cellulose;
(iv) about 5 % by weight of croscarmellose sodium;
(v) about 2 % by weight of sodium lauryl sulfate; and
(vi) about 1 % by weight of magnesium stearate;
(e)
(i) about 10 % by weight of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(ii-1) about 50 % by weight of mannitol;
(ii-2) about 30 % by weight of microcrystalline cellulose;
(iii) about 2 % by weight of hydroxypropyl cellulose;
(iv) about 5 % by weight of croscarmellose sodium;
(v) about 2 % by weight of sodium lauryl sulfate; and
(vi) about 1 % by weight of magnesium stearate; or
(f)
(i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(ii-1) about 54 % by weight of mannitol;
(ii-2) about 31 % by weight of microcrystalline cellulose;
(iii) about 2 % by weight of hydroxypropyl cellulose;
(iv) about 5 % by weight of croscarmellose sodium;
(v) about 2 % by weight of sodium lauryl sulfate; and
(vi) about 1 % by weight of the magnesium stearate.

5. The formulation of any of the preceding claims, wherein the formulation is for oral administration; and/or wherein the formulation is for administration once daily.

6. A tablet comprising:
(i) a core having a total core weight comprising:
(a) an intra-granular component comprising:
(a-i) (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(a-ii) an intra-granular filler;
(a-iii) an intra-granular binder;
(a-iv) an intra-granular disintegrant; and
(a-v) an intra-granular surfactant;
and
(b) an extra-granular component comprising:
(b-i) an extra-granular filler;
(b-ii) an extra-granular disintegrant; and
(b-iii) an extra-granular lubricant;
and optionally
(ii) a coating layer comprising a coating agent.

7. The tablet of claim 6, wherein:
(a) the intra-granular filler is selected from the group consisting of powdered cellulose, microcrystalline cellulose, silicified microcrystalline cellulose, kaolin, corn starch, maize starch, starch derivatives, pregelatinized starch, calcium phosphate, calcium hydrogen phosphate, dicalcium phosphate, tricalcium phosphate, compressible sugar, sugar alcohol, mannitol, sorbitol, maltitol, xylitol, lactitol, lactose, dextrose, maltose, sucrose, glucose, fructose, saccharose, raffinose, dextrates, trehalose, maltodextrines, and mixtures of any of the foregoing; and/or
(b) the intra-granular binder is selected from the group consisting of arabic gum, acacia gum, alginate, alginic acid, corn starch, copolyvidone, polyvinylpyrrolidone, gelatin, glyceryl behenate, hydroxyethylcellulose, hydroxypropyl cellulose, carboxymethylcellulose, carboxymethylcellulose calcium, carboxymethylcellulose sodium, methylcellulose, hypromellose, lactose, polyvinyl alcohol, povidone, polyethylene oxide, polyacrylates, potato starch, pregelatinized starch, sodium alginate, sodium starch, sodium carboxy methyl cellulose, starch, and mixtures of any of the foregoing; and/or
(c) the intra-granular disintegrant is selected from the group consisting of alginic acid, croscarmellose sodium, cellulose, carboxymethylcellulose calcium, carboxymethylcellulose sodium, microcrystalline cellulose, crospovidone, sodium starch glycolate, low-substituted hydroxypropyl cellulose, polacrillin potassium, pregelatinized starch, partially hydrolyzed starch, sodium carboxymethyl starch, starch, sodium alginate, sodium carboxy methyl cellulose, and mixtures of any of the foregoing; and/or
(d) the intra-granular surfactant is selected from the group consisting of cetylpyridine chloride, heptadecaethylene oxycetanol, lecithins, polyoxyethylene stearate, nonoxynol 9, nonoxynol 10, octoxynol 9, sorbitan fatty acid esters, Span 20, Span 40, Span 60, Span 80, Span 85, polysorbates, polysorbate 20, polysorbate 21, polysorbate 40, polysorbate 60, polysorbate 61, polysorbate 65, polysorbate 80, sodium salts of fatty alcohol sulfates, sodium lauryl sulfate, sodium salts of sulfosuccinates, sodium dioctylsulfosuccinate, partial esters of fatty acids with alcohols, glycerine monostearate, glyceryl monooleate, ethers of fatty alcohols with polyoxyethylene, esters of fatty acids with polyoxyethylene, copolymers of ethylenoxide and propylenoxide (Pluronic^{®}), benzalkonium chloride, ethoxylated triglycerides, and mixtures of any of the foregoing; and/or
(e) the extra-granular filler is selected from the group consisting of powdered cellulose, microcrystalline cellulose, silicified microcrystalline cellulose, kaolin, corn starch, maize starch, starch derivatives, pregelatinized starch, calcium phosphate, calcium hydrogen phosphate, dicalcium phosphate, tricalcium phosphate, compressible sugar, sugar alcohol, mannitol, sorbitol, maltitol, xylitol, lactitol, lactose, dextrose, maltose, sucrose, glucose, fructose, saccharose, raffinose, dextrates, trehalose, maltodextrines, and mixtures of any of the foregoing; and/or
(f) the extra-granular disintegrant is selected from the group consisting of alginic acid, croscarmellose sodium, cellulose, carboxymethylcellulose calcium, carboxymethylcellulose sodium, microcrystalline cellulose, crospovidone, sodium starch glycolate, low-substituted hydroxypropyl cellulose, polacrillin potassium, pregelatinized starch, partially hydrolyzed starch, sodium carboxymethyl starch, starch, sodium alginate, sodium carboxy methyl cellulose, and mixtures of any of the foregoing; and/or
(g) the extra-granular lubricant is selected from the group consisting of hydrogenated castor oil, magnesium stearate, glyceryl monostearate, calcium stearate, glyceryl behenate, glycerol distearate, glyceryl dipalmitostearate, behenoyl polyoxyl-8 glycerides, sodium stearyl fumarate, stearic acid, talc, zinc stearate, mineral oil, polyethylene glycol, polaxamer, sodium lauryl sulfate, and mixtures of any of the foregoing.

8. The tablet of any one of claims 6-7, wherein the core comprises:
(a) an intra-granular component comprising:
(a-i) about 1 % to about 80 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(a-ii) about 10 % to about 80 % of the total core weight of the intra-granular filler;
(a-iii) about 0.1 % to about 10 % of the total core weight of the intra-granular binder;
(a-iv) about 0.1 % to about 5 % of the total core weight of the intra-granular disintegrant; and
(a-v) about 0.1 % to about 5 % of the total core weight of the intra-granular surfactant;
and
(b) an extra-granular component comprising:
(b-i) about 5 % to about 15 % of the total core weight of the extra-granular filler;
(b-ii) about 0.1 % to about 5 % of the total core weight of the extra-granular disintegrant; and
(b-iii) about 0.1 % to about 5 % of the total core weight of the extra-granular lubricant.

9. The tablet of any one of claims 6-8, wherein the core comprises:
(A)
(a) an intra-granular component comprising:
(a-i) about 20 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(a-ii-1) about 44 % of the total core weight of mannitol;
(a-ii-2) about 15 % of the total core weight of microcrystalline cellulose;
(a-iii) about 2 % of the total core weight of hydroxypropyl cellulose;
(a-iv) about 3 % of the total core weight of croscarmellose sodium; and
(a-v) about 2 % of the total core weight of sodium lauryl sulfate;
and
(b) an extra-granular component comprising:
(b-i) about 11 % of the total core weight of microcrystalline cellulose;
(b-ii) about 2 % of the total core weight of croscarmellose sodium; and
(b-iii) about 1 % of the total core weight of magnesium stearate;
(B)
(a) an intra-granular component comprising:
(a-i) about 10 % of the total core weight of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(a-ii-1) about 50 % of the total core weight of mannitol;
(a-ii-2) about 19 % of the total core weight of microcrystalline cellulose;
(a-iii) about 2 % of the total core weight of hydroxypropyl cellulose;
(a-iv) about 3 % of the total core weight of croscarmellose sodium; and
(a-v) about 2 % of the total core weight of sodium lauryl sulfate;
and
(b) an extra-granular component comprising:
(b-i) about 11 % of the total core weight of microcrystalline cellulose;
(b-ii) about 2 % of the total core weight of croscarmellose sodium; and
(b-iii) about 1 % of the total core weight of magnesium stearate; or
(C)
(a) an intra-granular component comprising:
(a-i) about 5 % by weight of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(a-ii-1) about 54 % by weight of mannitol;
(a-ii-2) about 20 % by weight of microcrystalline cellulose;
(a-iii) about 2 % by weight of hydroxypropyl cellulose;
(a-iv) about 3 % by weight of croscarmellose sodium; and
(a-v) about 2 % by weight of sodium lauryl sulfate;
and
(b) an extra-granular component comprising:
(b-i) about 11 % by weight of microcrystalline cellulose;
(b-ii) about 2 % by weight of croscarmellose sodium; and
(b-iii) about 1 % by weight of magnesium stearate.

10. The tablet of any one of claims 6-9, comprising a coating layer comprising a coating agent; for example wherein the coating agent is selected from the group consisting of Opadry QX White 21A180025, Opadry I, and Opadry II; for example wherein the coating agent is Opadry QX White 21A180025.

11. The tablet of claim 6-8, wherein the core comprises:
(A)
(a) an intra-granular component comprising:
(a-i) about 2.5 mg of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(a-ii-1) about 27 mg of mannitol;
(a-ii-2) about 10 mg of microcrystalline cellulose;
(a-iii) about 1 mg of hydroxypropyl cellulose;
(a-iv) about 1.5 mg of croscarmellose sodium; and
(a-v) about 1 mg of sodium lauryl sulfate;
and
(b) an extra-granular component comprising:
(b-i) about 5.5 mg of microcrystalline cellulose;
(b-ii) about 1 mg of croscarmellose sodium; and
(b-iii) about 0.5 mg of magnesium stearate;
(B)
(a) an intra-granular component comprising:
(a-i) about 20 mg of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(a-ii-1) about 44 mg of the total core weight of mannitol;
(a-ii-2) about 15 mg of the total core weight of microcrystalline cellulose;
(a-iii) about 2 mg of the total core weight of hydroxypropyl cellulose;
(a-iv) about 3 mg of the total core weight of croscarmellose sodium; and
(a-v) about 2 mg of the total core weight of sodium lauryl sulfate;
and
(b) an extra-granular component comprising:
(b-i) about 11 mg of the total core weight of microcrystalline cellulose;
(b-ii) about 2 mg of the total core weight of croscarmellose sodium; and
(b-iii) about 1 mg of the total core weight of magnesium stearate;
(C)
(a) an intra-granular component comprising:
(a-i) about 5 mg of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(a-ii-1) about 54 mg of mannitol;
(a-ii-2) about 20 mg of microcrystalline cellulose;
(a-iii) about 2 mg of hydroxypropyl cellulose;
(a-iv) about 3 mg of croscarmellose sodium; and
(a-v) about 2 mg of sodium lauryl sulfate;
and
(b) an extra-granular component comprising:
(b-i) about 11 mg of microcrystalline cellulose;
(b-ii) about 2 mg of croscarmellose sodium; and
(b-iii) about 1 mg of magnesium stearate;
(D)
(a) an intra-granular component comprising:
(a-i) about 40 mg of the total core weight of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(a-ii-1) about 88 mg of the total core weight of mannitol;
(a-ii-2) about 30 mg of the total core weight of microcrystalline cellulose;
(a-iii) about 4 mg of the total core weight of hydroxypropyl cellulose;
(a-iv) about 6 mg of the total core weight of croscarmellose sodium; and
(a-v) about 4 mg of the total core weight of sodium lauryl sulfate; and
(b) an extra-granular component comprising:
(b-i) about 22 mg of the total core weight of microcrystalline cellulose;
(b-ii) about 4 mg of the total core weight of croscarmellose sodium; and
(b-iii) about 2 mg of the total core weight of magnesium stearate;
(E)
(a) an intra-granular component comprising:
(a-i) about 10 mg of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(a-ii-1) about 108 mg of mannitol;
(a-ii-2) about 40 mg of microcrystalline cellulose;
(a-iii) about 4 mg of hydroxypropyl cellulose;
(a-iv) about 6 mg of croscarmellose sodium; and
(a-v) about 4 mg of sodium lauryl sulfate;
and
(b) an extra-granular component comprising:
(b-i) about 22 mg of microcrystalline cellulose;
(b-ii) about 4 mg of croscarmellose sodium; and
(b-iii) about 2 mg of magnesium stearate;
(F)
(a) an intra-granular component comprising:
(a-i) about 7 mg of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(a-ii-1) about 35 mg of mannitol;
(a-ii-2) about 13.3 mg of microcrystalline cellulose;
(a-iii) about 1.4 mg of hydroxypropyl cellulose;
(a-iv) about 2.1 mg of croscarmellose sodium; and
(a-v) about 1.4 mg of sodium lauryl sulfate;
and
(b) an extra-granular component comprising:
(b-i) about 7.7 mg of microcrystalline cellulose;
(b-ii) about 1.4 mg of croscarmellose sodium; and
(b-iii) about 0.7 mg of magnesium stearate; or
(G)
(a) an intra-granular component comprising:
(a-i) about 40 mg of the (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof;
(a-ii-1) about 200 mg of mannitol;
(a-ii-2) about 76 mg of microcrystalline cellulose;
(a-iii) about 8 mg of hydroxypropyl cellulose;
(a-iv) about 12 mg of croscarmellose sodium; and
(a-v) about 8 mg of sodium lauryl sulfate;
and
(b) an extra-granular component comprising:
(b-i) about 44 mg of microcrystalline cellulose;
(b-ii) about 8 mg of croscarmellose sodium; and
(b-iii) about 4 mg of magnesium stearate.

12. A process for making the tablet of any one of claims 6-11, wherein the process comprises:
(1) preparing granules comprising (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof, the intra-granular filler, the intra-granular binder, the intra-granular disintegrant, and the intra-granular surfactant;
(2) milling the granules comprising (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof, the intra-granular filler, the intra-granular binder, the intra-granular disintegrant, and the intra-granular surfactant to form the intra-granular component;
(3) blending the intra-granular component with the extra-granular filler, the extra-granular disintegrant, and the extra-granular lubricant to form a final blend mixture;
(4) compressing the final blend mixture to form the core;
(5) optionally coating the core with the coating layer;
for example wherein preparing granules comprising (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof, the intra-granular filler, the intra-granular binder, the intra-granular disintegrant, and the intra-granular surfactant comprises a wet granulation step; for example wherein the wet granulation step further comprises a wet milling step.

13. The process of claim 12, wherein preparing granules comprising (R)-N-(5-(5-ethyl-1,2,4-oxadiazol-3-yl)-2,3-dihydro-1H-inden-1-yl)-1-methyl-1H-pyrazole-4-carboxamide, or a hydrate, solvate, polymorph, or pharmaceutically acceptable salt thereof, the intra-granular filler, the intra-granular binder, the intra-granular disintegrant, and the intra-granular surfactant further comprises drying the granules.

14. The process of claim 12 or 13, comprising coating the core with the coating layer.

15. The formulation of any one of claims 1-5, or the tablet of any one of claims 6-11, for use in treating heart disease in a subject in need thereof.

16. The formulation or the tablet according to claim 15, for use in treating heart disease in a subject in need thereof, wherein the heart disease is hypertrophic cardiomyopathy (HCM).

17. The formulation or the tablet according to claim 16, for use in treating hypertrophic cardiomyopathy (HCM) in a subject in need thereof, wherein the HCM is obstructive or nonobstructive or is associated with a sarcomeric and/or non-sarcomeric mutation.

18. The formulation or the tablet according to claim 15, for use in treating heart disease in a subject in need thereof, wherein the heart disease is heart failure with preserved ejection fraction (HFpEF).

## Patentansprüche

1. Formulierung, umfassend:
(i) (R)-N-(5-(5-Ethyl-1,2,4-oxadiazol-3-yl)-2,3-dihydro-1H-inden-1-yl)-1-methyl-1H-pyrazol-4-carboxamid oder ein Hydrat, Solvat, Polymorph oder pharmazeutisch unbedenkliches Salz davon;
(ii) einen Füllstoff;
(iii) ein Bindemittel;
(iv) ein Sprengmittel;
(v) ein Tensid und
(vi) ein Schmiermittel.

2. Formulierung nach Anspruch 1, wobei es sich bei (R)-N-(5-(5-Ethyl-1,2,4-oxadiazol-3-yl)-2,3-dihydro-1H-inden-1-yl)-1-methyl-1H-pyrazol-4-carboxamid oder einem Hydrat, Solvat, Polymorph oder pharmazeutisch unbedenklichen Salz davon um (R)-N-(5-(5-Ethyl-1,2,4-oxadiazol-3-yl)-2,3-dihydro-1H-inden-1-yl)-1-methyl-1H-pyrazol-4-carboxamid handelt.

3. Formulierung nach Anspruch 1 oder 2, wobei:
(a) der Füllstoff aus der Gruppe bestehend aus pulverförmiger Cellulose, mikrokristalliner Cellulose, silifizierter mikrokristalliner Cellulose, Kaolin, Maisstärke, Stärkederivaten, vorverkleisterter Stärke, Calciumphosphat, Calciumhydrogenphosphat, Dicalciumphosphat, Tricalciumphosphat, komprimierbarem Zucker, Zuckeralkohol, Mannitol, Sorbitol, Maltitol, Xylitol, Lactitol, Lactose, Dextrose, Maltose, Sucrose, Glucose, Fructose, Saccharose, Raffinose, Dextraten, Trehalose, Maltodextrinen und Mischungen beliebiger der vorstehend genannten Substanzen ausgewählt ist und/oder
(b) das Bindemittel aus der Gruppe bestehend aus Gummi arabicum, Akaziengummi, Alginat, Alginsäure, Maisstärke, Copolyvidon, Polyvinylpyrrolidon, Gelatine, Glycerylbehenat, Hydroxyethylcellulose, Hydroxypropylcellulose, Carboxymethylcellulose, Carboxymethylcellulose-Calcium, Carboxymethylcellulose-Natrium, Methylcellulose, Hypromellose, Lactose, Polyvinylalkohol, Povidon, Polyethylenoxid, Polyacrylaten, Kartoffelstärke, vorverkleisterter Stärke, Natriumalginat, Natriumstärke, Natriumcarboxymethylcellulose, Stärke und Mischungen beliebiger der vorstehend genannten Substanzen ausgewählt ist und/oder
(c) das Sprengmittel aus der Gruppe bestehend aus Alginsäure, Croscarmellose-Natrium, Cellulose, Carboxymethylcellulose-Calcium, Carboxymethylcellulose-Natrium, mikrokristalliner Cellulose, Crospovidon, Natriumstärkeglykolat, niedrigsubstituierter Hydroxypropylcellulose, Polacrilin-Kalium, vorverkleisterter Stärke, teilhydrolysierter Stärke, Natriumcarboxymethylstärke, Stärke, Natriumalginat, Natriumcarboxymethylcellulose und Mischungen beliebiger der vorstehend genannten Substanzen ausgewählt ist und/oder
(d) das Tensid aus der Gruppe bestehend aus Cetylpyridinchlorid, Heptadecaethylenoxycetanol, Lecithinen, Polyoxyethylenstearat, Nonoxynol 9, Nonoxynol 10, Octoxynol 9, Sorbitanfettsäureestern, Span 20, Span 40, Span 60, Span 80, Span 85, Polysorbaten, Polysorbat 20, Polysorbat 21, Polysorbat 40, Polysorbat 60, Polysorbat 61, Polysorbat 65, Polysorbat 80, Natriumsalzen von Fettalkoholsulfaten, Natriumlaurylsulfat, Natriumsalzen von Sulfosuccinaten, Natriumdioctylsulfosuccinat, Partialestern von Fettsäuren mit Alkoholen, Glycerinmonostearat, Glycerylmonooleat, Ethern von Fettalkoholen mit Polyoxyethylen, Estern von Fettsäuren mit Polyoxyethylen, Copolymeren von Ethylenoxid und Propylenoxid (Pluronic^{®}), Benzalkoniumchlorid, ethoxylierten Triglyceriden und Mischungen beliebiger der vorstehend genannten Substanzen ausgewählt ist und/oder
(e) das Schmiermittel aus der Gruppe bestehend aus hydriertem Ricinusöl, Magnesiumstearat, Glycerylmonostearat, Calciumstearat, Glycerylbehenat, Glycerindistearat, Glyceryldipalmitostearat, Behenoylpolyoxyl-8-glyceriden, Natriumstearylfumarat, Stearinsäure, Talkum, Zinkstearat, Mineralöl, Polyethylenglykol, Poloxamer, Natriumlaurylsulfat und Mischungen beliebiger der vorstehend genannten Substanzen ausgewählt ist und/oder
(f) die Formulierung Folgendes umfasst:
(i) etwa 1 Gew.-% bis etwa 80 Gew.-% des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(ii) etwa 15 Gew.-% bis etwa 90 Gew.-% des Füllstoffs;
(iii) etwa 0,1 Gew.-% bis etwa 10 Gew.-% des Bindemittels;
(iv) etwa 1 Gew.-% bis etwa 10 Gew.-% des Sprengmittels;
(v) etwa 0,1 Gew.-% bis etwa 10 Gew.-% des Tensids und
(vi) etwa 0,1 Gew.-% bis etwa 10 Gew.-% des Schmiermittels.

4. Formulierung nach einem der Ansprüche 1-3, wobei die Formulierung Folgendes umfasst:
(a)
(i) etwa 20 Gew.-% des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(ii) etwa 70 Gew.-% des Füllstoffs;
(iii) etwa 2 Gew.-% des Bindemittels;
(iv) etwa 5 Gew.-% des Sprengmittels;
(v) etwa 2 Gew.-% des Tensids und
(vi) etwa 1 Gew.-% des Schmiermittels;
(b)
(i) etwa 10 Gew.-% des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(ii) etwa 80 Gew.-% des Füllstoffs;
(iii) etwa 2 Gew.-% des Bindemittels;
(iv) etwa 5 Gew.-% des Sprengmittels;
(v) etwa 2 Gew.-% des Tensids und
(vi) etwa 1 Gew.-% des Schmiermittels;
(c)
(i) etwa 5 Gew.-% des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(ii) etwa 85 Gew.-% des Füllstoffs;
(iii) etwa 2 Gew.-% des Bindemittels;
(iv) etwa 5 Gew.-% des Sprengmittels;
(v) etwa 2 Gew.-% des Tensids und
(vi) etwa 1 Gew.-% des Schmiermittels;
(d)
(i) etwa 20 Gew.-% des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(ii-1) etwa 44 Gew.-% Mannitol;
(ii-2) etwa 26 Gew.-% mikrokristalline Cellulose
(iii) etwa 2 Gew.-% Hydroxypropylcellulose;
(iv) etwa 5 Gew.-% Croscarmellose-Natrium;
(v) etwa 2 Gew.-% Natriumlaurylsulfat und
(vi) etwa 1 Gew.-% Magnesiumstearat;
(e)
(i) etwa 10 Gew.-% des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(ii-1) etwa 50 Gew.-% Mannitol;
(ii-2) etwa 30 Gew.-% mikrokristalline Cellulose
(iii) etwa 2 Gew.-% Hydroxypropylcellulose;
(iv) etwa 5 Gew.-% Croscarmellose-Natrium;
(v) etwa 2 Gew.-% Natriumlaurylsulfat und
(vi) etwa 1 Gew.-% Magnesiumstearat; oder
(f)
(i) etwa 5 Gew.-% des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(ii-1) etwa 54 Gew.-% Mannitol;
(ii-2) etwa 31 Gew.-% mikrokristalline Cellulose
(iii) etwa 2 Gew.-% Hydroxypropylcellulose;
(iv) etwa 5 Gew.-% Croscarmellose-Natrium;
(v) etwa 2 Gew.-% Natriumlaurylsulfat und
(vi) etwa 1 Gew.-% Magnesiumstearat.

5. Formulierung nach einem der vorhergehenden Ansprüche, wobei die Formulierung für die orale Verabreichung vorgesehen ist und/oder wobei die Formulierung für die einmal tägliche Verabreichung vorgesehen ist.

6. Tablette, umfassend:
(i) einen Kern mit einem Kerngesamtgewicht, umfassend:
(a) eine intragranuläre Komponente, umfassend:
(a-i) (R)-N-(5-(5-Ethyl-1,2,4-oxadiazol-3-yl)-2,3-dihydro-1H-inden-1-yl)-1-methyl-1H-pyrazol-4-carboxamid oder ein Hydrat, Solvat, Polymorph oder pharmazeutisch unbedenkliches Salz davon;
(a-ii) einen intragranulären Füllstoff;
(a-iii) ein intragranuläres Bindemittel;
(a-iv) ein intragranuläres Sprengmittel und
(a-v) ein intragranuläres Tensid;
und
(b) eine extragranuläre Komponente, umfassend:
(b-i) einen extragranulären Füllstoff;
(b-ii) ein extragranuläres Sprengmittel und
(b-iii) ein extragranuläres Schmiermittel;
und gegebenenfalls
(ii) eine Überzugsschicht, umfassend ein Überzugsmittel.

7. Tablette nach Anspruch 6, wobei:
(a) der intragranuläre Füllstoff aus der Gruppe bestehend aus pulverförmiger Cellulose, mikrokristalliner Cellulose, silifizierter mikrokristalliner Cellulose, Kaolin, Maisstärke, Stärkederivaten, vorverkleisterter Stärke, Calciumphosphat, Calciumhydrogenphosphat, Dicalciumphosphat, Tricalciumphosphat, komprimierbarem Zucker, Zuckeralkohol, Mannitol, Sorbitol, Maltitol, Xylitol, Lactitol, Lactose, Dextrose, Maltose, Sucrose, Glucose, Fructose, Saccharose, Raffinose, Dextraten, Trehalose, Maltodextrinen und Mischungen beliebiger der vorstehend genannten Substanzen ausgewählt ist und/oder
(b) das intragranuläre Bindemittel aus der Gruppe bestehend aus Gummi arabicum, Akaziengummi, Alginat, Alginsäure, Maisstärke, Copolyvidon, Polyvinylpyrrolidon, Gelatine, Glycerylbehenat, Hydroxyethylcellulose, Hydroxypropylcellulose, Carboxymethylcellulose, Carboxymethylcellulose-Calcium, Carboxymethylcellulose-Natrium, Methylcellulose, Hypromellose, Lactose, Polyvinylalkohol, Povidon, Polyethylenoxid, Polyacrylaten, Kartoffelstärke, vorverkleisterter Stärke, Natriumalginat, Natriumstärke, Natriumcarboxymethylcellulose, Stärke und Mischungen beliebiger der vorstehend genannten Substanzen ausgewählt ist und/oder
(c) das intragranuläre Sprengmittel aus der Gruppe bestehend aus Alginsäure, Croscarmellose-Natrium, Cellulose, Carboxymethylcellulose-Calcium, Carboxymethylcellulose-Natrium, mikrokristalliner Cellulose, Crospovidon, Natriumstärkeglykolat, niedrigsubstituierter Hydroxypropylcellulose, Polacrilin-Kalium, vorverkleisterter Stärke, teilhydrolysierter Stärke, Natriumcarboxymethylstärke, Stärke, Natriumalginat, Natriumcarboxymethylcellulose und Mischungen beliebiger der vorstehend genannten Substanzen ausgewählt ist und/oder
(d) das intragranuläre Tensid aus der Gruppe bestehend aus Cetylpyridinchlorid, Heptadecaethylenoxycetanol, Lecithinen, Polyoxyethylenstearat, Nonoxynol 9, Nonoxynol 10, Octoxynol 9, Sorbitanfettsäureestern, Span 20, Span 40, Span 60, Span 80, Span 85, Polysorbaten, Polysorbat 20, Polysorbat 21, Polysorbat 40, Polysorbat 60, Polysorbat 61, Polysorbat 65, Polysorbat 80, Natriumsalzen von Fettalkoholsulfaten, Natriumlaurylsulfat, Natriumsalzen von Sulfosuccinaten, Natriumdioctylsulfosuccinat, Partialestern von Fettsäuren mit Alkoholen, Glycerinmonostearat, Glycerylmonooleat, Ethern von Fettalkoholen mit Polyoxyethylen, Estern von Fettsäuren mit Polyoxyethylen, Copolymeren von Ethylenoxid und Propylenoxid (Pluronic^{®}), Benzalkoniumchlorid, ethoxylierten Triglyceriden und Mischungen beliebiger der vorstehend genannten Substanzen ausgewählt ist; und/oder
(e) der extragranuläre Füllstoff aus der Gruppe bestehend aus pulverförmiger Cellulose, mikrokristalliner Cellulose, silifizierter mikrokristalliner Cellulose, Kaolin, Maisstärke, Stärkederivaten, vorverkleisterter Stärke, Calciumphosphat, Calciumhydrogenphosphat, Dicalciumphosphat, Tricalciumphosphat, komprimierbarem Zucker, Zuckeralkohol, Mannitol, Sorbitol, Maltitol, Xylitol, Lactitol, Lactose, Dextrose, Maltose, Sucrose, Glucose, Fructose, Saccharose, Raffinose, Dextraten, Trehalose, Maltodextrinen und Mischungen beliebiger der vorstehend genannten Substanzen ausgewählt ist und/oder
(f) das intragranuläre Sprengmittel aus der Gruppe bestehend aus Alginsäure, Croscarmellose-Natrium, Cellulose, Carboxymethylcellulose-Calcium, Carboxymethylcellulose-Natrium, mikrokristalliner Cellulose, Crospovidon, Natriumstärkeglykolat, niedrigsubstituierter Hydroxypropylcellulose, Polacrilin-Kalium, vorverkleisterter Stärke, teilhydrolysierter Stärke, Natriumcarboxymethylstärke, Stärke, Natriumalginat, Natriumcarboxymethylcellulose und Mischungen beliebiger der vorstehend genannten Substanzen ausgewählt ist und/oder (g) das extragranuläre Schmiermittel aus der Gruppe bestehend aus hydriertem Ricinusöl, Magnesiumstearat, Glycerylmonostearat, Calciumstearat, Glycerylbehenat, Glycerindistearat, Glyceryldipalmitostearat, Behenoylpolyoxyl-8-glyceriden, Natriumstearylfumarat, Stearinsäure, Talkum, Zinkstearat, Mineralöl, Polyethylenglykol, Poloxamer, Natriumlaurylsulfat und Mischungen beliebiger der vorstehend genannten Substanzen ausgewählt ist.

8. Tablette nach einem der Ansprüche 6-7, wobei der Kern Folgendes umfasst:
(a) eine intragranuläre Komponente, umfassend:
(a-i) etwa 1 % bis etwa 80 %, bezogen auf das Kerngesamtgewicht, des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(a-ii) etwa 10 % bis etwa 80 %, bezogen auf das Kerngesamtgewicht, des intragranulären Füllstoffs;
(a-iii) etwa 0,1 % bis etwa 10 %, bezogen auf das Kerngesamtgewicht, des intragranulären Bindemittels;
(a-iv) etwa 0,1 % bis etwa 5 %, bezogen auf das Kerngesamtgewicht, des intragranulären Sprengmittels und
(a-v) etwa 0,1 % bis etwa 5 %, bezogen auf das Kerngesamtgewicht, des intragranulären Tensids;
und
(b) eine extragranuläre Komponente, umfassend:
(b-i) etwa 5 % bis etwa 15 %, bezogen auf das Kerngesamtgewicht, des extragranulären Füllstoffs;
(b-ii) etwa 0,1 % bis etwa 5 %, bezogen auf das Kerngesamtgewicht, des extragranulären Sprengmittels und
(b-iii) etwa 0,1 % bis etwa 5 %, bezogen auf das Kerngesamtgewicht, des extragranulären Schmiermittels.

9. Tablette nach einem der Ansprüche 6-8, wobei der Kern Folgendes umfasst:
(A)
(a) eine intragranuläre Komponente, umfassend:
(a-i) etwa 20 %, bezogen auf das Kerngesamtgewicht, des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(a-ii-1) etwa 44 %, bezogen auf das Kerngesamtgewicht, Mannitol;
(a-ii-2) etwa 15 %, bezogen auf das Kerngesamtgewicht, mikrokristalline Cellulose
(a-iii) etwa 2 %, bezogen auf das Kerngesamtgewicht, Hydroxypropylcellulose;
(a-iv) etwa 3 %, bezogen auf das Kerngesamtgewicht, Croscarmellose-Natrium und
(a-v) etwa 2 %, bezogen auf das Kerngesamtgewicht, Natriumlaurylsulfat;
und
(b) eine extragranuläre Komponente, umfassend:
(b-i) etwa 11 %, bezogen auf das Kerngesamtgewicht, mikrokristalline Cellulose;
(b-ii) etwa 2 %, bezogen auf das Kerngesamtgewicht, Croscarmellose-Natrium und
(b-iii) etwa 1 %, bezogen auf das Kerngesamtgewicht, Magnesiumstearat;
(B)
(a) eine intragranuläre Komponente, umfassend:
(a-i) etwa 10 %, bezogen auf das Kerngesamtgewicht, des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(a-ii-1) etwa 50 %, bezogen auf das Kerngesamtgewicht, Mannitol;
(a-ii-2) etwa 19 %, bezogen auf das Kerngesamtgewicht, mikrokristalline Cellulose
(a-iii) etwa 2 %, bezogen auf das Kerngesamtgewicht, Hydroxypropylcellulose;
(a-iv) etwa 3 %, bezogen auf das Kerngesamtgewicht, Croscarmellose-Natrium und
(a-v) etwa 2 %, bezogen auf das Kerngesamtgewicht, Natriumlaurylsulfat;
und
(b) eine extragranuläre Komponente, umfassend:
(b-i) etwa 11 %, bezogen auf das Kerngesamtgewicht, mikrokristalline Cellulose;
(b-ii) etwa 2 %, bezogen auf das Kerngesamtgewicht, Croscarmellose-Natrium und
(b-iii) etwa 1 %, bezogen auf das Kerngesamtgewicht, Magnesiumstearat; oder
(C)
(a) eine intragranuläre Komponente, umfassend:
(a-i) etwa 5 Gew.-% des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(a-ii-1) etwa 54 Gew.-% Mannitol;
(a-ii-2) etwa 20 Gew.-% mikrokristalline Cellulose
(a-iii) etwa 2 Gew.-% Hydroxypropylcellulose;
(a-iv) etwa 3 Gew.-% Croscarmellose-Natrium und
(a-v) etwa 2 Gew.-% Natriumlaurylsulfat;
und
(b) eine extragranuläre Komponente, umfassend:
(b-i) etwa 11 Gew.-% mikrokristalline Cellulose;
(b-ii) etwa 2 Gew.-% Croscarmellose-Natrium und
(b-iii) etwa 1 Gew.-% Magnesiumstearat.

10. Tablette nach einem der Ansprüche 6-9, umfassend eine Überzugsschicht, umfassend ein Überzugsmittel; beispielsweise wobei das Überzugsmittel aus der Gruppe bestehend aus Opadry QX White 21A180025, Opadry I und Opadry II ausgewählt ist; beispielsweise wobei es sich bei dem Überzugsmittel um Opadry QX White 21A180025 handelt.

11. Tablette nach Anspruch 6-8, wobei der Kern Folgendes umfasst:
(A)
(a) eine intragranuläre Komponente, umfassend:
(a-i) etwa 2,5 mg des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(a-ii-1) etwa 27 mg Mannitol;
(a-ii-2) etwa 10 mg mikrokristalline Cellulose
(a-iii) etwa 1 mg Hydroxypropylcellulose;
(a-iv) etwa 1,5 mg Croscarmellose-Natrium und
(a-v) etwa 1 mg Natriumlaurylsulfat;
und
(b) eine extragranuläre Komponente, umfassend:
(b-i) etwa 5,5 mg mikrokristalline Cellulose;
(b-ii) etwa 1 mg Croscarmellose-Natrium und
(b-iii) etwa 0,5 mg Magnesiumstearat;
(B)
(a) eine intragranuläre Komponente, umfassend:
(a-i) etwa 20 mg des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(a-ii-1) etwa 44 mg, bezogen auf das Kerngesamtgewicht, Mannitol;
(a-ii-2) etwa 15 mg, bezogen auf das Kerngesamtgewicht, mikrokristalline Cellulose
(a-iii) etwa 2 mg, bezogen auf das Kerngesamtgewicht, Hydroxypropylcellulose;
(a-iv) etwa 3 mg, bezogen auf das Kerngesamtgewicht, Croscarmellose-Natrium und
(a-v) etwa 2 mg, bezogen auf das Kerngesamtgewicht, Natriumlaurylsulfat;
und
(b) eine extragranuläre Komponente, umfassend:
(b-i) etwa 11 mg, bezogen auf das Kerngesamtgewicht, mikrokristalline Cellulose;
(b-ii) etwa 2 mg, bezogen auf das Kerngesamtgewicht, Croscarmellose-Natrium und
(b-iii) etwa 1 mg, bezogen auf das Kerngesamtgewicht, Magnesiumstearat;
(C)
(a) eine intragranuläre Komponente, umfassend:
(a-i) etwa 5 mg des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(a-ii-1) etwa 54 mg Mannitol;
(a-ii-2) etwa 20 mg mikrokristalline Cellulose
(a-iii) etwa 2 mg Hydroxypropylcellulose;
(a-iv) etwa 3 mg Croscarmellose-Natrium und
(a-v) etwa 2 mg Natriumlaurylsulfat;
und
(b) eine extragranuläre Komponente, umfassend:
(b-i) etwa 11 mg mikrokristalline Cellulose;
(b-ii) etwa 2 mg Croscarmellose-Natrium und
(b-iii) etwa 1 mg Magnesiumstearat;
(D)
(a) eine intragranuläre Komponente, umfassend:
(a-i) etwa 40 mg, bezogen auf das Kerngesamtgewicht, des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(a-ii-1) etwa 88 mg, bezogen auf das Kerngesamtgewicht, Mannitol;
(a-ii-2) etwa 30 mg, bezogen auf das Kerngesamtgewicht, mikrokristalline Cellulose
(a-iii) etwa 4 mg, bezogen auf das Kerngesamtgewicht, Hydroxypropylcellulose;
(a-iv) etwa 6 mg, bezogen auf das Kerngesamtgewicht, Croscarmellose-Natrium und
(a-v) etwa 4 mg, bezogen auf das Kerngesamtgewicht, Natriumlaurylsulfat;
und
(b) eine extragranuläre Komponente, umfassend:
(b-i) etwa 22 mg, bezogen auf das Kerngesamtgewicht, mikrokristalline Cellulose;
(b-ii) etwa 4 mg, bezogen auf das Kerngesamtgewicht, Croscarmellose-Natrium und
(b-iii) etwa 2 mg, bezogen auf das Kerngesamtgewicht, Magnesiumstearat;
(E)
(a) eine intragranuläre Komponente, umfassend:
(a-i) etwa 10 mg des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(a-ii-1) etwa 108 mg Mannitol;
(a-ii-2) etwa 40 mg mikrokristalline Cellulose
(a-iii) etwa 4 mg Hydroxypropylcellulose;
(a-iv) etwa 6 mg Croscarmellose-Natrium und
(a-v) etwa 4 mg Natriumlaurylsulfat;
und
(b) eine extragranuläre Komponente, umfassend:
(b-i) etwa 22 mg mikrokristalline Cellulose;
(b-ii) etwa 4 mg Croscarmellose-Natrium und
(b-iii) etwa 2 mg Magnesiumstearat;
(F)
(a) eine intragranuläre Komponente, umfassend:
(a-i) etwa 7 mg des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(a-ii-1) etwa 35 mg Mannitol;
(a-ii-2) etwa 13,3 mg mikrokristalline Cellulose
(a-iii) etwa 1,4 mg Hydroxypropylcellulose;
(a-iv) etwa 2,1 mg Croscarmellose-Natrium und
(a-v) etwa 1,4 mg Natriumlaurylsulfat;
und
(b) eine extragranuläre Komponente, umfassend:
(b-i) etwa 7,7 mg mikrokristalline Cellulose;
(b-ii) etwa 1,4 mg Croscarmellose-Natrium, und
(b-iii) etwa 0,7 mg Magnesiumstearat; oder
(G)
(a) eine intragranuläre Komponente, umfassend:
(a-i) etwa 40 mg des (R)-N-(5-(5-Ethyl-1,2,4-oxadiazol-3-yl)-2,3-dihydro-1H-inden-1-yl)-1-methyl-1H-pyrazol-4-carboxamids oder eines Hydrats, Solvats, Polymorphs oder pharmazeutisch unbedenklichen Salzes davon;
(a-ii-1) etwa 200 mg Mannitol;
(a-ii-2) etwa 76 mg mikrokristalline Cellulose
(a-iii) etwa 8 mg Hydroxypropylcellulose;
(a-iv) etwa 12 mg Croscarmellose-Natrium und
(a-v) etwa 18 mg Natriumlaurylsulfat;
und
(b) eine extragranuläre Komponente, umfassend:
(b-i) etwa 44 mg mikrokristalline Cellulose;
(b-ii) etwa 8 mg Croscarmellose-Natrium, und
(b-iii) etwa 4 mg Magnesiumstearat.

12. Verfahren zur Herstellung der Tablette nach einem der Ansprüche 6-11, wobei das Verfahren Folgendes umfasst:
(1) Herstellen von Granulat, umfassend (R)-N-(5-(5-Ethyl-1,2,4-oxadiazol-3-yl)-2,3-dihydro-1H-inden-1-yl)-1-methyl-1H-pyrazol-4-carboxamid oder ein Hydrat, Solvat, Polymorph oder pharmazeutisch unbedenkliches Salz davon, den intragranulären Füllstoff, das intragranuläre Bindemittel, das intragranuläre Sprengmittel und das intragranuläre Tensid;
(2) Mahlen des Granulats, umfassend (R)-N-(5-(5-Ethyl-1,2,4-oxadiazol-3-yl)-2,3-dihydro-1H-inden-1-yl)-1-methyl-1H-pyrazol-4-carboxamid oder ein Hydrat, Solvat, Polymorph oder pharmazeutisch unbedenkliches Salz davon, den intragranulären Füllstoff, das intragranuläre Bindemittel, das intragranuläre Sprengmittel und das intragranuläre Tensid, zur Bildung der intragranulären Komponente;
(3) Mischen der intragranulären Komponente mit dem extragranulären Füllstoff, dem extragranulären Sprengmittel und dem extragranulären Schmiermittel zur Bildung einer fertigen Mischung;
(4) Verpressen der fertigen Mischung zur Bildung des Kerns;
(5) gegebenenfalls Überziehen des Kerns mit der Überzugsschicht;
beispielsweise wobei das Herstellen von Granulat, umfassend (R)-N-(5-(5-Ethyl-1,2,4-oxadiazol-3-yl)-2,3-dihydro-1H-inden-1-yl)-1-methyl-1H-pyrazol-4-carboxamid oder ein Hydrat, Solvat, Polymorph oder pharmazeutisch unbedenkliches Salz davon, den intragranulären Füllstoff, das intragranuläre Bindemittel, das intragranuläre Sprengmittel und das intragranuläre Tensid, einen Nassgranulationsschritt umfasst; beispielsweise wobei der Nassgranulationsschritt einen Nassmahlschritt umfasst.

13. Verfahren nach Anspruch 12, wobei das Herstellen von Granulat, umfassend (R)-N-(5-(5-Ethyl-1,2,4-oxadiazol-3-yl)-2,3-dihydro-1H-inden-1-yl)-1-methyl-1H-pyrazol-4-carboxamid oder ein Hydrat, Solvat, Polymorph oder pharmazeutisch unbedenkliches Salz davon, den intragranulären Füllstoff, das intragranuläre Bindemittel, das intragranuläre Sprengmittel und das intragranuläre Tensid, ferner das Trocknen des Granulats umfasst.

14. Verfahren nach Anspruch 12 oder 13, umfassend das Überziehen des Kerns mit der Überzugsschicht.

15. Formulierung nach einem der Ansprüche 1 bis 5 oder Tablette nach einem der Ansprüche 6-11 zur Verwendung bei der Behandlung einer Herzkrankheit bei einem Individuum, bei dem diesbezüglicher Bedarf besteht.

16. Formulierung oder Tablette nach Anspruch 15 zur Verwendung bei der Behandlung einer Herzkrankheit bei einem Individuum, bei dem diesbezüglicher Bedarf besteht, wobei es sich bei der Herzkrankheit um hypertrophe Kardiomyopathie (HCM) handelt.

17. Formulierung oder Tablette nach Anspruch 16 zur Verwendung bei der Behandlung von hypertropher Kardiomyopathie (HCM) bei einem Individuum, bei dem diesbezüglicher Bedarf besteht, wobei die HCM obstruktiv oder nicht obstruktiv ist oder mit einer sarkomeren und/oder nicht sarkomeren Mutation assoziiert ist.

18. Formulierung oder Tablette nach Anspruch 15 zur Verwendung bei der Behandlung eine Herzkrankheit bei einem Individuum, bei dem diesbezüglicher Bedarf besteht, wobei es sich bei der Herzkrankheit um Herzinsuffizienz mit erhaltener Ejektionsfraktion (HFpEF) handelt.

## Revendications

1. Formulation comprenant :
(i) du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(ii) une charge ;
(iii) un liant ;
(iv) un délitant ;
(v) un tensioactif ; et
(vi) un lubrifiant.

2. Formulation selon la revendication 1, dans laquelle le (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci, est le (R)-N-(5-(5-éthyl-1,2,4-oxadiazol-3-yl)-2,3-dihydro-1H-indén-1-yl)-1-méthyl-1H-pyrazole-4-carboxamide.

3. Formulation selon la revendication 1 ou 2, dans laquelle :
(a) la charge est choisie dans le groupe constitué de : la cellulose pulvérulente, la cellulose microcristalline, la cellulose microcristalline silicifiée, le kaolin, l'amidon de maïs, l'amidon de maïs, des dérivés d'amidon, l'amidon prégélatinisé, le phosphate de calcium, l'hydrogénophosphate de calcium, le phosphate dicalcique, le phosphate tricalcique, un sucre compressible, un alcool de sucre, le mannitol, le sorbitol, le maltitol, le xylitol, le lactitol, le lactose, le dextrose, le maltose, le saccharose, le glucose, le fructose, le saccharose, le raffinose, des dextrates, le tréhalose, des maltodextrines, et des mélanges quelconques de ceux-ci ; et/ou
(b) le liant est choisi dans le groupe constitué de : la gomme arabique, la gomme d'acacia, l'alginate, l'acide alginique, l'amidon de maïs, la copolyvidone, la polyvinylpyrrolidone, la gélatine, le béhénate de glycéryle, l'hydroxyéthylcellulose, l'hydroxypropylcellulose, la carboxyméthylcellulose, la carboxyméthylcellulose calcique, la carboxyméthylcellulose sodique, la méthylcellulose, l'hypromellose, le lactose, l'alcool polyvinylique, la povidone, le poly(oxyde d'éthylène), des polyacrylates, l'amidon de pomme de terre, l'amidon prégélatinisé, l'alginate de sodium, l'amidon sodique, la carboxyméthylcellulose sodique, l'amidon, et des mélanges quelconques de ceux-ci ; et/ou
(c) le délitant est choisi dans le groupe constitué de : l'acide alginique, la croscarmellose sodique, la cellulose, la carboxyméthylcellulose calcique, la carboxyméthylcellulose sodique, la cellulose microcristalline, la crospovidone, le glycolate d'amidon sodique, l'hydroxypropylcellulose faiblement substituée, la polacriline potassique, l'amidon prégélatinisé, l'amidon partiellement hydrolysé, le carboxyméthylamidon sodique, l'amidon, l'alginate de sodium, la carboxyméthylcellulose sodique, et des mélanges quelconques de ceux-ci ; et/ou
(d) le tensioactif est choisi dans le groupe constitué de : le chlorure de cétylpyridine, l'heptadécaéthylène oxycétanol, des lécithines, le stéarate de polyoxyéthylène, nonoxynol 9, nonoxynol 10, octoxynol 9, des esters d'acide gras de sorbitane, Span 20, Span 40, Span 60, Span 80, Span 85, des polysorbates, le polysorbate 20, le polysorbate 21, le polysorbate 40, le polysorbate 60, le polysorbate 61, le polysorbate 65, le polysorbate 80, des sels de sodium de sulfates d'alcool gras, le laurylsulfate de sodium, des sels de sodium de sulfosuccinates, le dioctylsulfosuccinate de sodium, des esters partiels d'acides gras avec des alcools, le monostéarate de glycérine, le monooléate de glycéryle, des éthers d'alcools gras avec le polyoxyéthylène, des esters d'acides gras avec le polyoxyéthylène, des copolymères d'oxyde d'éthylène et d'oxyde de propylène (Pluronic^{®}), le chlorure de benzalkonium, des triglycérides éthoxylés, et des mélanges quelconques de ceux-ci ; et/ou
(e) le lubrifiant est choisi dans le groupe constitué de : l'huile de ricin hydrogénée, le stéarate de magnésium, le monostéarate de glycéryle, le stéarate de calcium, le béhénate de glycéryle, le distéarate de glycérol, le dipalmitostéarate de glycéryle, des béhénoyl-polyoxyl-8-glycérides, le stéarylfumarate de sodium, l'acide stéarique, le talc, le stéarate de zinc, une huile minérale, le polyéthylène glycol, un polaxamère, le laurylsulfate de sodium, et des mélanges quelconques de ceux-ci ; et/ou
(f) la formulation comprend :
(i) environ 1 % en poids à environ 80 % en poids du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(ii) environ 15 % en poids à environ 90 % en poids de la charge ;
(iii) environ 0,1 % en poids à environ 10 % en poids du liant ;
(iv) environ 1 % en poids à environ 10 % en poids du délitant ;
(v) environ 0,1 % en poids à environ 10 % en poids du tensioactif ; et
(vi) environ 0,1 % en poids à environ 10 % en poids du lubrifiant.

4. Formulation selon l'une quelconque des revendications 1 à 3, la formulation comprenant :
(a)
(i) environ 20 % en poids du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(ii) environ 70 % en poids de la charge ;
(iii) environ 2 % en poids du liant ;
(iv) environ 5 % en poids du délitant ;
(v) environ 2 % en poids du tensioactif ; et
(vi) environ 1 % en poids du lubrifiant ;
(b)
(i) environ 10 % en poids du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(ii) environ 80 % en poids de la charge ;
(iii) environ 2 % en poids du liant ;
(iv) environ 5 % en poids du délitant ;
(v) environ 2 % en poids du tensioactif ; et
(vi) environ 1 % en poids du lubrifiant ;
(c)
(i) environ 5 % en poids du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(ii) environ 85 % en poids de la charge ;
(iii) environ 2 % en poids du liant ;
(iv) environ 5 % en poids du délitant ;
(v) environ 2 % en poids du tensioactif ; et
(vi) environ 1 % en poids du lubrifiant ;
(d)
(i) environ 20 % en poids du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(ii-1) environ 44 % en poids de mannitol ;
(ii-2) environ 26 % en poids de cellulose microcristalline ;
(iii) environ 2 % en poids d'hydroxypropylcellulose ;
(iv) environ 5 % en poids de croscarmellose sodique ;
(v) environ 2 % en poids de laurylsulfate de sodium ; et
(vi) environ 1 % en poids de stéarate de magnésium ;
(e)
(i) environ 10 % en poids du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(ii-1) environ 50 % en poids de mannitol ;
(ii-2) environ 30 % en poids de cellulose microcristalline ;
(iii) environ 2 % en poids d'hydroxypropylcellulose ;
(iv) environ 5 % en poids de croscarmellose sodique ;
(v) environ 2 % en poids de laurylsulfate de sodium ; et
(vi) environ 1 % en poids de stéarate de magnésium ; ou
(f)
(i) environ 5 % en poids du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(ii-1) environ 54 % en poids de mannitol ;
(ii-2) environ 31 % en poids de cellulose microcristalline ;
(iii) environ 2 % en poids d'hydroxypropylcellulose ;
(iv) environ 5 % en poids de croscarmellose sodique ;
(v) environ 2 % en poids de laurylsulfate de sodium ; et
(vi) environ 1 % en poids de stéarate de magnésium.

5. Formulation selon l'une quelconque des revendications précédentes, la formulation étant pour administration orale ; et/ou la formulation étant pour administration une fois par jour.

6. Comprimé comprenant :
(i) un noyau ayant un poids total de noyau comprenant :
(a) un composant intragranulaire comprenant :
(a-i) du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(a-ii) une charge intragranulaire ;
(a-iii) un liant intragranulaire ;
(a-iv) un délitant intragranulaire ; et
(a-v) un tensioactif intragranulaire ;
et
(b) un composant extragranulaire comprenant :
(b-i) une charge extragranulaire ;
(b-ii) un délitant extragranulaire ; et
(b-iii) un lubrifiant extragranulaire ;
et facultativement
(ii) une couche d'enrobage comprenant un agent d'enrobage.

7. Comprimé selon la revendication 6, dans lequel :
(a) la charge intragranulaire est choisie dans le groupe constitué de : la cellulose pulvérulente, la cellulose microcristalline, la cellulose microcristalline silicifiée, le kaolin, l'amidon de maïs, l'amidon de maïs, des dérivés d'amidon, l'amidon prégélatinisé, le phosphate de calcium, l'hydrogénophosphate de calcium, le phosphate dicalcique, le phosphate tricalcique, un sucre compressible, un alcool de sucre, le mannitol, le sorbitol, le maltitol, le xylitol, le lactitol, le lactose, le dextrose, le maltose, le saccharose, le glucose, le fructose, le saccharose, le raffinose, des dextrates, le tréhalose, des maltodextrines, et des mélanges quelconques de ceux-ci ; et/ou
(b) le liant intragranulaire est choisi dans le groupe constitué de : la gomme arabique, la gomme d'acacia, l'alginate, l'acide alginique, l'amidon de maïs, la copolyvidone, la polyvinylpyrrolidone, la gélatine, le béhénate de glycéryle, l'hydroxyéthylcellulose, l'hydroxypropylcellulose, la carboxyméthylcellulose, la carboxyméthylcellulose calcique, la carboxyméthylcellulose sodique, la méthylcellulose, l'hypromellose, le lactose, l'alcool polyvinylique, la povidone, le poly(oxyde d'éthylène), des polyacrylates, l'amidon de pomme de terre, l'amidon prégélatinisé, l'alginate de sodium, l'amidon sodique, la carboxyméthylcellulose sodique, l'amidon, et des mélanges quelconques de ceux-ci ; et/ou
(c) le délitant intragranulaire est choisi dans le groupe constitué de : l'acide alginique, la croscarmellose sodique, la cellulose, la carboxyméthylcellulose calcique, la carboxyméthylcellulose sodique, la cellulose microcristalline, la crospovidone, le glycolate d'amidon sodique, l'hydroxypropylcellulose faiblement substituée, la polacriline potassique, l'amidon prégélatinisé, l'amidon partiellement hydrolysé, le carboxyméthylamidon sodique, l'amidon, l'alginate de sodium, la carboxyméthylcellulose sodique, et des mélanges quelconques de ceux-ci ; et/ou
(d) le tensioactif intragranulaire est choisi dans le groupe constitué de : le chlorure de cétylpyridine, l'heptadécaéthylène oxycétanol, des lécithines, le stéarate de polyoxyéthylène, nonoxynol 9, nonoxynol 10, octoxynol 9, des esters d'acide gras de sorbitane, Span 20, Span 40, Span 60, Span 80, Span 85, des polysorbates, le polysorbate 20, le polysorbate 21, le polysorbate 40, le polysorbate 60, le polysorbate 61, le polysorbate 65, le polysorbate 80, des sels de sodium de sulfates d'alcool gras, le laurylsulfate de sodium, des sels de sodium de sulfosuccinates, le dioctylsulfosuccinate de sodium, des esters partiels d'acides gras avec des alcools, le monostéarate de glycérine, le monooléate de glycéryle, des éthers d'alcools gras avec le polyoxyéthylène, des esters d'acides gras avec le polyoxyéthylène, des copolymères d'oxyde d'éthylène et d'oxyde de propylène (Pluronic^{®}), le chlorure de benzalkonium, des triglycérides éthoxylés, et des mélanges quelconques de ceux-ci ; et/ou
(e) la charge extragranulaire est choisie dans le groupe constitué de : la cellulose pulvérulente, la cellulose microcristalline, la cellulose microcristalline silicifiée, le kaolin, l'amidon de maïs, l'amidon de maïs, des dérivés d'amidon, l'amidon prégélatinisé, le phosphate de calcium, l'hydrogénophosphate de calcium, le phosphate dicalcique, le phosphate tricalcique, un sucre compressible, un alcool de sucre, le mannitol, le sorbitol, le maltitol, le xylitol, le lactitol, le lactose, le dextrose, le maltose, le saccharose, le glucose, le fructose, le saccharose, le raffinose, des dextrates, le tréhalose, des maltodextrines, et des mélanges quelconques de ceux-ci ; et/ou
(f) le délitant extragranulaire est choisi dans le groupe constitué de : l'acide alginique, la croscarmellose sodique, la cellulose, la carboxyméthylcellulose calcique, la carboxyméthylcellulose sodique, la cellulose microcristalline, la crospovidone, le glycolate d'amidon sodique, l'hydroxypropylcellulose faiblement substituée, la polacriline potassique, l'amidon prégélatinisé, l'amidon partiellement hydrolysé, le carboxyméthylamidon sodique, l'amidon, l'alginate de sodium, la carboxyméthylcellulose sodique, et des mélanges quelconques de ceux-ci ; et/ou
(g) le lubrifiant extragranulaire est choisi dans le groupe constitué de : l'huile de ricin hydrogénée, le stéarate de magnésium, le monostéarate de glycéryle, le stéarate de calcium, le béhénate de glycéryle, le distéarate de glycérol, le dipalmitostéarate de glycéryle, des béhénoyl-polyoxyl-8-glycérides, le stéarylfumarate de sodium, l'acide stéarique, le talc, le stéarate de zinc, une huile minérale, le polyéthylène glycol, un polaxamère, le laurylsulfate de sodium, et des mélanges quelconques de ceux-ci.

8. Comprimé selon l'une quelconque des revendications 6 à 7, dans lequel le noyau comprend :
(a) un composant intragranulaire comprenant :
(a-i) environ 1 % à environ 80 % du poids total de noyau du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(a-ii) environ 10 % à environ 80 % du poids total de noyau de la charge intragranulaire ;
(a-iii) environ 0,1 % à environ 10 % du poids total de noyau du liant intragranulaire ;
(a-iv) environ 0,1 % à environ 5 % du poids total de noyau du délitant intragranulaire ; et
(a-v) environ 0,1 % à environ 5 % du poids total de noyau du tensioactif intragranulaire ;
et
(b) un composant extragranulaire comprenant :
(b-i) environ 5 % à environ 15 % du poids total de noyau de la charge extragranulaire ;
(b-ii) environ 0,1 % à environ 5 % du poids total de noyau du délitant extragranulaire ; et
(b-iii) environ 0,1 % à environ 5 % du poids total de noyau du lubrifiant extragranulaire.

9. Comprimé selon l'une quelconque des revendications 6 à 8, dans lequel le noyau comprend :
(A)
(a) un composant intragranulaire comprenant :
(a-i) environ 20 % du poids total de noyau du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(a-ii-1) environ 44 % du poids total de noyau de mannitol ;
(a-ii-2) environ 15 % du poids total de noyau de cellulose microcristalline ;
(a-iii) environ 2 % du poids total de noyau d'hydroxypropylcellulose ;
(a-iv) environ 3 % du poids total de noyau de croscarmellose sodique ;
et
(a-v) environ 2 % du poids total de noyau de laurylsulfate de sodium ;
et
(b) un composant extragranulaire comprenant :
(b-i) environ 11 % du poids total de noyau de cellulose microcristalline ;
(b-ii) environ 2 % du poids total de noyau de croscarmellose sodique ; et
(b-iii) environ 1 % du poids total de noyau de stéarate de magnésium ;
(B)
(a) un composant intragranulaire comprenant :
(a-i) environ 10 % du poids total de noyau du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(a-ii-1) environ 50 % du poids total de noyau de mannitol ;
(a-ii-2) environ 19 % du poids total de noyau de cellulose microcristalline ;
(a-iii) environ 2 % du poids total de noyau d'hydroxypropylcellulose ;
(a-iv) environ 3 % du poids total de noyau de croscarmellose sodique ;
et
(a-v) environ 2 % du poids total de noyau de laurylsulfate de sodium ; et
(b) un composant extragranulaire comprenant :
(b-i) environ 11 % du poids total de noyau de cellulose microcristalline ;
(b-ii) environ 2 % du poids total de noyau de croscarmellose sodique ; et
(b-iii) environ 1 % du poids total de noyau de stéarate de magnésium ; ou
(C)
(a) un composant intragranulaire comprenant :
(a-i) environ 5 % en poids du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(a-ii-1) environ 54 % en poids de mannitol ;
(a-ii-2) environ 20 % en poids de cellulose microcristalline ;
(a-iii) environ 2 % en poids d'hydroxypropylcellulose ;
(a-iv) environ 3 % en poids de croscarmellose sodique ; et
(a-v) environ 2 % en poids de laurylsulfate de sodium ; et
(b) un composant extragranulaire comprenant :
(b-i) environ 11 % en poids de cellulose microcristalline ;
(b-ii) environ 2 % en poids de croscarmellose sodique ; et
(b-iii) environ 1 % en poids de stéarate de magnésium.

10. Comprimé selon l'une quelconque des revendications 6 à 9, comprenant une couche d'enrobage comprenant un agent d'enrobage ; par exemple dans lequel l'agent d'enrobage est choisi dans le groupe constitué d'Opadry QX White 21A180025, Opadry I, et Opadry II ; par exemple dans lequel l'agent d'enrobage est Opadry QX White 21A180025.

11. Comprimé selon la revendication 6 à 8, dans lequel le noyau comprend :
(A)
(a) un composant intragranulaire comprenant :
(a-i) environ 2,5 mg du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(a-ii-1) environ 27 mg de mannitol ;
(a-ii-2) environ 10 mg de cellulose microcristalline ;
(a-iii) environ 1 mg d'hydroxypropylcellulose ;
(a-iv) environ 1,5 mg de croscarmellose sodique ; et
(a-v) environ 1 mg de laurylsulfate de sodium ;
et
(b) un composant extragranulaire comprenant :
(b-i) environ 5,5 mg de cellulose microcristalline ;
(b-ii) environ 1 mg de croscarmellose sodique ; et
(b-iii) environ 0,5 mg de stéarate de magnésium ;
(B)
(a) un composant intragranulaire comprenant :
(a-i) environ 20 mg du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(a-ii-1) environ 44 mg du poids total de noyau de mannitol ;
(a-ii-2) environ 15 mg du poids total de noyau de cellulose microcristalline ;
(a-iii) environ 2 mg du poids total de noyau d'hydroxypropylcellulose ;
(a-iv) environ 3 mg du poids total de noyau de croscarmellose sodique ;
et
(a-v) environ 2 mg du poids total de noyau de laurylsulfate de sodium ;
et
(b) un composant extragranulaire comprenant :
(b-i) environ 11 mg du poids total de noyau de cellulose microcristalline ;
(b-ii) environ 2 mg du poids total de noyau de croscarmellose sodique ; et
(b-iii) environ 1 mg du poids total de noyau de stéarate de magnésium ;
(C)
(a) un composant intragranulaire comprenant :
(a-i) environ 5 mg du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(a-ii-1) environ 54 mg de mannitol ;
(a-ii-2) environ 20 mg de cellulose microcristalline ;
(a-iii) environ 2 mg d'hydroxypropylcellulose ;
(a-iv) environ 3 mg de croscarmellose sodique ; et
(a-v) environ 2 mg de laurylsulfate de sodium ;
et
(b) un composant extragranulaire comprenant :
(b-i) environ 11 mg de cellulose microcristalline ;
(b-ii) environ 2 mg de croscarmellose sodique ; et
(b-iii) environ 1 mg de stéarate de magnésium ;
(D)
(a) un composant intragranulaire comprenant :
(a-i) environ 40 mg du poids total de noyau du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(a-ii-1) environ 88 mg du poids total de noyau de mannitol ;
(a-ii-2) environ 30 mg du poids total de noyau de cellulose microcristalline ;
(a-iii) environ 4 mg du poids total de noyau d'hydroxypropylcellulose ;
(a-iv) environ 6 mg du poids total de noyau de croscarmellose sodique ;
et
(a-v) environ 4 mg du poids total de noyau de laurylsulfate de sodium ; et
(b) un composant extragranulaire comprenant :
(b-i) environ 22 mg du poids total de noyau de cellulose microcristalline ;
(b-ii) environ 4 mg du poids total de noyau de croscarmellose sodique ; et
(b-iii) environ 2 mg du poids total de noyau de stéarate de magnésium ;
(E)
(a) un composant intragranulaire comprenant :
(a-i) environ 10 mg du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(a-ii-1) environ 108 mg de mannitol ;
(a-ii-2) environ 40 mg de cellulose microcristalline ;
(a-iii) environ 4 mg d'hydroxypropylcellulose ;
(a-iv) environ 6 mg de croscarmellose sodique ; et
(a-v) environ 4 mg de laurylsulfate de sodium ;
et
(b) un composant extragranulaire comprenant :
(b-i) environ 22 mg de cellulose microcristalline ;
(b-ii) environ 4 mg de croscarmellose sodique ; et
(b-iii) environ 2 mg de stéarate de magnésium ;
(F)
(a) un composant intragranulaire comprenant :
(a-i) environ 7 mg du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(a-ii-1) environ 35 mg de mannitol ;
(a-ii-2) environ 13,3 mg de cellulose microcristalline ;
(a-iii) environ 1,4 mg d'hydroxypropylcellulose ;
(a-iv) environ 2,1 mg de croscarmellose sodique ; et
(a-v) environ 1,4 mg de laurylsulfate de sodium ;
et
(b) un composant extragranulaire comprenant :
(b-i) environ 7,7 mg de cellulose microcristalline ;
(b-ii) environ 1,4 mg de croscarmellose sodique ; et
(b-iii) environ 0,7 mg de stéarate de magnésium ; ou
(G)
(a) un composant intragranulaire comprenant :
(a-i) environ 40 mg du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci ;
(a-ii-1) environ 200 mg de mannitol ;
(a-ii-2) environ 76 mg de cellulose microcristalline ;
(a-iii) environ 8 mg d'hydroxypropylcellulose ;
(a-iv) environ 12 mg de croscarmellose sodique ; et
(a-v) environ 8 mg de laurylsulfate de sodium ;
et
(b) un composant extragranulaire comprenant :
(b-i) environ 44 mg de cellulose microcristalline ;
(b-ii) environ 8 mg de croscarmellose sodique ; et
(b-iii) environ 4 mg de stéarate de magnésium.

12. Procédé de fabrication du comprimé selon l'une quelconque des revendications 6 à 11, le procédé comprenant :
(1) la préparation de granules comprenant du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci, la charge intragranulaire, le liant intragranulaire, le délitant intragranulaire, et le tensioactif intragranulaire ;
(2) le broyage des granules comprenant du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci, la charge intragranulaire, le liant intragranulaire, le délitant intragranulaire, et le tensioactif intragranulaire pour former le composant intragranulaire ;
(3) le mélange du composant intragranulaire avec la charge extragranulaire, le délitant extragranulaire, et le lubrifiant extragranulaire pour former un mélange de formulation final ;
(4) la compression du mélange de formulation final pour former le noyau ;
(5) facultativement l'enrobage du noyau avec la couche d'enrobage ;
par exemple dans lequel la préparation de granules comprenant du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci, la charge intragranulaire, le liant intragranulaire, le délitant intragranulaire, et le tensioactif intragranulaire comprend une étape de granulation humide ; par exemple dans lequel l'étape de granulation humide comprend en outre une étape de broyage humide.

13. Procédé selon la revendication 12, dans lequel la préparation de granules comprenant du (R)-N-(5-(5-éthyl-1,2,4-oxadiazol-3-yl)-2,3-dihydro-1H-indén-1-yl)-1-méthyl-1H-pyrazole-4-carboxamide, ou un hydrate, solvate, polymorphe ou sel pharmaceutiquement acceptable de celui-ci, la charge intragranulaire, le liant intragranulaire, le délitant intragranulaire, et le tensioactif intragranulaire comprend en outre le séchage des granules.

14. Procédé selon la revendication 12 ou 13, comprenant l'enrobage du noyau avec la couche d'enrobage.

15. Formulation selon l'une quelconque des revendications 1 à 5, ou comprimé selon l'une quelconque des revendications 6 à 11, pour utilisation dans le traitement d'une maladie cardiaque chez un sujet en ayant besoin.

16. Formulation ou comprimé selon la revendication 15, pour utilisation dans le traitement d'une maladie cardiaque chez un sujet en ayant besoin, la maladie cardiaque étant une cardiomyopathie hypertrophique (CMH).

17. Formulation ou comprimé selon la revendication 16, pour utilisation dans le traitement d'une cardiomyopathie hypertrophique (CMH) chez un sujet en ayant besoin, la CMH étant obstructive ou non obstructive ou étant associée à une mutation sarcomérique et/ou non sarcomérique.

18. Formulation ou comprimé selon la revendication 15, pour utilisation dans le traitement d'une maladie cardiaque chez un sujet en ayant besoin, la maladie cardiaque étant une insuffisance cardiaque à fraction d'éjection préservée (ICFEP).
